(19) Europäisches Patentamt
European Patent Office
Office européen des brevets

(11) Veröffentlichungsnummer: **0 181 831 B1**

(12) # EUROPÄISCHE PATENTSCHRIFT

(45) Veröffentlichungstag der Patentschrift: **25.08.93**

(51) Int. Cl.[5]: **C07D 205/08**, C07F 7/18, C07D 303/48, C07C 235/04, //C07D499/00

(21) Anmeldenummer: **85810435.9**

(22) Anmeldetag: **25.09.85**

Die Akte enthält technische Angaben, die nach dem Eingang der Anmeldung eingereicht wurden und die nicht in dieser Patentschrift enthalten sind.

(54) **Verfahren zur Herstellung von optisch aktiven Acyloxyazetidinonen.**

(30) Priorität: **01.10.84 CH 4700/84**
**05.02.85 CH 513/85**

(43) Veröffentlichungstag der Anmeldung:
**21.05.86 Patentblatt 86/21**

(45) Bekanntmachung des Hinweises auf die Patenterteilung:
**25.08.93 Patentblatt 93/34**

(84) Benannte Vertragsstaaten:
**AT BE CH DE FR GB IT LI NL SE**

(56) Entgegenhaltungen:
**EP-A- 0 003 960**

**RECENT ADVANCES OM THE CHEMISTRY OF BETA-LACTAM ANTIBIOTICS, Nr. 38, 30. Juni / 2. Juli 1980, Seiten 330-348, The Royal Society of Chemistry, London, GB; S. OIDA: "Penems, synthetic approach to thienamycin analogues"**

**THE JOURNAL OF ORGANIC CHEMISTRY, Band 39, Nr. 1, 11. Januar 1974, Seiten 77-83; A. DE BOER et al.: "Baeyer-Villiger oxidation of delta1(9)-octalone-2 and delta1(8)-indanone-2"**

**TETRAHEDRON LETTERS, Nr. 12, März 1973, Seiten 923-926, Pergamon Press, Oxford, GB; M.J. BOGDANOWICZ et al.: "New synthetic reactions: lactone annelation"**

(73) Patentinhaber: **CIBA-GEIGY AG**
**Klybeckstrasse 141**
**CH-4002 Basel(CH)**

(72) Erfinder: **Kalvoda, Jaroslav, Dr.**
**Leimgrubenweg 21**
**CH-4102 Binningen(CH)**
Erfinder: **Ernest, Ivan, Dr.**
**Rüttihard 10**
**CH-4127 Birsfelden(CH)**
Erfinder: **Biollaz, Michel, Dr.**
**Im Hirshalm 50**
**CH-4125 Riehen(CH)**
Erfinder: **Hungerbühler, Ernst, Dr.**
**Zeisigweg 4**
**CH-4310 Rheinfelden(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft ein Verfahren zur Herstellung von (4R)-3,4-trans-disubstituierten Azetidinonen der Formel

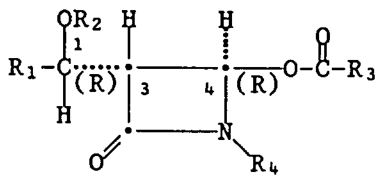

(I) ,

worin $R_1$ Wasserstoff oder $C_1$-$C_4$-Alkyl, $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeuten und die neuen Verbindungen der Formel I, worin $R_1$, $R_2$, $R_3$ und $R_4$ die angegebenen Bedeutungen haben.

Die im Rahmen dieser Erfindung verwendeten allgemeinen Definitionen haben vorzugsweise die folgenden Bedeutungen:

Der im Zusammenhang mit Gruppen oder Resten, z.B. Niederalkyl, Niederalkoxy, Niederalkanoyl etc., verwendete Ausdruck "Nieder" bedeutet, dass die so bezeichneten Gruppen oder Reste, falls nicht ausdrücklich anders definiert, bis einschliesslich 7, bevorzugt bis einschliesslich 4, C-Atome enthalten.

$C_1$-$C_4$-Alkyl $R_1$ ist vorzugsweise Methyl, ferner Ethyl, n-Propyl, Isopropyl, n-Butyl, Isobutyl oder tert-Butyl.

$R_1$ ist vorzugsweise Methyl oder auch Wasserstoff.

$R_2$ ist vorzugsweise Wasserstoff.

Hydroxyschutzgruppen $R_2'$ , sowie deren Einführung und Abspaltung sind beispielsweise in "Protective Groups in Organic Chemistry", Plenum Press, London und New York 1973, sowie in "Protective Groups in Organic Chemistry", Wiley, New York 1974 beschrieben.

Eine Hydroxyschutzgruppe $R_2'$ ist beispielsweise die Acylgruppe einer substituierten Carbon- oder Sulfonsäure, z.B. durch Halogen, wie Fluor oder Chlor, substituiertes Niederalkanoyl, z.B. 2,2-Dichlor- oder 2,2,2-Trifluoracetyl, die gegebenenfalls substituierte, z.B. durch Halogen, z.B Chlor, Phenyl oder p-Hitrophenyl substituierte, Acylgruppe eines Niederalkyl- oder Niederalkenyl-Kohlensäurehalbesters, z.B. 2,2,2-Trichlorethoxycarbonyl, Allyloxycarbonyl, Benzyloxycarbonyl oder p-Nitrobenzyloxycarbonyl, gegebenenfalls z.B. durch Nitro, Halogen oder Niederalkoxy substituiertes Aroyl, wie Benzoyl, z.B. 4-Nitro-, 3,5-Dinitro-, 4-Chlor- oder 4-Methoxybenzoyl, Niederalkansulfonyl, wie Methansulfonyl, Arylsulfonyl, wie Toluolsulfonyl, 2-Oxa- oder 2-Thiacycloalkyl mit 5 bis 7 Ringatomen, z.B. 2-Tetrahydrofuryl oder 2-Tetrahydropyranyl oder ein Thiaanaloges davon, 1-Niederalkoxyniederalkyl, z.B. Methoxymethyl oder 1-Ethoxyethyl, oder eine durch Niederalkyl, Arylniederalkyl und/oder Aryloxy dreifach substituierte Silylgruppe.

$R_2'$ ist vorzugsweise die substituierte Acylgruppe eines Kohlensäurehalbesters, z.B. 2,2,2-Trichlorethoxycarbonyl, Allyloxycarbonyl, Benzyloxycarbonyl oder p-Nitrobenzyloxycarbonyl, durch Nitro ein bis zweifach substituiertes Benzoyl, z.B. 4-Nitro- oder 3,5-Dinitrobenzoyl, oder eine durch die genannten Gruppen substituierte Silylgruppe, z.B. Triniederalkylsilyl, wie Trimethyl- oder Triethylsilyl, Diniederalkyl-triniederalkylmethylsilyl, z.B. Dimethyl- oder Diethyl-(2-ethyl-2-propyl)silyl, Dimethyl-(tert-butyl)silyl, oder Dimethyl-(2,3-dimethylbut-2-yl)silyl, Diaryl(niederalkyl)silyl, z.B. Diphenyl-(tert-butyl)silyl, Aryl-(diniederalkyl)silyl, z.B. tert-Butyl-methyl-phenylsilyl, sowie Tri-niederalkylaryloxy-(diniederalkyl)silyl, z.B. 2,4,6-Tri-(tert-butyl)-phenoxy(dimethyl)silyl.

Besonders bevorzugte Schutzgruppen $R_2'$ sind wegen besonders leichter Abspaltbarkeit 2,2,2-Trichlorethoxycarbonyl, Allyloxycarbonyl und 3,5-Dinitrobenzoyl.

Substituiertes Phenyl $R_3$ ist mono- bis trisubstituiert durch $C_1$-$C_4$-Alkoxy, z.B. Methoxy, $C_1$-$C_4$-Alkyl, z.B. Methyl, und/oder Halogen, z.B. Chlor, z.B. 4-Methoxy-oder 4-Nitrophenyl, 2-, 3- oder 4-Chlorphenyl oder 4-Tolyl. $R_3$ ist bevorzugt Phenyl.

Eine Aminoschutzgruppe $R_4'$ ist vorzugsweise oxidativ abspaltbares Arylmethyl oder Aryl, wobei Aryl bevorzugt durch eine oder mehrere, z.B. zwei, Niederalkoxygruppen, z.B. Methoxy, substituiertes Phenyl ist, und insbesondere 4-Methoxybenzyl oder 2,4-Dimethoxybenzyl bzw. 4-Methoxyphenyl oder 3,4-Dimethoxyphenyl bedeutet.

Die Erfindung betrifft vorzugsweise Verbindungen der Formel I, worin $R_1$ Wasserstoff oder Methyl, $R_2$ Wasserstoff, 2,2,2-Trichlorethoxycarbonyl, Allyloxycarbonyl oder 3,5-Dinitrobenzoyl, $R_3$ Phenyl und $R_4$

Wasserstoff oder p-Methoxyphenyl ist.

House, Modern Synthetic Reactions, 2nd Ed., Seiten 321-328; Montury. M., et al., Tetrahedron 33, 2819-2824 (1977); und Momose, T., et al., Chem. Pharmac. Bull 26, 2589-2593 (1978) zeigen, dass mit Acetyl oder Benzoyl substituierte cyclische Verbindungen bei Behandlung mit Persäuren eine Baeyer-Villiger-Reaktion eingehen. Während bei Acetyl-substituierten Ausgangsverbindungen stets die Acetoxy-substituierten Produkte erhalten werden, ergibt sich aus diesen Referenzen, dass für Benzoyl-substituierte Cyclen mit vier Ringgliedern die Reaktion zum Phenyloxycarbonylprodukt zu erwarten ist. Im Gegensatz zu diesem Ergebnis entstehen beim erfindungsgemässen Verfahren durch Benzoyloxy substituierte Cyclen mit vier Ringgliedern.

Das erfindungsgemässe Verfahren ist dadurch gekennzeichnet, dass man eine Verbindung der Formel

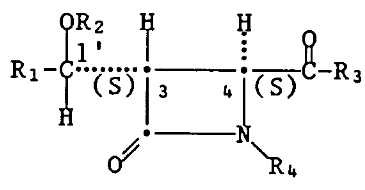

(II) ,

worin $R_1$, $R_2$, $R_3$ und $R_4$ die unter Formel I genannten Bedeutungen haben, durch Behandeln mit einer Persäure einer Baeyer-Villiger-Reaktion unterwirft, und gewünschtenfalls in einer erhältlichen Verbindung der Formel I, worin $R_2$ Wasserstoff bedeutet, die freie Hydroxygruppe in die geschützte Hydroxygruppe $OR_2'$ überführt, und/oder gewünschtenfalls in einer erhältlichen Verbindung der Formel I die Hydroxyschutzgruppe $R_2'$ und/oder die Aminoschutzgruppe $R_4'$ abspaltet.

Bevorzugt ist in einem Ausgangsmaterial der Formel II $R_1$ Wasserstoff oder Methyl, $R_2$ Wasserstoff oder eine der genannten Hydroxyschutzgruppen $R_2'$ wie 2,2,2-Trichlorethoxycarbonyl, Allyloxycarbonyl, 3,5-Dinitrobenzoyl, Dimethyl-tert-butylsilyl oder Dimethyl-2,2-dimethylbut-2-ylsilyl, $R_4$ Wasserstoff oder eine Aminoschutzgruppe $R_4$, insbesondere p-Methoxyphenyl, und $R_3$ Phenyl.

In die Reaktion können organische, anorganische Persäuren oder Iminopercarbonsäuren oder Salze davon, wie Alkalimetall- oder Erdalkalimetallsalze, z.B. Natrium-, Kalium- oder Magnesiumsalze, eingesetzt werden, z.B. gegebenenfalls halogenierte Niederalkanpercarbonsäuren, wie Peressigsäure oder Trifluorperessigsäure, gegebenenfalls durch Nitro und/oder Halogen mono- oder disubstituierte aromatische Persäuren, wie Perbenzoesäure, p-Nitroperbenzoesäure, m-Chlorperbenzoesäure, 3,5-Dinitroperbenzoesäure, Monoperphthalsäure oder ein Salz davon, z.B. Magnesium-monoperphthalat, Peroxoschwefelsäuren, z.B. Peroxoschwefelsäure, Trihalogeniminoperessigsäure, z.B. Trichlor- oder Trifluoriminoporessigsäure. Bevorzugt sind Peressigsäure, m-Chlorperbenzoesäure und Monoperphthalsäure.

Die Persäuren können auch in situ gebildet werden, z.B. aus der entsprechenden Säure oder einem sauren Salz davon und Wasserstoffperoxid. Insbesondere die Iminopercarbonsäuren werden üblicherweise in situ hergestellt, z.B. aus den entsprechenden Nitrilen und Wasserstoffperoxid, bevorzugt in Gegenwart eines schwach sauren Puffers, z.B. Kaliumhydrogenphosphat. Die Reaktion findet in einem üblichen Lösungsmittel, z.B. einem halogenierten Kohlenwasserstoff, wie Methylenchlorid oder Chloroform, einem Ester, wie Essigsäureethylester, einer flüssigen Säure, wie Eisessig, oder einem Alkohol, wie einem niederen Alkanol, z.B. Ethanol, bei Temperaturen zwischen etwa 0° und etwa 100°C, gegebenenfalls in einer Inertgasatmosphäre, und gegebenenfalls unter Druck, z.B. bei etwa bis zu 10 bar, statt.

Die Hydroxyschutzgruppe $R_2'$ kann in an sich bekannter Weise eingeführt werden, z.B. durch Umsetzung einer Verbindung der Formel I, worin $R_2$ Wasserstoff und $R_4$ bevorzugt eine Aminoschutzgruppe $R_4'$ bedeutet, mit einem reaktionsfähigen Derivat einer Säure, deren Acylgruppe die Hydroxyschutzgruppe ist, z.B. durch Umsetzung mit einem Anhydrid, z.B. Trifluoressigsäureanhydrid, einem gemischten Anhydrid, z.B. einem Säurehalogenid, z.B. 2,2-Dichloracetylchlorid, einem gemischten Anhydrid eines Kohlensäurehalbesters, z.B. 2,2,2-Trichlorethyl-, Allyl-, Phenyl- oder p-Nitrophenylchlorformiat, durch Reaktion mit 1,2-Dihydrofuran oder 1,2-Dihydropyran in Gegenwart einer Säure, z.B. p-Toluolsulfonsäure, oder durch Reaktion mit einem Halogensilan, z.B. einem Triniederalkylhalogensilan, wie Trimethylchlor- oder Dimethyl-tert-butylchlorsilan.

In einer erhältlichen Verbindung der Formel I erfolgt die Abspaltung der Schutzgrupe $R_2'$ in an sich bekannter Weise, z.B. durch Solvolyse, z.B. Acidolyse, Reduktion oder Oxidation.

Die Acylgruppe $R_2'$ kann unter sauren oder alkalischen Bedingungen verseift werden, je nach der Bedeutung von $R_2'$ beispielweise mit Säuren, wie Ameisen- oder Trifluoressigsäure oder mit Basen, wie einem Alkalimetallhydroxid oder -carbonat, z.B. Natriumbicarbonat, einem bicyclischen Amidin, wie 1,5-Diazabicyclo[5.4.0]undec-5-en, oder einem Alkalimetallhalogenid. Eine 2-Oxa- oder 2-Thiacycloalkylgruppe wird in Gegenwart von Säuren abgespalten.

Halogenniederalkoxycarbonyl und gegebenenfalls durch Nitro substituiertes Benzyloxycarbonyl $R_2'$ können durch Reduktion, beispielsweise durch katalytische Hydrierung mit Wasserstoff in Gegenwart eines geeigneten Hydrierkatalysators, z.B. Platinoxid oder Palladium, oder durch Behandeln mit chemischen Reduktionsmitteln, wie Zink in Gegenwart von wässriger Essigsäure, abgespalten und durch Wasserstoff ersetzt werden.

Die wie angegeben substituierte Silylgruppe $R_2'$ kann in Gegenwart hydroxylgruppenhaltiger Lösungsmittel neutral, sauer oder alkalisch, oder insbesondere durch Behandeln mit einem Fluoridanionen liefernden Salz der Fluorwasserstoffsäure, wie mit einem Alkalimetallfluorid, z.B. Natriumfluorid, gegebenenfalls in Anwesenheit eines makrocyclischen Polyethers ("Kronenether"), oder mit dem Fluorid einer organischen quaternären Base, wie Tetra-niederalkylammoniumfluorid, z.B. Tetrabutylammoniumfluorid, abgespalten werden.

Die Ueberführung der Niederalkenyloxycarbonyloxy-Gruppe $-OR'_2$ in Hydroxy, wobei Niederalkenyl insbesondere für Allyl steht, erfolgt bevorzugt mit einem Niederalkenylgruppen-Akzeptor in Gegenwart von Tetrakis-triphenylphosphin-palladium und gegebenenfalls in Gegenwart von Triphenylphosphin. Geeignete Akzeptoren für Niederalkenylgruppen, wie insbesondere die Allylgruppe, sind z.B. Amine, wie insbesondere sterisch gehinderte Amine, z.B. tert-Butylamin, ferner Tri-niederalkylamine, z.B. Triethylamin, Morpholin oder Thiomorpholin, aliphatische oder cycloaliphatische ß-Dicarbonylverbindungen, z.B. Acetylaceton, Acetessigsäureethylester oder Dimedon, ferner auch Niederalkancarbonsäuren, z.B. Essigsäure oder Propionsäure. Ein bevorzugter Akzeptor ist Dimedon. Die Reaktion wird mit 1,5 bis 10 Moläquivalenten des Niederalkenylgruppen-Akzeptors in Gegenwart von 2 bis 10 Mol-%, insbesondere 5 bis 8 Mol-% (bezogen auf die Ausgangsverbindung der Formel I), Tetrakis-triphenylphosphin-palladium-Katalysator, gegebenenfalls in Gegenwart von bis zu 50 Mol-% Triphenhylphosphin in einem inerten Lösungsmittel, wie einem Ether, z.B. Dioxan oder insbesondere Tetrahydrofuran, einem Halogenkohlenwasserstoff, z.B. Methylenchlorid, einem niederen Alkanol, z.B. Ethanol, einem Ester, z.B. Ethylacetat, oder in einem Gemisch davon bei Raumtemperatur oder etwas erhöhter oder erniedrigter Temperatur, z.B. bei etwa 0° bis etwa 40°C, bevorzugt bei etwa 20° bis etwa 25°C, erforderlichenfalls in einer Inertgasatmosphäre, wie in einer Stickstoff- oder Argonatmosphäre, durchgeführt.

Die Arylmethylgruppe $R_4'$ , z.B. 4-Methoxy- oder 2,4-Dimethoxybenzyl, wird z.B. oxidativ abgespalten, z.B. durch Umsetzung mit einem starken Oxydationsmittel, z.B. einem anorganischen Peroxosalz, z.B. Natrium- oder Kaliumperoxodisulfat, in Gegenwart eines sauren Salzes, z.B. Dikaliumhydrogenphosphat, und gegebenenfalls in Gegenwart eines Katalysators, z.B. eines Metallsalzes, z.B. eines Eisen(II)- und/oder Kupfer(II)-salzes, z.B. Eisen(II)-sulfatheptahydrat und/oder Kupfer(II)-acetathydrat, oder insbesondere durch Umsetzung mit einem Cer(IV)-Salz, z.B. Cer(IV)iodat, Cer(IV)nitrat ($CeOH(NO_3)_3$), Cer(IV)-sulfat oder, bevorzugt mit Diammonium-Cer(IV)-hexanitrat, z.B. in wässrig-organischen Medien, bevorzugt in wässrigem Acetonitril.

Die Arylgruppe $R_4'$ , z.B. 4-Methoxyphenyl oder 3,4-Dimethoxyphenyl, wird bevorzugt oxidativ abgespalten, z.B. ozonolytisch in Ethylacetat und anschliessende Zersetzung der gebildeten Ozonide durch Reduktion mit einem Reduktionsmittel, z.B. Natriumthiosulfat, oder durch Umsetzung mit einem Cer(IV)-Salz, z.B. Diammonium-Cer(IV)-hexanitrat.

$R_4'$ kann, abhängig von der Bedeutung von $R_2'$ , entweder selektiv oder zusammen mit $R'_2$ abgespalten werden.

Die beschriebenen Spaltungsreaktionen werden unter an sich bekannten Bedingungen durchgeführt, wenn notwendig unter Kühlen oder Erwärmen und gegebenenfalls in einer Inertgas-, z.B. Stickstoffatmosphäre.

Die vorliegende Erfindung hat vorzugsweise ein Verfahren zur Herstellung von Verbindungen der Formel I zum Gegenstand, worin $R_1$ Wasserstoff oder Methyl, $R_2$ Wasserstoff oder eine Hydroxyschutzgruppe $R_2'$ , z.B. die substituierte Acylgruppe eines Kohlensäurehalbesters, z.B. 2,2,2-Trichlorethoxycarbonyl, Allyloxycarbonyl, Benzyloxycarbonyl oder p-Nitrobenzyloxycarbonyl, 4-Nitro- oder 3,5-Dinitrobenzoyl oder Triniederalkylsilyl, z.B. Trimethylsilyl, Dimethyl-tert-butylsilyl, oder Dimethyl-(2,3-dimethylbut-2-yl)silyl, $R_3$ Phenyl oder Methyl und $R_4$ Wasserstoff oder eine Aminoschutzgruppe $R_4'$ , z.B. 4-Methoxybenzyl, 2,4-Dimethoxybenzyl, 4-Methoxyphenyl oder 3,4-Dimethoxyphenyl, bedeuten, und worin das 1'-C-Atom der Seitenkette die R- oder die S-Konfiguration hat, wenn $R_1$ Niederalkyl bedeutet.

Verbindungen der Formel I, insbesondere solche, worin $R_1$ Wasserstoff oder Methyl ist, $R_2$ Wasserstoff oder auch eine Hydroxyschutzgruppe $R_2'$ darstellt, und $R_4$ Wasserstoff ist, sind wertvolle Zwischenprodukte, die auf an sich bekannte Weise zu antibiotisch wirksamen Penem- oder Carbapenemverbindungen weiterverarbeitet werden können. Die Weiterverarbeitung erfolgt analog derjenigen von bekannten Azetidinonverbindungen der Formel I, worin $R_3$ Methyl ist, z.B. wie von T. Hayashi et al., Chem. Pharm. Bull. 29, 3158, 1981, oder in den Europäischen Patentanmeldungen Nr 42026 (für Peneme) oder Nr. 78026 (für Carbapeneme) beschrieben ist.

Die Erfindung betrifft ebenfalls die Ausgangsverbindungen der Formel II, insbesondere diejenigen, worin $R_1$, $R_2$, $R_3$ und $R_4$ die bevorzugten Bedeutungen hat, und Verfahren zu ihrer Herstellung.

Vorzugsweise sind solche Ausgangsverbindungen der Formel II, worin $R_1$ Wasserstoff oder Methyl, $R_2$ Wasserstoff oder eine Hydroxyschutzgruppe $R_2'$, $R_3$ Phenyl und $R_4$ Wasserstoff oder einen Aminoschutzgruppe $R_4'$ bedeuten, betroffen.

Sehr bevorzugt ist eine Ausgangsverbindung der Formel II, worin $R_1$ Methyl, $R_2$ Wasserstoff, 2,2,2-Trichlorethoxycarbonyl, Allyloxycarbonyl, tert-Butyl-dimethylsilyl, Dimethyl-2,3-dimethylbut-2-ylsilyl oder 3,5-Dinitrobenzoyl, $R_3$ Phenyl und $R_4$ Wasserstoff oder p-Methoxyphenyl bedeutet, betroffen.

Das Verfahren zur Herstellung von Verbindungen der Formel II ist dadurch gekennzeichnet, dass man a) ein $\alpha$-Carbanion einer Verbindung der Formel

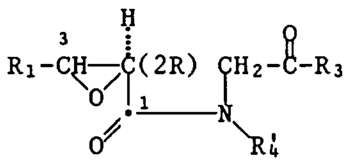

(III) ,

worin $R_1$, $R_3$ und $R_4'$ die unter Formel I genannten Bedeutungen haben, und das 2-C-Atom die R- und, wenn $R_1$ $C_1$-$C_4$-Alkyl, insbesondere Methyl, bedeutet, das 3-C-Atom die R- oder die S-Konfiguration hat, oder eine Verbindung der Formel

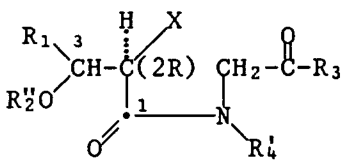

(IV),

worin $R_1$, $R_3$ und $R_4'$ die unter Formel I genannten Bedeutungen haben, $R_2''$ eine unter den Bedingungen des Ringschlussverfahrens nicht abspaltbare Hydroxyschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, zyklisiert, oder b) eine cis-Verbindung der Formel

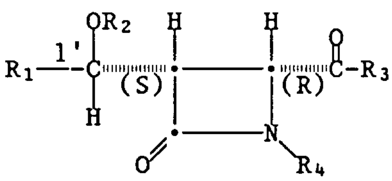

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt, und/oder, wenn erwünscht, eine erhältliche Verbindung der Formel II in eine andere Verbindung der Formel II umwandelt.

In einem Ausgangsmaterial der Formel IV ist eine unter den Bedingungen des Ringschlussverfahrens nicht abspaltbare Hydroxyschutzgruppe $R_2''$ eine der unter $R_2'$ genannten Acylgruppen einer substituierten Carbon- oder Sulfonsäure, z.B. 2,2,2-Trifluoracetyl, die substituierte Acylgruppe eines Kohlensäurehalbesters, z.B. p-Nitrobenzyloxycarbonyl, 2-Tetrahydrofuryl oder -pyranyl, oder 1-Niederalkoxyniederalkyl, z.B. Methoxymethyl oder 1-Ethoxyethyl, oder eine gegebenenfalls substituierte Aroyl-, z.B. 4-Nitrobenzoyl- oder 3,5-Dinitrobenzoylgruppe, und eine geeignete nukleofuge Abgangsgruppe X ist beispielsweise Halogen, z.B. Chlor, Brom oder Jod, Niederalkanoyloxy, z.B. Acetoxy, Arylsulfonyloxy, z.B. Phenylsulfonyloxy oder Tosyloxy, oder Niederalkylsulfonyloxy, z.B. Mesyloxy.

Unter dem α-Carbanion einer Verbindung der Formeln III oder IV ist ein Anion zu verstehen, bei dem sich die negative Ladung auf dem Kohlenstoffatom in α-Stellung zur -CO-$R_3$ Gruppe befindet, d.h. eine entsprechende Verbindung mit der Teilformel -N-$CH^{\ominus}$-CO-$R_3$. Solche Carbanionen werden in situ hergestellt, indem man eine Verbindung der Formeln III oder IV in einem geeigneten Lösungsmittel mit einer Base behandelt, die solche Carbanionen bildet. Solche Basen sind beispielsweise Alkalimetallbasen, wie Hydroxide oder Carbonate von Natrium, Kalium oder Lithium, oder organische Alkalimetallverbindungen, z.B. Niederalkyllithiumverbindungen, wie n-Butyl- oder tert-Butyllithium, Alkalimetallamide, z.B. Lithiumdiisopropylamid oder -di-tert-butylamid, und insbesondere Alkalimetallamide, worin zwei Wasserstoffatome durch Silyl-, z.B. Trimethylsilylgruppen ersetzt sind, z.B. Lithium-, Natrium- oder Kalium-bis-(trimethylsilyl)-amid.

Weitere Basen sind organische Stickstoffbasen, z.B. Amidine, wie 1,5-Diazabicyclo[4.3.0]non-5-en und 1,5-Diazabicyclo[5.4.0]undec-5-en, sowie Reagenzien, die in einem aprotischen, organischen Lösungsmittel Fluoridionen liefern und besonders zur Herstellung von Carbanionen der Verbindungen der Formel III geeignet sind.

Ein Reagenz, welches in einem aprotischen, organischen Lösungsmittel Fluoridionen liefert, ist vorzugsweise ein Tetra-niederalkylammoniumfluorid, insbesondere Tetra-n-butylammoniumfluorid.

Geeignete Lösungsmittel zur Carbanionbildung sind beispielsweise Kohlenwasserstoffe, wie Benzol oder Toluol, halogenierte Kohlenwasserstoffe, wie Methylenchlorid oder Chloroform, Ether, wie Dioxan, Tetrahydrofuran oder Dimethoxyethan, Amide, wie Dimethylformamid oder Hexamethylphosphorsäuretriamid, Sulfoxide, wie Dimethylsulfoxid oder Nitrile, wie Acetonitril, oder Mischungen davon. Die Reaktion kann auch unter Phasentransferbedingungen vorgenommen werden, z.B. im System bestehend aus Methylenchlorid und 50 prozentiger Natronlauge in Gegenwart eines Phasentransferkatalysators, wie Benzyltriethylammoniumchlorid oder Tetrabutylammoniumhydrogensulfat. Die Reaktionstemperatur hängt ebenfalls von der verwendeten Base ab und liegt zwischen ca. -80 °C und 80 °C. Man arbeitet vorzugsweise unter Inertgasatmosphäre, z.B. Argon- oder Stickstoffatmosphäre.

Bei dem Verfahren findet eine Inversion der Konfiguration am 2-C-Atom des Ausgangsmaterials statt, so dass aus einer 2R-Verbindung der Formeln III oder IV eine 3S-Verbindung der Formel II entsteht. Die Konfiguration des 1'-C-Atoms ($R_1$ = $C_1$-$C_4$-Alkyl) in der Seitenkette bleibt unverändert. Beispielsweise erhält man aus R-Glycidsäureamiden der Formel (III) 3S-Azetidinone der Formel II oder aus (2 R,3 R)-2,3-Epoxybuttersäureamiden der Formel III (3S-1'R)-3-Hydroxyethylazetidinone der Formel II.

Das Isomerisierungsverfahren b) findet in einem geeigneten Lösungsmittel oder Lösungsmittelgemisch in Gegenwart einer Base statt. Geeignete Lösungsmittel und Basen sowie auch die Reaktionsbedingungen sind die gleichen, die für das Zyklisierungsverfahren a) eingesetzt werden können. Bevorzugt werden aprotische dipolare Lösungsmittel, wie Dimethylformamid, N-Methylpyrrolidin, Dimethylsulfoxid oder dergleichen eingesetzt. Als Basen kommen vor allem Kaliumcarbonat oder 1,5-Diazabicyclo[5.4.0]undec-5-en in Frage, wobei auch unter den genannten Phasentransferbedingungen gearbeitet werden kann.

Die cis-Verbindungen der Formel II' können bei Zyklisierungsreaktion a) als Nebenprodukte entstehen. Sie können auf übliche Weise, z.B. durch Chromatographieren, abgetrennt werden.

Verbindungen der Formel III können in an sich bekannter Weise durch Umsetzung von Verbindungen der Formel

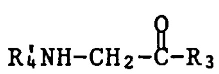

(VII),

worin $R_3$ und $R_4'$ die unter Formel V genannten Bedeutungen haben, mit Epoxysäuren der Formel

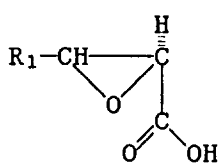

(VIII)

oder deren Salzen hergestellt werden, analog wie weiter unten für die Umsetzung einer Carbonsäure der Formel VI mit einem Amin der Formel VII angegeben ist. Die Epoxysäuren der Formel VIII sind bekannt oder können auf an sich bekannte Weise hergestellt werden.

In einer bevorzugten Ausführungsform des Verfahrens werden Ausgangsmaterialien der Formel III aus Verbindungen der Formel

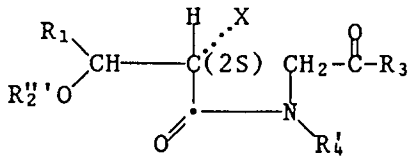

(V),

worin $R_1$, $R_3$, $R_4'$ und X die genannten Bedeutungen haben, $R_2'''$ Wasserstoff oder eine unter den Bedingungen der Epoxidbildung abspaltbare Hydroxyschutzgruppe darstellt und worin das 3-C-Atom die R- oder S-Konfiguration hat, wenn $R_1$ $C_1$-$C_4$-Alkyl bedeutet, unter den genannten carbanionbildenden Bedingungen und unter Waldenscher Umkehr am 2-C-Atom in situ hergestellt.

In einer Verbindung der Formel V ist eine unter den Bedingungen der Epoxidbildung abspaltbare Hydroxyschutzgruppe $R_2'''$ eine Triniederalkylsilylgruppe, z.B. Dimethyl-tert-butylsilyl oder Trimethylsilyl. X bedeutet bevorzugt Halogen, z.B. Chlor oder Brom, Niederalkansulfonyloxy, z.B. Mesyloxy, oder Arylsulfonyloxy, z.B. Tosyloxy.

Die Abspaltung von $R_2'''$-X, worin $R_2'''$ Wasserstoff ist, erfolgt mit einer der genannten carbanionbildenden Basen, z.B. Natrium- oder Kaliumhydroxid oder Natrium- oder Kaliumcarbonat, vorzugsweise mit einem Amidin, z.B. 1,5-Diazabicyclo[5.4.0]undec-5-en, oder auch mit einem Tetraniederalkylammoniumfluorid, z.B. entwässertem Tetra-n-butylammoniumfluorid. Wenn $R_2'''$ eine unter den Bedingungen der Epoxidbildung abspaltbare Triniederalkylsilylgruppe ist, wird bevorzugt ein Tetraniederalkylammoniumfluorid verwendet.

Die Umsetzung zu einer Epoxyverbindung der Formel III wird vorzugsweise in einem inerten, vorzugsweise wasserfreien, Lösungsmittel oder Lösungsmittelgemisch vorgenommen, beispielsweise in einem Carbonsäureamid, z.B. Dimethylformamid, einem halogenierten Kohlenwasserstoff, z.B. Methylenchlorid, Chloroform, Tetrachlorkohlenstoff oder Chlorbenzol, einem Keton, z.B. Aceton, einem cyclischen Ether, z.B. Dioxan oder Tetrahydrofuran, einem Ester, z.B. Essigsäureethylester, oder einem Nitril, z.B. Acetonitril, oder in Mischungen davon, gegebenenfalls bei erniedrigter oder erhöhter Temperatur, z.B. in einem Temperaturbereich von etwa -40 °C bis etwa +100 °C, bevorzugt von etwa -10 °C bis etwa +50 °C, und gegebenenfalls unter Inertgas-, z.B. Stickstoffatmosphäre.

Bei der Umsetzung gemäss diesem Verfahren kehrt sich die Konfiguration am 2-C-Atom um, während die Konfiguration des 3-C-Atoms ($R_1$ = $C_1$-$C_4$-Alkyl) erhalten bleibt. Aus 2S-Halogen-3R-hydroxycarbonsäureamiden der Formel V entstehen die (2R,3R)-Verbindungen der Formel III. Entsprechendes gilt für die 2S-Halogen-3S-hydroxycarbonsäureamide der Formel III.

Das Epoxid der Formel III kann auch nach seiner Herstellung durch Abspaltung von $R_2'''$-X aus einer Verbindung der Formel V isoliert werden.

Die Umwandlung einer Verbindung der Formel V, worin $R_2'''$ Wasserstoff ist, in die Epoxyverbindung der Formel III und deren Isolierung erfolgt in an sich bekannter Weise, z.B. unter den aus der Steroidchemie, siehe C. Djerassi et al. "Steroid Reactions", Holden Day, San Francisco 1963, S. 606-613, bekannten Reaktionsbedingungen, insbesondere mittels einem der genannten Amidine, wie 1,5-Diazabicyclo[5.4.0]-undec-5-en. Wenn die Verbindung der Formel III vor ihrer Weiterverarbeitung isoliert werden soll, benutzt man bevorzugt eines der genannten Amidine in etwa äquimolarer Menge.

Verbindungen der Formel

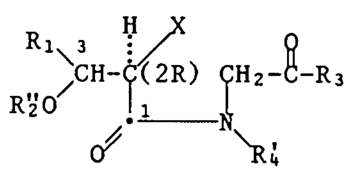

(IV),

worin $R_1$ Wasserstoff oder $C_1$-$C_4$-Alkyl, $R_2''$ eine unter den Bedingungen des Ringschlussverfahrens nicht abspaltbare Hydroxyschutzgruppe, $R_3$ Phenyl, durch $C_1$-$C_4$-Alkoxy, $C_1$-$C_4$-Alkyl und/oder Halogen mono- bis tri-substituiertes Phenyl oder 4-Nitrophenyl, $R_4'$ eine Aminoschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, lassen sich herstellen, indem man eine Carbonsäure der Formel

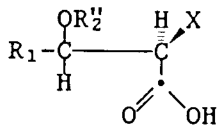

(VI')

worin $R_1$, $R_2''$ und X die unter Formel IV genannten Bedeutungen haben, mit einem Amin der Formel

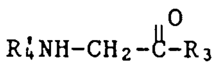

(VII),

worin $R_3$ und $R'_4$ die unter Formel (V) genannten Bedeutungen haben, umsetzt.

Verbindungen der Formel V lassen sich herstellen, indem man eine Carbonsäure der Formel

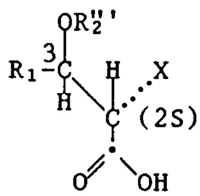

(VI)

worin $R_1$, $R_2''$ und X die unter Formel V genannten Bedeutung haben, mit einem Amin der Formel

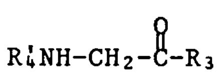

(VII),

worin $R_3$ und $R_4'$ die unter Formel V genannten Bedeutungen haben, umsetzt.

Die Umsetzung von Aminen der Formel VII mit einer Säure der Formel VI erfolgt auf an sich bekannte Weise unter acylierenden Bedingungen, beispielsweise, indem man die beiden Verbindungen in Gegenwart eines Kondensationsmittels miteinander kondensiert, oder ein reaktionsfähiges funktionelles Derivat einer Carbonsäure der Formel VI mit einem Amin der Formel VII umsetzt. Geeignete Kondensationsmittel sind beispielsweise Diimide, wie Diethyl- oder Dicyclohexylcarbodiimid, oder Di-N-heterocyclylcarbonylverbindungen, wie Carbonyldiimidazol, oder eine 1,2-Oxazoliumverbindung, wie 2-Ethyl-5-phenyl-1,2-oxazolium-3-sulfonat oder 2-tert-Butyl-5-methyl-1,2-oxazoliumperchlorat, die in Gegenwart eines inerten Lösungsmittels bei Raumtemperatur oder leicht erniedrigter oder erhöhter Temperatur angewendet werden. Reaktionsfähige funktionelle Derivate der Carbonsäure (VI) sind insbesondere Anhydride, insbesondere gemischte Anhydride, wie das entsprechende Carbonsäurechlorid oder -bromid, die gegebenenfalls in Gegenwart einer Base, mit dem Amin der Formel VII reagieren.

Verbindungen der Formel VI und VI', worin X Halogen ist, sind bekannt. Diese lassen sich beispielsweise in an sich bekannter Weise aus L-Serin oder L-Threonin bzw. deren D-Isomeren durch Diazotierung der Aminogruppe mit einem Nitritsalz, z.B. Kaliumnitrit, in Gegenwart einer Halogenwasserstoffsäure herstellen.

Bei Verwendung von L-Serin lassen sich nach den beschriebenen Verfahren ausgehend von Verbindungen der Formel VI Verbindungen der Formel I erhalten, worin der Kohlenstoff in 3-Stellung des Azetidinonrings die R-Konfiguration hat.

Bei Verwendung von L-Threonin erhält man Verbindungen der Formel I, worin das C-Atom in 3-Stellung des Azetidinonrings die R-Konfiguration und das 1'-C-Atom der Hydroxyethyl-Seitenkette die R-Konfiguration hat. Ist die Herstellung von Verbindungen der Formel I beabsichtigt, worin das 3-C-Atom die R-Konfiguration und das 1'-C-Atom der Hydroxyethyl-Seitenkette die S-Konfiguration hat, geht man von Allothreonin aus. Bei allen beschriebenen Umsetzungen bleibt die Konfiguration des C-Atoms, welches in Verbindungen der Formel I dem 1'-C-Atom der 3-Niederalkyl-CH-($OR_2$)-Seitenkette entspricht, unverändert.

Auf analoge Weise zur Herstellung von Verbindungen der Formel V lassen sich, ausgehend von Verbindungen der Formel VIII und den Verbindungen der Formel

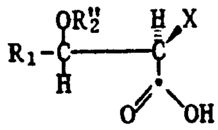

(VI')

die Verbindungen der Formel IV herstellen.

Verbindungen der Formel VII sind bekannt oder lassen sich, falls sie neu sind, in an sich bekannter Weise herstellen.

Die vorliegende Erfindung hat auch solche Ausführungsformen des Verfahrens zum Gegenstand, in denen man von einer auf irgendeiner Vorstufe des Verfahrens erhältlichen Verbindung ausgeht und die anschliessenden Verfahrensschritte ausführt.

Die Erfindung betrifft auch die Kombination der Verfahrensstufen ausgehend von den Verbindungen der Formeln VI, VI', VII und VIII bis zu den Verbindungen der Formel I.

Das erfindungsgemässe Verfahren hat den Vorteil, dass man die Verbindungen der Formel I in hoher Ausbeute und wirtschaftlicher Weise stereospezifisch herstellen kann.

Bevorzugt sind die neuen in den Beispielen angegebenen Verbindungen und Verfahren.

Die folgenden Beispiele dienen zur Illustration der Erfindung. Temperaturen sind in Graden Celsius und IR-Werte in $cm^{-1}$ angegeben. Für die $^1H$-NMR-Spektren sind die chemischen Verschiebungen ($\delta$) in ppm und Kopplungskonstanten J in Hertz (Hz) angegeben. Die spezifischen Drehwerte [$\alpha$] sind $[\alpha]_D^{20}$ -Werte. Für die chromatographische Trennung bei der präparativen Aufarbeitung lassen sich Säulen gefüllt mit Merck-Kieselgel 60 verwenden. Rf-Werte gelten für Fertigplatten für die Dünnschichtchromatographie (Merck 60 F 254).

Abkürzungen:

| | |
|---|---|
| m | = mittelstarke Absorptionsbande |
| sh | = scharfe Absorptionsbande |
| s | = Singulett |
| m | = Multiplett |
| d | = Dublett |
| F. | = Schmelzpunkt |
| Kp. | = Siedepunkt |
| dd | = Dublett von Dubletts |
| AB | = Signalgruppe vom AB-Typ |

Beispiel 1: (2S,3R)-2-Brom-3-hydroxybuttersäure-N-p-methoxyphenyl-N-phenacylamid

Eine Lösung von 13,73 g (2S,3R)-2-Brom-3-hydroxybuttersäure und 18,08 g Phenacyl-p-anisidin in 70 ml trockenem Tetrahydrofuran wird mit 15,47 g Dicyclohexylcarbodiimid versetzt und unter Argon-Atmosphäre 48 Stunden bei Raumtemperatur gerührt. Der während der Umsetzung gebildete Dicyclohexylharnstoff wird abgenutscht und das Filtrat im Wasserstrahlvakuum eingedampft. Der amorphe Rückstand wird zwecks Reinigung an der 60-fachen Gewichtsmenge Kieselgel mit einem Toluol/Essigester (9:1)-Gemisch chromatographiert. Die erhaltene Titelverbindung (NMR-Spektrum ($CDCl_3$) Signale bei 1,23 (d), 3,83 (s), 3,88 (s), 4,10 (m), 4,18 (d), 4,75/4,80/5,31/5,36 (AB) und 6,94 (dd)) kann direkt für die nachfolgende Zyklisation verwendet werden.

Beispiel 2: (2R,3R)-2,3-Epoxybuttersäure-N-p-methoxyphenyl-N-phenacylamid

Eine Lösung von 406 mg (1 mMol) (2S,3R)-2-Brom-3-hydroxybuttersäure-N-p-methoxyphenyl-N-phenacylamid in 7 ml abs. Tetrahydrofuren wird unter Argon bei -10° mit 165 $\mu$l (169 mg; 1,1 mMol) 1,5-Diazabicyclo[5.4.0]undec-5-en versetzt und das erhaltene Reaktionsgemisch 5 Stunden bei 0° und weitere 3 Stunden bei Raumtemperatur gerührt. Zur Aufarbeitung wird das Reaktionsgemisch mit Methylenchlorid verdünnt und nacheinander mit Wasser, 5 %-iger wässeriger Citronensäure-Lösung und 8%-iger wässeriger Natriumbicarbonat-Lösung gewaschen. Die wässerigen Anteile werden mit Methylenchlorid nachextrahiert. Der durch Trocknen und Eindampfen im Vakuum der vereinigten organischen Anteile erhaltene Rückstand wird an Kieselgel-Platten in Toluol/Ethylacetat (1:1)-Gemisch chromatographiert. Man erhält die Titelverbindung als viskoses Oel, Rf (Merck Kieselgel Fertigplatten, Toluol/Ethylacetat 1:1): 0,27; (IR $CH_2Cl_2$): u.a.

Banden bei 1700, 1670, 1595, 1580, 1505, 1427, 1392, 1365, 1348, 1300, 1243, 1221, 1180, 1170, 1140, 1103, 1028, 982; NMR ($CDCl_3$): Signale bei 1,50 (d), 3,08 (m), 3,36 (d), 3,82 (s), 4,86 (d), 5,42 (d), 6,91 (m), 7,34 (m), 7,46 (m), 7,57(m), 7,93 (m).

Beispiel 3: (3S,4S,1'R)-N-p-Methoxyphenyl-3-(1'-hydroxyethyl)-4-benzoylazetidin-2-on

a) mit Lithium-bis-(trimethylsilyl)amid: Eine auf -20° abgekühlte Lösung von 3,37 g (2S,3R)-2-Brom-3-hydroxybuttersäure-N-p-methoxyphenyl-N-phenacylamid in 20 ml Tetrahydrofuran wird unter Rühren in Argon-Atmosphäre mit 33 ml einer 0,5-molaren Lösung von Lithium- bis-(trimethylsilyl)amid in Tetrahydrofuran versetzt und unter langsamem Erwärmen auf -10° 1 1/2 Stunden weitergerührt. Das Reaktionsgemisch wird auf Eiswasser gegossen und mit Methylenchlorid extrahiert. Die organische Phase wird nacheinander mit eiskalter 1N-Schwefelsäure, Wasser, eiskalter, gesättigter, wässriger Natriumhydrogencarbonat-Lösung und mit Wasser gewaschen, getrocknet und im Rotationsverdampfer eingedampft. Das erhaltene Rohprodukt wird an Kieselgel chromatographiert. Mit einem Toluol/Essigester (9:1)-Gemisch erhält man das (3S,4S,1'R)-N-p-Methoxyphenyl-3-(1'-hydroxyethyl)-4-benzoylazetidin-2-on, das nach Umlösen aus Methylenchlorid/Ether bei 111-113° schmilzt. [α] = -95,1° (c = 1,121 % in $CHCl_3$).

b) mit Natriumhydroxid: Eine Mischung von 950 mg (2S,3R)-2-Brom-3-hydroxy-buttersäure-N-p-methoxyphenyl-N-phenacylamid in 25 ml Methylenchlorid und 8 ml 50 %iger wässriger Natriumhydroxid-Lösung wird unter Zugabe von 75 mg Benzyltriethylammoniumchlorid 5 1/2 Stunden bei Raumtemperatur heftig gerührt. Von dem Reaktionsgemisch wird die Natronlauge abgetrennt, die organische Phase zweimal mit Wasser gewaschen, getrocknet und im Wasserstrahlvakuum eingedampft. Durch anschliessendes Chromatographieren an Kieselgel mit Toluol/Essigester (9:1)-Gemisch gefolgt von Kristallisation der einheitlichen Fraktionen wird die Titelverbindung rein erhalten.

c) mit Tetrabutylammoniumfluorid:
Eine Lösung von 300 mg (0,95 mMol) Tetrabutylammoniumfluorid-trihydrat in 4 ml Tetrahydrofuran wird zur Entwässerung mit aktivierten Molekularsieben ($4x10^{-10}$m) versetzt und unter Argon 18 Stunden bei 0° belassen. Dann wird unter Rühren bei 0° eine Lösung von 206 mg (0,63 mMol) (2R,3R)-2,3-Epoxybuttersäure-N-p-methoxyphenyl-N-phenacylamid in 2 ml abs. Tetrahydrofuran zugetropft und das Reaktionsgemisch bei 0° 2 Stunden weitergerührt. Die Molekularsiebe werden abfiltriert, am Filter mit Tetrahydrofuran nachgewaschen, die vereinigten Filtrate im Vakuum eingedampft und der Rückstand, in Methylenchlorid gelöst, nacheinander mit 1N $H_2SO_4$ und Wasser gewaschen. Die wässrigen Anteile werden mit Methylenchlorid nachextrahiert. Die vereinigten organischen Anteile ergeben, nach Trocknen und Eindampfen im Vakuum ein Rohprodukt, welches an Kieselgelplatten mit Toluol/Ethylacetat (9:1) chromatographiert wird. Man erhält so die Titelverbindung mit den oben beschriebenen Daten.

d) mit Kaliumcarbonat: Eine Lösung von 13,0 g (2R,3R)-2,3-Epoxybuttersäure-N-p-methoxyphenyl-N-phenacylamid in 74 ml Dimethylsulfoxid wird mit 55,2 mg Kaliumcarbonat versetzt und unter Argon-Atmosphäre 6 Stunden bei 21 bis 24° gerührt. Das Reaktionsgemisch wird auf 1,6 l Eiswasser gegossen und während 2 Stunden gerührt. Der Niederschlag wird abgenutscht, mit Wasser gewaschen und in Methylenchlorid aufgenommen. Die organische Phase wird vom Wasser abgetrennt, mit gesättigter, wässriger Kochsalzlösung gewaschen, getrocknet und eingedampft. Das erhaltene Rohprodukt wird auf einer Kieselgel-Säule chromatographiert. Durch Elution mit Hexan/Ethylacetat (7:3)-Gemisch erhält man eine kleine Menge chromatographisch einheitliches (3S,4R,1'R)-N-p-Methoxyphenyl-3-(1'-hydroxyethyl)-4-benzoylazetidin-2-on, welches nach Kristallisation aus Methylenchlorid-Diisopropyläther bei 168-170° schmilzt. Weiteres Eluieren mit Hexan/Ethylacetat (1:1)-Gemisch ergibt das chromatographisch einheitliche (3S,4S,1'R)-N-p-Methoxyphenyl-3-(1'-hydroxyethyl)-4-benzoylazetidin-2-on, welches nach Kristallisation aus Methylenchlorid-Diisopropyläther bei 111-113° schmilzt.

e) mit 1,5-Diazabicyclo[5.4.0]undec-5-en: Analog d) wird die gleiche Verbindung erhalten, wenn, unter sonst gleichen Bedingungen, anstelle von Kaliumcarbonat die äquivalente Menge 1,5-Diazabicyclo[5.4.0]-undec-5-en eingesetzt wird.

f) durch Isomerisierung des cis-Isomeren mit $K_2CO_3$: Das bei der Zyklisierung von (2R,3R)-2,3-Epoxybuttersäure-N-p-methoxyphenyl-N-phenacylamid als Nebenprodukt in kleinen Mengen anfallende (3S,4R)-Isomere der Titelverbindung (Beispiel 3d) kann zur Titelverbindung isomerisiert werden, indem man 3,9 g dieser cis-Verbindung in 80 ml Dimethylformamid mit 6,6 g Kaliumcarbonat 2,5 Stunden bei 60° (Bad-Temperatur) rührt. Das anorganische Salz wird abfiltriert, das Filtrat unter vermindertem Druck eingeengt, der Rückstand in Methylenchlorid aufgenommen, die so entstandene Lösung mit Wasser und mit wässriger Kochsalz-Lösung gewaschen, getrocknet, im Vakuum eingeengt, und der Rückstand an einer Kieselgel-Säule chromatographiert. Mit Hexan-Ethylacetat (2:1)-Gemisch wird zuerst eine kleine

Menge des (3S,4R)-Isomeren eluiert, gefolgt nach Eluieren, mit Hexan-Ethylacetat 1:1-Gemisch, von der reinen Titelverbindung, die nach Kristallisation aus Methylenchlorid/Diisopropylether bei 111-113° schmilzt.

g) durch Isomerisierung des cis-Isomeren mit NaOH: In einer zweiphasigen Mischung gleicher Volumina Methylenchlorid und 1N Natronlauge und in Gegenwart von 60 mg Tetrabutylammoniumhydrogensulfat werden 3,9 g (3S,4R,1'R)-N-p-Methoxyphenyl-3-(1'-hydroxyethyl)-4-benzoylazetidin-2-on (cis-Isomer) während 2 Stunden bei 21° gerührt. Die Natronlauge wird abgetrennt und die organische Phase nach Waschen mit Wasser getrocknet und im Vakuum eingedampft. Der Rückstand enthaltend ein (7:3)-Gemisch der (3S,4S)- und (3S,4R)-Verbindung wird chromatographisch mit Toluol-Ethylacetat an Kieselgel in die reinen Isomeren aufgetrennt. Nach Umkristallisation der die (3S,4S)-Verbindung enthaltenden und eingedampften Fraktionen erhält man die reine Titelverbindung, F. 111-113°.

Beispiel 4: (3R,4R,1'R)-N-p-Methoxyphenyl-3-(1'-hydroxyethyl)-4-benzoyloxyazetidin-2-on:

Eine Lösung von 416 mg (3S,4S,1'R)-N-p-Methoxyphenyl-3-(1'-hydroxyethyl)-4-benzoylazetidinon in 20 ml Methylenchlorid wird mit 1,27 g m-Chlorperbenzoesäure versetzt und 72 Stunden im Bombenrohr auf 45-50° erwärmt. Das Reaktionsgemisch wird auf Eiswasser gegossen und mit Methylenchlorid verdünnt. Die organische Phase wäscht man nacheinander mit Kaliumiodid-Natriumthiosulfat-, eiskalter gesättigter Natriumhydrogencarbonat-Lösung und Wasser, trocknet und dampft am Rotationsverdampfer ein. Nach Chromatographieren an Kieselgel (Elutionsmittel: Toluol/Essigester 9:1-Gemisch) wird das reine (3R,4R,1'R)-N-p-Methoxyphenyl-3-(1'-hydroxyethyl)-4-benzoyloxyazetidin-2-on erhalten. IR.: Banden u.a. bei 3600, 1765, 1725, 1600, 1515, 1450, 1240, 1180, 1070 und 1025. NMR.: Signale u.a. bei 1,42 (d), 3,38 (d), 3,7 (s), 4,30 (m), 6,52/7,42 (AA'BB').

Dieselbe Verbindung wird unter analogen Bedingungen auch bei Verwendung von p-Nitroperbenzoesäure oder mit Peressigsäure in Essigsäure oder Essigester als Lösungsmittel erhalten.

Beispiel 5: (3R,4R,1'R)-3-(1'-Hydroxyethyl)-4-benzoyloxyazetidin-2-on:

Eine auf 0° abgekühlte Lösung von 325 mg (3R,4R,1'R)-N-p-Methoxyphenyl-3-(1'-hydroxyethyl)-4-benzoyloxyazetidinon in 15 ml Acetonitril wird mit einer Lösung von 1,642 g Cer(IV)ammoniumnitrat in 15 ml Wasser versetzt und 30 Minuten bei -5 bis 0° gerührt. Das Reaktionsgemisch wird mit 200 ml Wasser verdünnt und dreimal mit Methylenchlorid extrahiert. Die organischen Phasen werden nacheinander mit verdünnter, wässriger Natriumhydrogencarbonat-, 10 %iger Natriumsulfit-, Natriumhydrogencarbonat-Lösung und Wasser gewaschen, getrocknet und eingedampft. Das erhaltene Rohprodukt liefert nach Filtration durch die 20-fache Gewichtsmenge Kieselgel (Elutionsmittel: Toluol/Essigester-8:2) und anschliessender Kristallisation der dünnschichtchromatographisch einheitlichen Fraktionen aus Chloroform-Petrolether das reine (3R,4R,1'R)-3-(1'-Hydroxyethyl)-4-benzoyloxyazetidin-2-on. F. 144,5-146°. [α] = +67,6 ± 2,4° (c = 0,414 %, $CHCl_3$).

Beispiel 6: (3S,4S,1'R)-3-(1'-Hydroxyethyl)-4-benzoylazetidin-2-on

a) Eine auf -18° abgekühlte Lösung von 325 mg (3S,4S,1'R)-N-p-Methoxyphenyl-3-(1'-hydroxyethyl)-4-benzoylazetidin-2-on in 10 ml Acetonitril wird mit einer Lösung von 2,2 g Cer(IV)ammoniumnitrat in 5 ml Wasser versetzt und 35 Minuten bei -16 bis -18° gerührt. Das Reaktionsgemisch wird auf Eis-Wasser gegossen und dreimal mit Ethylacetat extrahiert. Die organischen Phasen werden mit eiskalter Natriumhydrogencarbonat-Lösung gewaschen, getrocknet und eingedampft. Nach Umkristallisieren des Rohproduktes aus Methylenchlorid/Ether wird das reine, bei 132-134° schmelzende (3S,4S,1'R)-3-(1'-Hydroxyethyl)-4-benzoylazetidin-2-on erhalten.

b) durch Isomerisierung des cis-Isomeren mit 1,5-Diazabicyclo[5.4.0]undec-5-en: Eine Lösung von 660 mg (3S,4R,1'R)-3-(1'-Hydroxyethyl)-4-benzoylazetidin-2-on in 14 ml Chloroform wird mit 0,05 ml 1,5-Diazabicyclo[5.4.0]undec-5-en versetzt und 15 Stunden auf 75° erwärmt. Das Reaktionsgemisch wird am Vakuum eingedampft und der Rückstand an einer Kieselgel-Säule chromatographiert. Mit Hexan-Ethylacetat (1:1) wird zuerst eine kleine Menge des (3S,4R)-Isomeren eluiert, gefolgt von dem (3S,4S)-Isomeren, das aus Methylenchlorid/Diisopropyläther kristallisiert wird. F. 131-135°.

Das als Ausgangsstoff verwendete (3S,4R,1'R)-(1'-Hydroxyethyl)-4-benzoylazetidinon-2-on kann folgendermassen hergestellt werden:

Eine auf -18° abgekühlte Suspension von 9,7 g (3S,4R,1'R)-N-p-Methoxyphenyl-3-(1'-hydroxyethyl)-4-benzoylazetidin-2-on (cis-Isomer aus Beispiel 3d) in 225 ml Acetonitril wird mit einer Lösung von 36,3 g

Cer(IV)ammoniumnitrat in 150 ml Wasser versetzt und 30 Minuten bei -15° bis -5° gerührt. Das Reaktionsgemisch wird mit 800 ml Eiswasser verdünnt und dreimal mit 600 ml Ethylacetat extrahiert. Die organischen Phasen werden nacheinander mit 1%iger Natriumsulfit- und Natriumchloridlösung gewaschen, mit Natriumsulfat getrocknet und eingedampft. Das erhaltene Rohprodukt liefert nach Kristallisation aus Methylenchlorid/Diisopropyläther das reine (3S,4R,1'R)-3-(1'-Hydroxyethyl-4-benzoylazetidin-2-on. F. 145-147°.

Beispiel 7: (3R,4R,1'R)-3-(1-Hydroxyethyl)-4-benzoyloxyazetidin-2-on

a) mit m-Chlorperbenzoesäure: Eine Lösung von 1,9 g (3S,4S,1'R)-3-(1'-Hydroxyethyl)-4-benzoylazetidinon in 172 ml Methylenchlorid wird mit 10,1 g 85 %iger m-Chlorperbenzoesäure versetzt und 165 Minuten bei ca. Raumtemperatur gerührt. Das Reaktionsgemisch wird auf Eis-Wasser gegossen und zweimal mit Methylenchlorid extrahiert. Die organischen Phasen werden nacheinander mit wässriger Kaliumjodid/Natriumthiosulfat- und Natriumhydrogencarbonat-Lösung und anschliessend mit Wasser gewaschen, getrocknet und eingedampft. Nach Filtration des anfallenden Rohproduktes in Toluol/Ethylacetat-(8:2)-Gemisch über 300 g Kieselgel und anschliessender Kristallisation der einheitlichen Fraktionen aus Methylenchlorid/Ether wird das reine (3R,4R,1'R)-3-(1-Hydroxyethyl)-4-benzoyloxyazetidin-2-on vom Schmelzpunkt 145-147° erhalten. Die Verbindung ist bezüglich IR-und NMR-Spektrum und Optischer Drehung mit dem im Beispiel 5 beschriebenen Präparat identisch.
b) mit Peressigsäure: Die Titelverbindung kann auch hergestellt werden, indem man 4,27 g (3S,4S,1'R)-3-(1-Hydroxyethyl)-4-benzoylazetidin-2-on in 60 ml Methylenchlorid mit 12,2 ml einer 30 %-igen Peressigsäure-Lösung in Essigsäure versetzt und 6 Stunden unter Rückfluss erhitzt. Das entstandene Reaktionsgemisch wird mit Methylenchlorid verdünnt und nacheinander mit 5 %iger wässriger $NaHSO_3$-, 8 %-iger wässriger $NaHCO_3$- und gesättigter wässriger Kochsalz-Lösung gewaschen. Die wässrigen Anteile werden mit Methylenchlorid nachextrahiert, die vereinigten organischen Anteile über $Na_2SO_4$ getrocknet und eingedampft. Das kristalline Rohprodukt wird direkt aus Methylenchlorid/Ether umkristallisiert und ergibt die reine Titelverbindung mit den oben beschriebenen Daten.
c) mit Magnesium-monoperphthalat-hexahydrat: Eine Lösung von 5 g (3S,4S,1'R)-3-(1'-Hydroxyethyl)-4-benzoylazetidin-2-on in 200 ml Ethylacetat wird mit einer Lösung von 16,5 g Magnesium-monoperphthalat-hexahydrat in 60 ml Ethanol (96 %-ig) versetzt und das entstandene Reaktionsgemisch 5 Stunden bei 50° Badtemperatur gerührt. Dabei scheidet sich ein kristalliner Niederschlag ab. Die Suspension wird mit Methylenchlorid und 5 %iger wässriger Natriumsulfit-Lösung versetzt und die organische Phase nachher noch mit 8 %iger wässriger Natrumbicarbonat-Lösung gewaschen. Die wässrigen Anteile werden mit Methylenchlorid nachextrahiert, die organischen Extrakte vereinigt, eingedampft und der Rückstand aus Methylenchlorid/Pentan umkristallisiert. Man erhält die reine Titelverbindung mit den oben erwähnten Eigenschaften.
d) mit Wasserstoffperoxid und Trichloracetonitril: Die Titelverbindung kann auch hergestellt werden durch Umsatz von 11 g (3S,4S,1'R)-3-(1'-Hydroxyethyl)-4-benzoylazetidin-2-on mit 19 ml 30%igem Wasserstoffperoxid und 21 ml Trichloracetonitril in Gegenwart von 10 g Kaliumhydrogenphosphat in 200 ml Chloroform analog zu Beispiel 13c).

Beispiel 8: (3S,4S,1'R)-N-p-Methoxyphenyl-3-[1'-(2,2,2-trichlorethoxycarbonyloxy)ethyl]-4-benzoylazetidin-2-on:

Eine auf 0° abgekühlte Lösung von 325 mg (3S,4S,1'R)-N-p-Methoxyphenyl-3-(1'-hydroxyethyl)-4-benzoylazetidin-2-on in 5 ml Methylenchlorid wird unter Argon mit 0,18 ml Triethylamin, 0,18 ml Chlorameisensäure-2,2,2-trichlorethylester und 130 mg Dimethylaminopyridin versetzt und 7 Stunden bei gleicher Temperatur gerührt. Das Reaktionsgemisch wird auf Eis-Wasser gegossen und zweimal mit Methylenchlorid extrahiert. Die organischen Phasen werden mit 5 %iger, wässriger Citronensäure-Lösung und mit eiskalter, wässriger Natriumhydrogencarbonat-Lösung gewaschen, getrocknet und eingedampft. Durch Filtration des Rückstandes über Kieselgel (Toluol/Ethylacetat-9:1) wird das reine (3S,4S,1'R)-N-p-Methoxyphenyl-3-[1'-(2,2,2-trichlorethoxycarbonyloyy)-ethyl]-4-benzoylazetidin-2-on als farbloser Schaum erhalten. IR: Banden u.a. bei 1760, 1690, 1512, 1380, 1240 und 1183 cm; [α] = -9,5° (c = 0,980 %, Chloroform].

Beispiel 9: (3S,4S,1'R)-3-[1'-(2,2,2-Trichlorethoxycarbonyloxy)ethyl]-4-benzoylazetidin-2-on:

Eine auf -18° abgekühlte Lösung von 400 mg (3S,4S,1'R-)-N-p-Methoxyphenyl-3-[1'-(2,2,2-trichlorethoxycarbonyloxy)ethyl]-4-benzoylazetidin-2-on in 10 ml Acetonitril wird mit einer Lösung von 965 mg Cer-(IV)ammoniumnitrat in 5 ml Wasser versetzt und innerhalb 2 1/2 Stunden unter Rühren auf -5° erwärmt. Das Reaktionsgemisch wird auf Eis-Wasser gegossen und zweimal mit Ethylacetat extrahiert. Die organischen Phasen werden zweimal mit eiskalter, wässriger Natriumhydrogencarbonat-Lösung gewaschen, getrocknet und eingedampft. Durch Kristallisation des anfallenden Rohproduktes aus Methylenchlorid/Ether/Petrolether wird das reine, bei 106-108° schmelzende (3S,4S,1'R)-3-[1'-(2,2,2-Trichlorethoxycarbonyloxy)ethyl]-4-benzoylazetidin-2-on erhalten.

Beispiel 10: (3R,4R,1'R)-3-[1'-(2,2,2-Trichlorethoxycarbonyloxy)ethyl]-4-benzoyloxyazetidin-2-on:

Eine Lösung von 230 mg (3S,4S,1'R)-3-[1'-(2,2,2-Trichlorethoxycarbonyloxy)ethyl]-4-benzoylazetidinon in 20 ml Methylenchlorid wird mit 683 mg 85 %iger m-Chlorperbenzoesäure versetzt und 3 1/2 Stunden bei Raumtemperatur gerührt. Das Reaktionsgemisch wird auf Eis-Wasser gegossen und zweimal mit Methylenchlorid extrahiert. Die organischen Phasen werden nacheinander mit wässriger Kaliumjodid/Natriumthiosulfat-Lösung, Wasser, eiskalter, wässriger Natriumhydrogencarbonat-Lösung und Wasser gewaschen, getrocknet und eingedampft. Das so erhaltene Rohprodukt liefert nach Filtration über Kieselgel (Toluol und Toluol/Ethylacetat-8:2) und anschliessender Kristallisation der einheitlichen Fraktionen aus Methylenchlorid/Ether/Petrolether das reine (3R,4R,1'R)-3-[1'-(2,2,2-Trichlorethoxycarbonyloxy)ethyl]-4-benzoyloxyazetidin-2-on vom Schmelzpunkt 118-120°.
[α] = +75° (c = 1,01 %, Chloroform).

Beispiel 11: (3S,4S,1'R)-N-p-Methoxyphenyl-3-(1'-allyloxycarbonyloxyethyl)-4-benzoylazetidin-2-on

Eine auf 0° abgekühlte Lösung von 325 mg (3S,4S,1'R)-N-p-Methoxyphenyl-3-(1'-hydroxyethyl)-4-benzoylazetidinon in 5 ml Methylenchlorid wird unter Argon mit 0,18 ml Triethylamin, 0,16 ml Chlorameisensäureallylester und 130 mg Dimethylaminopyridin versetzt und 1 Stunde bei gleicher Temperatur gerührt. Nach erneuter Zugabe der angegebenen Mengen von Triethylamin, Chlorameisensäureallylester und Dimethylaminopyridin wird weitere 17 Stunden bei 0° gerührt, dann noch eine dritte Portion gleicher Mengen der drei Reagenzien zugefügt und 24 Stunden bei Raumtemperatur gerührt. Das Reaktionsgemisch wird auf Eis-Wasser gegossen und zweimal mit Methylenchlorid extrahiert. Die organischen Phasen werden mit 5 %iger, wässriger Citronensäure-Lösung und mit eiskalter, wässriger Natriumhydrogencarbonat-Lösung gewaschen, getrocknet und eingedampft. Nach Filtration des Rückstandes über 40 g Kieselgel (Toluol/Ethylacetat-9:1) wird das reine (3S,4S,1'R)-N-p-Methoxyphenyl-3-(1'-allyloxycarbonyloxyethyl)-4-benzoylazetidin-2-on erhalten.
F. 78-80°.

Beispiel 12: (3S,4S,1'R)-3-(1'-Allyloxycarbonyloxyethyl)-4-benzoylazetidin-2-on:

Eine auf -18° abgekühlte Lösung von 270 mg (3S,4S,1'R)-N-p-Methoxyphenyl-3-(1'-allyloxycarbonyloxyethyl)-4-benzoylazetidin-2-on in 10 ml Acetonitril wird mit einer Lösung von 800 mg Cer(IV)-ammoniumnitrat in 5 ml Wasser versetzt und innerhalb 3 Stunden unter Rühren auf 0° erwärmt. Das Reaktionsgemisch wird auf Eis-Wasser gegossen und zweimal mit Essigester extrahiert. Die organischen Phasen werden zweimal mit eiskalter, wässriger Natriumhydrogencarbonat-Lösung gewaschen, getrocknet und eingedampft. Durch Filtration des anfallenden Rohproduktes über 20 g Kieselgel (Toluol/Essigester-8:2) und anschliessender Kristallisation der reinen Fraktionen aus Methylenchlorid/Ether/Petrolether wird das reine, bei 93-95° schmelzende (3S,4S,1'R)-3-(1'-Allyloxycarbonyloxyethyl)-4-benzoylazetidin-2-on erhalten.

Beispiel 13: (3R,4R,1'R)-3-(1'-Allyloxycarbonyloxyethyl)-4-benzoyloxyazetidin-2-on

a) mit m-Chlorperbenzoesäure: Eine Lösung von 60 mg (3S,4S,1'R)-3-(1'-Allyloxycarbonyloxyethyl)-4-benzoylazetidin-2-on in 5 ml Methylenchlorid wird mit 234 mg 85 %iger m-Chlorperbenzoesäure versetzt und 3 1/2 Stunden bei Raumtemperatur gerührt. Das Reaktionsgemisch wird auf Eis-Wasser gegossen und zweimal mit Methylenchlorid extrahiert. Die organischen Phasen werden nacheinander mit wässriger Kaliumjodid/Natriumthiosulfat-Lösung, Wasser, eiskalter wässriger Natriumhydrogencarbonat-Lösung und Wasser gewaschen, getrocknet und eingedampft. Das so erhaltene Rohprodukt liefert nach Filtration

über Kieselgel (Toluol und Toluol/Ethylacetat-8:2) und anschliessender Kristallisation der einheitlichen Fraktionen aus Methylenchlorid/ Ether/Petrolether das reine (3R,4R,1'R)-3-(1'-Allyloxycarbonyloxyethyl)-4-benzoyloxyazetidin-2-on vom Schmelzpunkt 84-85°.

b) mit Peressigsäure: Die Titelverbindung kann auch hergestellt werden, indem man eine Lösung von 91 mg (3S,4S,1'R)-3-(1'-Allyloxycarbonyloxyethyl)-4-benzoylazetidin-2-on in 5 ml Methylenchlorid in drei Portionen mit insgesamt 0,4 ml 30 %-iger Peressigsäure in Essigsäure versetzt und 48 Stunden bei Raumtemperatur rührt. Das erhaltene Reaktionsgemisch wird in Methylenchlorid nacheinander mit 5 %iger wässriger Natriumsulfit-, 8 %iger wässriger Natriumbicarbonat- und wässriger Kochsalzlösung gewaschen. Die wässrigen Anteile werden mit Methylenchlorid nachextrahiert, und die vereinigten organischen Extrakte, nach Trocknen mit $Na_2SO_4$, eingedampft. Nach Chromatographieren des erhaltenen Rückstandes an Kieselgel-Platten mit Hexan/Ethylacetat (1:1) erhält man die reine Titelverbindung mit den oben erwähnten Eigenschaften.

c) mit Wasserstoffperoxid und Trichloracetonitril: Die Titelverbindung kann ebenfalls hergestellt werden, indem man eine Lösung von 152 mg (3S,4S,1'R)-3-(1'-Allyloxycarbonyloxyethyl)-4-benzoylazetidin-2-on in 3 ml Chloroform bei Raumtemperatur mit 0,3 ml Trichloracetonitril, 1,2 ml 30 %igem wässrigem Wasserstoffperoxid und 138 mg Kaliumhydrogenphosphat versetzt und das entstandene Gemisch 21 Stunden bei Raumtemperatur rührt. Nach Verdünnen Methylenchlorid wird nacheinander mit 5 %iger wässriger Natrumbisulfit-, 8 %iger wässriger Natriumbicarbonat- und wässriger Kochsalz-Lösung gewaschen. Die wässrigen Anteile werden mit Methylenchlorid nachextrahiert, und die vereinigten organischen Extrakte, nach Trocknen über $Na_2SO_4$, eingedampft. Nach Chromatographieren an Kieselgel-Platten in Hexan/Ethylacetat (1:1) erhält man die reine Titelverbindung mit den oben beschriebenen Eigenschaften.

Beispiel 14: (3S,4R,1'R)-3-(1'-Allyloxycarbonyloxyethyl)-4-N-allyloxycarbonylglycylthioazetidin-2-on

26,75 g (75 mMol) N-Allyloxycarbonylthioglycin-dicyclohexylammoniumsalz werden in 200 ml Wasser und 75 ml 1N NaOH gelöst und dreimal mit je 100 ml Methylenchlorid extrahiert. Die wässrige Phase wird auf 0° gekühlt und unter pH Kontrolle (Glaselektrode) durch Zugabe von 1N NaOH auf pH 10,0 eingestellt. Unter kräftigem Rühren unter Argon wird eine Lösung von 8 g (25 mMol) (3R,4S,1'R)-3-(1'-Allyloxycarbonyloxyethyl)-4-benzoyloxyazetidin-2-on in 275 ml Aceton zugetropft, wobei die Temperatur unter 4° gehalten wird. Nach Beendigung der Zugabe wird weitere 15 Minuten bei 0-5° gerührt, die Lösung im Rotationsverdampfer auf 300 ml konzentriert und 4 mal mit je 100 ml Methylenchlorid extrahiert. Die vereinigten organischen Phasen werden noch zweimal mit je 100 ml Wasser und einmal mit 100 ml wässriger, gesättigter Natriumchloridlösung gewaschen, über $Na_2SO_4$ getrocknet und eingedampft. Der erhaltene Rückstand wird an 500 g Merck-Kieselgel im Flash Verfahren mit $CHCl_3$/Aceton (9:1) chromatographiert. Man erhält die Titelverbindung vom Rf-Wert (Merck Fertigplatten $CHCl_3$/Aceton 9:1):0,20.

Beispiel 15: (5R,6S,1'R)-6-(1'-Allyloxycarbonyloxyethyl)-2-allyloxycarbonylaminomethyl-2-penem-3-carbonsäureallylester

Eine Lösung von 930 mg (2,5 mMol) (3S,4R,1'R)-3-(1'-Allyloxycarbonyloxyethyl)-4-N-allyloxycarbonylglycylthioazetidin-2-on in 15 ml Methylenchlorid (frisch Alox-filtriert) wird unter Rühren in Argon-Atmosphäre bei -15° nacheinander mit 557 mg (3,75 mMol; 1,5 Aequiv.), Oxalsäureallylesterchlorid und 0.64 ml (3,75 mMol) (1,5 Aequiv.) Hünig-Base versetzt und 30 Minuten bei -15° weitergerührt. Zum Aufarbeiten wird das Reaktionsgemisch mit Methylenchlorid verdünnt und nacheinander mit kalter 0,1 N HCl und (zweimal) mit kalter, gesättigter $NaHCO_3$-Lösung kräftig und kurz durchgeschüttelt (die letzte Behandlung entfernt das überschüssige Allyloxyoxalylchlorid). Die wässrigen Anteile werden einmal mit Methylenchlorid nachextrahiert, und die vereinigten organischen Phasen mit $Na_2SO_4$ getrocknet und im Vakuum eingedampft. Das Rohprodukt wird mehrmals in Toluol aufgenommen, eingedampft und im Hochvakuum getrocknet. Der erhaltene dickflüssige Rückstand, enthaltend 2-[(3S,4R,1'R)-3-(1'-Allyloxycarbonyloxyethyl)-4-N-allyloxycarbonylglycylthio-2-oxo-1-azetidinyl]-2-oxoessigsäureallylester, wird zur Zyklisierung in 25 ml abs. Toluol gelöst, bei Raumtemperatur unter Rühren (Argon) mit 1,044 ml (6 mMol; 2,4 Aequiv.) destilliertem Triethylphosphit versetzt und das Reaktionsgemisch schnell in ein auf 100° vorgeheiztes Oelbad getaucht und unter Argon 7 Stunden bei gleicher Temperatur gerührt. Das Reaktionsgemisch wird im Vakuum eingedampft und der Rückstand an 100 g Merck Kieselgel (0,04-0,063 mm Korngrösse) im Flash-Verfahren chromatographiert. Es wird zuerst eine Vorfraktion von 500 ml Toluol/Essigester (9:1) und eine 150 ml Fraktion mit Toluol/Essigester (4:1) genommen. Die Titelverbindung wird in den folgenden 25 ml-Fraktionen mit Toluol/Essigester (4:1) eluiert, wobei eine kleine Menge einer mobileren Komponente (das entsprechende cis-Isomere) in den ersten Fraktionen abgetrennt werden kann. F. 58-59° (aus Ether-Pentan);
Rf (Merck Fertigplatten, Toluol/Essigester 2:1): 0,40;
[α] = +127,7° (1,05 % in $CH_2Cl_2$).

Beispiel 16: (5R,6S,1'R)-2-Aminomethyl-6-(1-hydroxyethyl)-2-penem-3-carbonsäure

Durch eine Lösung von 100 mg (0,22 mMol) (5R,6S,1'R)-2-Allyloxycarbonylaminomethyl-6-(1-allyloxycarbonyloxyethyl)-2-penem-3-carbonsäureallylester, 308 mg (2,2 mMol) Dimedon und 30 mg (0,11 mMol) Triphenylphosphin in 4 ml Tetrahydrofuran wird während 5 Minuten mit Argon geleitet. Dann werden bei Raumtemperatur 22 mg (0,019 mMol) Tetrakis-triphenylphosphin-palladium zugegeben. Nach 5 Minuten beginnt ein Niederschlag auszufallen. Die Suspension wird insgesamt 1 Stunde bei Raumtemperatur unter Argon gerührt. Das ausgefallene Produkt wird abfiltriert, mit Tetrahydrofuran, Ethylacetat und Hexan gewaschen und im Hochvakuum getrocknet.
Rf ($H_2O$, OPTI $UPC_{12}$): = 0,48.

50 mg rohe (5R,6S,1'R)-2-Aminomethyl-6-(1-hydroxyethyl)-2-penem-3-carbonsäure wird in 0,5 ml doppelt destilliertem Wasser bei 30° gelöst. Die wässrige Lösung wird auf 5° abgekühlt, mit wenig Aktiv-Kohle versetzt, 15 Minuten gerührt und anschliessend klarfiltriert. Das klare Filtrat wird im Hochvakuum vollständig eingeengt und der Rückstand in 0,3 ml Aethanol (96 %-ig) aufgeschlämmt. Die Suspension wird bei Raumtemperatur während 30 Minuten stark gerührt. Die weissen Kristalle (Nadeln) werden abfiltriert und mit Ethanol (96 %ig) gewaschen. Das Produkt wird bei 20° im Hochvakuum getrocknet. F. 165° (Zers.).

Beispiel 17: (2R,3R)-N-Acetonyl-N-p-methoxyphenyl-2,3-epoxybuttersäureamid:

Zu einer auf -20° abgekühlten Lösung von 73,2 g N-Acetonyl-p-anisidin-hydrochlorid in 1700 ml Toluol werden unter Rühren (bei -15 bis -18°) nacheinander innerhalb 15 Minuten 70,2 g N,N-Dicyclohexylcarbodiimid, gelöst in 170 ml Toluol, und innerhalb 60 Minuten 103 g einer Lösung des Dicyclohexylammoniumsalzes der (2R,3R)-2,3-Epoxybuttersäure in 340 ml Dichlormethan zugetropft. Das Reaktionsgemisch lässt man innerhalb 45 Minuten auf 0° erwärmen und rührt weitere 18 1/2 Stunden bei 0-5°. Die Suspension wird abgenutscht, der Rückstand gründlich mit Toluol gewaschen, das Filtrat zweimal mit je 1 Liter Wasser gewaschen, getrocknet und im Wasserstrahlvakuum eingeengt. Aus dem rotbraunen, öligen Rohprodukt können nach Abkühlen auf ca. 4° Kristalle abgetrennt werden. Durch Flash-Chromatographie der Mutterlauge mit Hexan/Essigester-1:4-Gemisch wird, neben regeneriertem N-Acetonyl-p-anisidin, als Hauptprodukt in Form eines hellbraunen Oels das (2R,3R,)-N-Acetonyl-N-p-methoxyphenyl-2,3-epoxybuttersäureamid erhalten. Die Verbindung kann ohne weitere Reinigung direkt für die Zyklisation verwendet werden. IR: Banden u.a. bei 2840, 1735, 1670, 1510, 1240, 1220, 1170 und 840.

Beispiel 18: (3S,4S,1'R)-N-p-Methoxyphenyl-3-(1'-hydroxyethyl)-4-acetylazetidin-2-on:

Eine Lösung von 10,1 g (2R,3R)-N-Acetonyl-N-p-2,3-epoxybuttersäureamid in (200 ml) Dimethylformamid wird bei 60° mit 19.1 g Kaliumcarbonat versetzt und 13 Stunden bei gleicher Temperatur gerührt. Das abgekühlte Reaktionsgemisch wird von ungelöstem Kaliumcarbonat abfiltriert, mit 1 Liter Methylenchlorid versetzt und nacheinander fünfmal mit verdünnter, wässriger Natriumchlorid-Lösung gewaschen, getrocknet und eingedampft. Nach Flash-Chromatographie mit Hexan/Ethylacetat (2:3) an 700 g Kieselgel wird als Hauptprodukt das nach Umkristallisieren aus Methylenchlorid/Ether bei 91-92° schmelzende (3S,4S,1'R)-N-pMethoxyphenyl-3-(1'-hydroxyethyl)-4-acetylazetidin-2-on ([α] = -119,9° (c = 0,853 in Chloroform) und als Nebenprodukt (ca. 10 %) das (3S,4R,1'R)-N-p-Methoxyphenyl-3-(1'-hydroxyethyl)-4-acetylazetidin-2-on (F. 165-168°) erhalten.

Beispiel 19: (3S,4S,1'R)-N-p-Methoxyphenyl-3-(1'-tert-butyl-dimethylsilyloxyethyl)-4-acetylazetidin-2-on

Eine Lösung von 3,39 g (3S,4S,1'R)-N-p-Methoxyphenyl-3-(1'-hydroxyethyl)-4-acetylazetidin-2-on und 1,58 g Imidazol in 20 ml Dimethylformamid wird bei Raumtemperatur mit 2,33 g tert-Butyl-dimethylchlorsilan versetzt und bei Raumtemperatur 5 Stunden weitergerührt. Zur Aufarbeitung wird das Reaktionsgemisch auf Eis-Natriumhydrogencarbonat-Lösung gegossen, in Methylenchlorid aufgenommen und die organische Phase nacheinander mit Natriumhydrogencarbonat-, 1 %iger Citronensäure- und nochmals mit Natriumhydrogencarbonat-Lösung gewaschen. Nach Trocknen über Natriumsulfat und Eindampfen unter vermindertem Druck wird ein Rohprodukt erhalten, welches aus Petrolether umkristallisiert wird. Man erhält die Titelverbindung als farblose Kristalle, F. 82-84°. [α] = -116,3° (c = 0,374 % in $CHCl_3$).

Beispiel 20: (3S,4S,1'R)-3-(1'-tert-Butyldimethylsilyloxyethyl)-4-acetylazetidin-2-on

Zu einer auf 0° abgekühlten Lösung von 4,34 g (3S,4S,1'R)-N-p-Methoxyphenyl-3-(1'-tert-butyldimethylsilyloxyethyl)-4-acetylazetidin-2-on in 140 ml Acetonitril werden innerhalb 15 Minuten bei 0° 13,85 g Cerammoniumnitrat, gelöst in 70 ml Wasser, zugetropft. Man lässt das Reaktionsgemisch 2 Stunden bei Raumtemperatur rühren, giesst es dann auf Eiswasser, extrahiert mit Methylenchlorid, wäscht die organische Schicht zweimal mit wässriger Natriumhydrogencarbonat-Lösung und gesättigter Kochsalzlösung, trocknet und dampft im Wasserstrahlvakuum ein. Durch Flash-Chromatographie des dunkelbraunen Rohproduktes in Hexan/Essigester (3:2) an Kieselgel und anschliessende Kristallisation der einheitlichen Fraktionen aus Ether/Petrolether wird das reine (3S,4S,1'R)-3-(1'-tert-Butyldimethylsilyloxyethyl)-4-acetylazetidin-2-on, F. 74-75°, erhalten. [α] = -14,6° (c = 0,397 %; $CHCl_3$).

Beispiel 21: (3R,4R,1'R)-3-(1'-tert-Butyl-dimethylsilyloxy)ethyl-4-acetoxyazetidin-2-on

Eine Lösung von 1,4 g (3S,4S,1'R)-3-(1'-tert-Butyldimethylsilyloxyethyl)-4-acetylazetidin-2-on in 70 ml Methylenchlorid wird mit 5,75 g 85 %-iger m-Chlorperbenzoesäure versetzt und 2 Stunden bei Raumtemperatur gerührt. Das gelbe Reaktionsgemisch wird auf Eis gegossen, mit Methylenchlorid extrahiert, die organische Phase nacheinander mit wässriger Kaliumjodid/Natriumthiosulfat-Lösung, Wasser, wässriger Natriumhydrogencarbonat-Lösung und Wasser gewaschen und eingedampft. Durch Flash-Chromatographie des Rohproduktes in Hexan/Essigester (4:1) und anschliessende Kristallisation der einheitlichen Fraktionen aus tiefsiedendem Petrolether erhält man das reine (3R,4R,1'R)-3-(1'-(tert-Butyl-dimethylsilyloxyethyl)-4-acetoxyazetidin-2-on, F. 108-110°. [α] = +50,1° (c = 0,838 %; $CHCl_3$).

Beispiel 22: (3S,4S,1'R)-N-p-Methoxyphenyl-3-[1'-(dimethyl-2,3-dimethylbut-2-ylsilyloxy)ethyl]-4-benzoylazetidin-2-on

Eine Lösung von 4,53 g (3S,4S,1'R)-N-p-Methoxyphenyl-3-(1'-hydroxyethyl)-4-benzoylazetidin-2-on in 40 ml Dimethylformamid wird mit 1,7 g Imidazol und 3,3 ml Dimethyl-(2,3-dimethylbut-2-yl)-chlorsilan versetzt und 23 Stunden bei 25° gerührt. Nach Zugabe von weiteren 1,7 g Imidazol und 3,3 ml Dimethyl-(2,3-dimethylbut-2-yl)-chlorsilan wird weitere 22 Stunden gerührt. Das Reaktionsgemisch wird auf eiskalte, wässrige Natriumhydrogencarbonat-Lösung gegossen und mit Chloroform extrahiert. Die organische Phase wird nacheinander mit wässriger Natriumhydrogencarbonat-Lösung, 1 %-iger wässriger Citronensäure, wässriger Natriumhydrogencarbonat-Lösung und Wasser gewaschen, über Natriumsulfat getrocknet und im Vakuum eingedampft. Das anfallende Rohprodukt wird in Toluol/Ethylacetat (9:1) an 521 g Kieselgel "Flash"-chromatographiert. Die vereinigten, einheitlichen Fraktionen liefern nach Umlösen aus Ether/Petrolether die reine, bei 89-90° schmelzende (3S,4S,1'R)-N-p-Methoxyphenyl-3-[1'-(dimethyl-2,3-dimethylbut-2-ylsilyloxy)ethyl]-4-benzoylazetidin-2-on.
[α] = -65,4° (c = 0,506 %; $CHCl_3$).

Beispiel 23: (3S,4S,1'R)-3-[1'-(Dimethyl-2,3-dimethylbut-2-ylsilyloxy)ethyl]-4-benzoylazetidin-2-on:

Zu einer auf 0° abgekühlten Lösung von 37,02 g (3S,4S,1'R)-N-p-Methoxyphenyl-3-[1'-(dimethyl-2,3-dimethylbut-2-ylsilyloxy)ethyl]-4-benzoylazetidin-2-on in 980 ml Acetonitril wird unter Argon innert 15 Minuten eine Lösung von 95,4 g Cer(IV)ammoniumnitrat in 490 ml Wasser zugetropft und das Reaktionsgemisch 1 Stunde bei 0-5° gerührt. Die organische Phase wird darauf abgetrennt und im Vakuum bei 30° eingedampft. Der Rückstand wird unter Zugabe von Eis mit der wässrigen Phase vereinigt und das Gemisch mit Essigester extrahiert. Der Extrakt wird nacheinander zweimal mit wässriger Natriumhydrogencarbonat-Lösung und verdünnter wässriger Natriumchlorid-Lösung gewaschen, getrocknet und eingedampft. Der bräunliche Rückstand wird aus Methylenchlorid/Ether bei 5° kristallisiert. Die mit 50 ml Hexan/Ether (3:2)-Gemisch gewaschenen Kristalle werden mit zusätzlichen 50 ml dieses Gemisches bei 20° und anschliessend bei 0° verrührt, abgenutscht, mit etwas Hexan/Ether (3:2)-Gemisch gewaschen und bei 50° im Hochvakuum getrocknet. Man erhält die reine, bei 165-166° schmelzende Titelverbindrnng. Durch "Flash"-Chromatographie der Mutterlauge mit Hexan/Ethylacetat (80:20) an 750 g Kieselgel werden weitere Mengen der Titelverbindung erhalten. [α] = -20,4° (c = 0,499 %; $CHCl_3$).

Beispiel 24: (3R,4R,1'R)-3-[1'-(Dimethyl-2,3-dimethylbut-2-ylsilyloxy)ethyl]-4-benzoyloxyazetidin-2-on

Eine Lösung von 15,14 g (3R,4R,1'R)-3-[1'-(Dimethyl-2,3-dimethylbut-2-ylsilyloxy)ethyl]-4-benzoylazetidin-2-on in 500 ml Methylenchlorid wird mit 47,3 g 85 %-iger m-Chlorperbenzoesäure versetzt und 5 1/2 Stunden bei Raumtemperatur belassen. Das Reaktionsgemisch giesst man darauf auf Eiswasser, extrahiert mit Methylenchlorid nach wäscht die organische Phase nacheinander mit wässriger Kaliumjodid/Natriumthiosulfat-Lösung, Wasser, wässriger Natriumhydrogencarbonat-Lösung und Wasser, trocknet und dampft ein. Beim Versetzen des Rohproduktes mit Ether kristallisieren ca. 1,5 g 3-Chlorbenzoylperoxid aus. Die Kristalle werden abgenutscht und aus dem Filtrat nach Einengen aus Hexan das Rohprodukt kristallisiert. Die so erhaltene, leicht verunreinigte Titelverbindung wird mit Hexan/Ethylacetat (90:10) "Flash"-chromatographiert. Die einheitlichen Fraktionen liefern nach Umlösen aus Hexan die reine, bei 83-84° schmelzende (3R,4R,1'R)-3-[1'-(Dimethyl-2,3-dimethylbut-2-ylsilyloxy)ethyl]-4-benzoyloxyazetidin-2-on
[α] = +62,5° (c = 0,256 %; $CHCl_3$).

Beispiel 25: (3S,4R,1'R)-3-[1'-(Dimethyl-2,3-dimethylbut-2-ylsilyloxy)ethyl]-4-N-allyloxycarbonylglycylthioazetidin-2-on

2,67 g (7,5 Mol) N-Allyloxycarbonylthioglycin-Dicyclohexylammoniumsalz werden in 7,5 ml N NaOH und 20 ml Wasser gelöst. Die entstandene Lösung wird dreimal mit Methylenchlorid extrahiert, der wässrige Anteil mit 10 ml Aceton und bei 0° unter Rühren unter Argon mit einer Lösung von 944 mg (2,5 Mol) (3R,4R,1'R)-3-[1'-(Dimethyl-2,3-dimethylbut-2-ylsilyloxy)ethyl]-4-N-allyloxycarbonylglycylthioazetidin-2-on in 20 ml Aceton versetzt. Das Reaktionsgemisch wird bei einem pH von 10,5-10,7 während 5 Stunden unter Kühlen weitergerührt. Das Aceton wird unter vermindertem Druck abdestilliert, der wässrige Rückstand mit Methylenchlorid extrahiert, die organische Phase nach Trocknen über $Na_2SO_4$ eingedampft und der Rückstand an Kieselgelplatten in Hexan/Ethylacetat (1:1) chromatographiert. Man erhält die Titelverbindung als farblose, kristalline Verbindung, welche aus Ether-Hexan umkristallisiert wird. F. 74-76°.
[α] = +110,7 ± 1,3° (c = 0,76 % in $CHCl_3$).

Beispiel 26: (5R,6S,1'R)-6-[1'-(Dimethyl-2,3-dimethylbut-2-ylsilyloxy)ethyl]-2-allyloxycarbonylaminomethyl-2-penem-3-carbonsäureallylester

Eine Lösung von 692 mg (1,6 mMol) (3S,4R,1'R)-3-[1'-(Dimethyl-2,3-dimethylbut-2-ylsilyloxy)ethyl]-4-N-allyloxycarbonylglycylthioazetidin-2-on in 9 ml Methylenchlorid wird bei -20° unter Argon nacheinander mit 360 mg (ca. 2,41 mMol) Oxalsäureallylesterchlorid und 410 ml (ca. 2,41 mMol) Hünig-Base versetzt und das entstandene Reaktionsgemisch zuerst 2,5 Stunden bei -10° und anschliessend 80 Minuten bei 0° gerührt. Es wird mit Methylenchlorid verdünnt und nacheinander mit eiskalter 0,1N HCl- und 8 %iger wässriger $NaHCO_3$-Lösung gewaschen. Der organische Anteil wird über $Na_2SO_4$ getrocknet und eingedampft. Der erhaltene Rückstand wird in 3 ml Triethylphosphit gelöst und die Lösung 2 Stunden unter Argon auf 60° erwärmt. Die flüchtigen Anteile werden zunächst unter vermindertem Druck, dann im Hochvakuum entfernt, der Rückstand in 10 ml abs. Dioxan gelöst und die resultierende Lösung unter Argon 8 Stunden auf 105° (Badtemperatur) erwärmt. Das nach Eindampfen von Dioxan unter vermindertem Druck erhaltene Rohprodukt wird auf einer Kieselgelsäule mit Hexan/Ethylacetat (9:1 und 4:1) chromatographiert. Die Titelverbindung wird als farbloses Oel erhalten. [α] = +62 ± 2,7° (c = 0,371 % in $CHCl_3$), U.V.: $\lambda_{max}$ = 320 nm ($\epsilon$ = 6230; in Ethanol).

Beispiel 27: (5R,6S,1'R)-2-Aminomethyl-6-[1'-(dimethyl-2,3-dimethylbut-2-ylsilyloxy)ethyl]-2-penem-3-carbonsäure

Eine Lösung von 367 mg (0,72 mMol) (5R,6S,1'R)-6-[1'-Dimethyl-(2,3-dimethylbut-2-ylsilyloxy)ethyl]-2-allyloxycarbonylaminomethyl-2-penem-3-carbonsäureallylester in 7 ml abs. Tetrahydrofuran wird bei Raumtemperatur mit 223 mg (1,59 mMol) Dimedon und 36 mg Tetrakis-triphenylphosphin-palladium versetzt und die entstandene Lösung bei Raumtemperatur weitergerührt. Bald beginnt die Ausscheidung der kristallinen Titelverbindung und ist nach 1 Stunde praktisch beendet. Die Kristalle werden abfiltriert und auf dem Filter mit etwas Tetrahydrofuran nachgewaschen. Die erhaltene Titelverbindung bildet farblose Kristalle, F. 119°; UV: $\lambda_{max}$ (EtOH):309 nm, 268 nm.

Beispiel 28: (3S,4S,1'R)-N-p-Methoxyphenyl-3-[1'-(3,5-dinitrobenzoyloxy)ethyl]-4-benzoylazetidin-2-on

Zu einer Lösung von 6,16 g 3,5-Dinitrobenzoesäure in 83 ml Pyridin werden bei Raumtemperatur innerhalb 10 Minuten 10,07 g p-Toluolsulfonsäurechlorid gegeben. Anschliessend wird eine Stunde gerührt. Zu dem auf 0-5° gekühlten Reaktionsgemisch werden 9,01 g (27,7 mMol) (3S,4S,1'R)-N-p-Methoxyphenyl-3-(1'-hydroxyethyl)-4-benzoylazetidin-2-on gegeben und 1 1/4 Stunden bei gleicher Temperatur gerührt. Das Reaktionsgemisch wird auf 500 ml Methylenchlorid gegossen und mit 250 ml eiskalter 4N Salzsäure extrahiert. Die organische Phase wird einmal mit 100 ml gesättigter wässriger Natriumbicarbonat-Lösung und einmal mit 100 ml gesättigter wässriger Kochsalzlösung gewaschen und über Natriumsulfat getrocknet. Nach Eindampfen wird der rote Rückstand an 250 g Kieselgel mit Toluol/Ethylacetat (9:1) gereinigt. Man erhält in quantitativer Ausbeute die Titelverbindung mit den folgenden physikalischen Daten:
IR ($CH_2Cl_2$): Banden u.a. bei 3100, 1765, 1735, 1550, 1515, 1345, 1250; Rf (Merck Kieselgel Fertigplatten, Ethylacetat/Toluol 1:1): 0,65.

Beispiel 29: (3S,4S,1'R)-3-[1'-(3,5-Dinitrobenzoyloxy)ethyl]-4-benzoylazetidin-2-on

Zu einer Lösung von 14,88 g (27,8 mMol) (3S,4S,1'R)-N-p-Methoxyphenyl-3-[1'-(3,5-dinitrobenzoyloxy)-ethyl]-4-benzoylazetidin-2-on in 290 ml Acetonitril wird bei 0° innerhalb 15 Minuten eine Lösung von 34,4 g (62,8 mMol) Cer(IV)-ammoniumnitrat in 146 ml $H_2O$ getropft. Nach weiteren 30 Minuten Reaktionszeit bei obiger Temperatur wird mit 500 ml Essigester versetzt und die organische Phase einmal mit eiskalter wässriger Natriumbicarbonatlösung und zweimal mit halbgesättigter wässriger Kochsalzlösung gewaschen. Nach Trocknen über Natriumsulfat und Eindampfen wird das Rohprodukt aus Methylenchlorid/Ether kristallisiert und man erhält die Titelverbindung in reiner Form. Aus der Mutterlauge werden durch Eindampfen und Digerieren in Toluol weitere Mengen der Titelverbindung erhalten. F. 156-160°; Rf-Wert (Merck Kieselgel Fertigplatten, Ethylscetat/Toluol 1:1): 0,42.

Beispiel 30: (3R,4R,1'R)-3-[1'-(3,5-Dinitrobenzoyloxy)ethyl]-4-benzoyloxyazetidin-2-on

Eine Lösung von 8,30 g (20 mMol)(3S,4S,1'R)-3-[1'-(3,5-Dinitrobenzoyloxy)ethyl]-4-benzoylazetidin-2-on in 151 ml Methylenchlorid wird mit 6,129 g (30 mMol) m-Chlorperbenzoesäure (85 %-ig) versetzt und zuerst eine Stunde bei Raumtemperatur, dann 2 1/2 Stunden bei 30°C gerührt. Zur Aufarbeitung wird mit 250 ml Methylenchlorid versetzt und bei 0°C nacheinander mit 100 ml 10 %-iger wässriger Natriumbicarbonatlösung, ca. 5 %-iger wässriger Natriumbisulfitlösung und 100 ml gesättigter wässriger Kochlsalzlösung gewaschen. Nach Trocknen und Einengen wird das Rohprodukt aus 50 ml Pentan kristallisiert. Man erhält die Titelverbindung vom Schmelzpunkt 165-169° (Zers.) Rf (Merck Kieselgel Fertigplatten, Ethylacetat/Toluol 1:1): 0,58.

Beispiel 31: (3S,4R,1'R)-3-[1'-(3,5-Dinitrobenzoyloxy)ethyl]-4-N-allyloxycarbonylglycylthio)azetidin-2-on

3,77 g (10,6 mMol) N-Allyloxycarbonylthioglycin-dicyclohexylammoniumsalz werden in 18,7 ml $H_2O$ und 10,6 ml wässriger 1N NaOH gelöst und dreimal mit je 5 ml $CH_2Cl_2$ extrahiert. Mit ca. 0,2 ml 0,1N wässriger HCl die Lösung wird auf pH 7-8 gebracht und anschliessend zu einer Lösung von 2,28 g (5,3 mMol) (3R,4R,1'R)-3-[1'-(3,5-Dinitrobenzoyloxy)ethyl]-4-benzoyloxyazetidin-2-on in 50 ml Dioxan bei 23° gegeben. Es wird 1 1/2 Stunden bei 23° weitergerührt und mit 100 ml gesättigter, wässriger $NaHCO_3$-Lösung aufgearbeitet. Die organische Phase wird noch zweimal mit je 20 ml gesättigter, wässriger Kochsalzlösung gewaschen, über Natriumsulfat getrocknet und vom Lösungsmittel befreit. Der Rückstand wird an 200 g Kieselgel mit Methylenchlorid/Aceton (9:1) chromatographisch gereinigt, wobei die Titelverbindung als Schaum erhalten wird. Rf (Merck Fertigplatten, Toluol/Essigester 1:1): 0,38; $[\alpha] = +25°$ (c = 0,72; $CHCl_3$). Dieses Produkt enthält noch ca. 5 % des 3S,4S-cis-Isomeren.

Beispiel 32: (5R,6S,1'R)-6-[1'-(3,5-Dinitrobenzoyloxy)ethyl]-2-allyloxycarbonylaminomethyl-2-penem-3-carbonsäureallylester

Eine Lösung von 241 mg (0,5 mmol) (3S,4R,1'R)-3-[1'-(3,5-Dinitrobenzoyloxy)ethyl]-4-N-allyloxycarbonylglycylthioazetidin-2-on in 3 ml Methylenchlorid (frisch über Alox filtriert) wird unter Rühren und Feuchtigkeitsausschluss bei -20° nacheinander mit 84,5 µl (0,69 mMol) Oxalsäureallylesterchlorid und 124,2 µl (0,73 mmol) Hünig-Base versetzt und 30 Minuten bei -10° weitergerührt. Eine IR-Probe zeigt die charakteristische Oxalimid-Carbonyl-Absorption bei 1820 $cm^{-1}$. Zum Aufarbeiten wir das Reaktionsgemisch

mit 5 ml $CH_2Cl_2$ verdünnt und die organische Phase nacheinander mit eiskalter wässriger 0,1N HCl, 5 %-iger $NaHCO_3$-Lösung und gesättigter, wässriger Kochsalzlösung gewaschen, über $Na_2SO_4$ getrocknet und im Vakuum eingedampft. Nach Trocknen im Hochvakuum erhält man (3S,4R,1'R)-4-N-Allyloxycarbonylglycylthio)-3-[1'-(3,5-dinitrobenzoyloxy)ethyl]-azetidin-2-on-1-yloxalsäure allylester als beigen Schaum. Dieser wird in 2,5 ml Dioxan (Merck, p.A.) gelöst und unter $N_2$-Atomosphäre bei Raumtemperatur mit 312 µl (1,49 mMol) Triethylphosphit versetzt. Es wird 1 Stunde bei Raumtemperatur und 2 Stunden bei 40° Badtemperatur gerührt, bis im IR kein Oxalimid-Signal mehr festgestellt werden kann (Bande bei 1820 $cm^{-1}$). Nach Eindampfen wird dreimal mit ca. 1,5 ml Decan verrührt und im Hochvakuum jeweils wieder eingedampft. Das erhaltene rohe Phosphoran wird direkt zur Zyklisierung in 12,5 ml Dioxan (Merck, p.A. ) gelöst und 17 Stunden bei einer Badtemperatur von 105° gerührt. Anschliessend wird im Rotationsverdampfer eingeengt und der Rückstand an 30 g Kieselgel mit Toluol/Essigester = (4:1) chromatographiert. Aus den mittleren Fraktionen wird die Titelverbindung isoliert, die wiederum ca. 5 % 5S,6S-cis-Isomeres als Verunreinigung enthält. Rf (Merck Fertigplatten, Toluol/Essigester 4:1): 0,48 (cis-Produkt) und 0,38 (trans-Produkt).

Beispiel 33: (5R,6S,1'R)-6-(1'-Hydroxyethyl)-2-allyloxycarbonylaminomethyl-2-penem-3-carbonsäureallylester

a) Aus (5R,6S,1'R)-6-[1'-(3,5-Dinitrobenzoyloxy)ethyl]-2-allyloxycarbonylamino-methyl-2-penem-3-carbonsäureallylester:
Zu einer Lösung von 1,28 g (2,27 mMol) (5R,6S,1'R)-6-[1'-(3,5-Dinitrobenzoyloxy)ethyl]-2-allyloxycarbonylaminomethyl-2-penem-3-carbonsäureallylester (trans:cis ca. 95:5) in 100 ml Methanol und 20 ml Wasser werden bei 0° 5 ml gesättigte, wässrige Natriumbicarbonatlösung gegeben. Nach 20 Minuten Reaktionszeit bei 0° wird das Reaktionsgemisch auf 300 ml Essigsäurethylester und 50 ml gesättigte, wässrige Kochsalzlösung gegossen, die organische Phase abgetrennt und die wässrige Phase nochmals mit 100 ml Ethylacetat nachextrahiert. Die vereinigten organischen Phasen werden über $Na_2SO_4$ getrocknet und im Vakuum auf ca. 30 ml Volumen eingeengt. Es wird mit 150 ml Methylenchlorid verdünnt, nochmals mit $Na_2SO_4$ getrocknet und anschliessend im Rotationsverdampfer vollständig eingedampft. Der Rückstand wird an 80 g Kieselgel mit Toluol/Essigsäureethylester 4:1 bis 3:2 chromatographiert, wobei die Titelverbindung nach Kristallisation aus Ether/Hexan (3:7) erhalten wird, F. 141-141,5°. Rf (Merck Fertigplatten, Toluol/Essigsäureethylester 1:1): 0,20.

b) Aus (5R,6S,1'R)-6-[1'-(Dimethyl-2,3-dimethylbut-2-ylsilyloxy)ethyl]-2-allyloxycarbonylaminomethyl-2-penem-3-carbonsäureallylester:
Eine Lösung von 50 mg (5R,6S,1'R)-6-[1'-(Dimethyl-2,3-dimethylbut-2-ylsilyloxy)ethyl]-2-allyloxycarbonylaminomethyl-2-penem-3-carbonsäureallylester in 1 ml 2,6-Lutidin wird mit 60 mg (ca. 30mMol) einer 70 %igen HF-Harnstoff-Mischung versetzt und 2 Stunden bei Raumtemperatur gerührt. Dann werden zur Reaktionslösung weitere 5 Tropfen (ca. 100 mg = ca. 50 mM) HF-Harnstoff zugefügt. Das viskose Gemisch wird mit 1 ml Methylenchlorid verdünnt, über Nacht bei Raumtemperatur gerührt und erneut mit 10 Tropfen HF-Harnstoff (ca. 100 mM) versetzt. Nach weiteren 24 Stunden Reaktionszeit bei Raumtemperatur wird das Reaktionsgemisch mit Wasser verdünnt und mit Essigester extrahiert. Die organische Phase wird anschliessend mit 4N Salzsäure und mit gesättigter, wässriger Natriumchlorid-Lösung gewaschen, getrocknet und im Rotationsverdampfer eingeengt. Durch Umlösen des anfallenden Rohproduktes aus Ether/Hexan wird die reine Titelverbindung gewonnen, die mit der unter a) erhaltenen Probe identisch ist.

Beispiel 34: (3S,4R,1'R)-3-(1'-Hydroxyethyl)-4-(N-allyloxycarbonylglycylthio)-2-azetidinon

Eine Lösung von 14,2 g (40 mmol) N-Allyloxycarbonylthioglycindicyclohexylammoniumsalz in 60 ml Wasser und 40 ml 1 N wässriger NaOH Lösung wird dreimal mit je 20 ml Methylenchlorid extrahiert und mit ca. 1 ml 0,1 N wässriger HCl der pH auf 7 - 8 gestellt. Die erhaltene wässrige Thiolsäurelösung wird bei 30° innert 5 Minuten zu einer Lösung von 4,70 g (20 mmol) (3R,4R,1'R)-3-(1'-Hydroxyethyl)-4-benzoyloxy-2-azetidinon in 100 ml Acetonitril gegeben. Bei einer Innentemperatur von 25° wird 2 ml 0,1 N wässrige NaOH Lösung zugegeben und 30 - 35 Min bei 25° weitergerührt. Zur Aufarbeitung werden in einem Scheidetrichter 250 ml Essigsäureethylester und 30 g NaCl vorgelegt und dazu das Reaktionsgemisch gegeben. Nach gutem Schütteln und Abtrennen der wässrigen Phase wird die organische Phase noch einmal mit 50 ml 5 %-iger wässriger $NaHCO_3$-Lösung und zweimal mit 50 ml Sole gewaschen und über Natriumsulfat getrocknet. Das Lösungsmittel wird am Rotationsverdampfer entfernt und man erhält die Titelverbindung als amorphes Pulver. Das Rohprodukt kann an Kieselgel chromatographisch gereinigt werden (Toluol/Essigester 2:3). Rf-Wert 0.23 (Merck Fertigplatten, Toluol/Essigester = 1:4, Ninhydrin als

Entwicklungsreagens).

Beispiel 35: (5R,6S,1'R)-(1'-Hydroxyethyl)-2-allyloxycarbonylaminomethyl-2-penem-3-carbonsäureallylester

Eine Lösung von 2,88 g (10,0 mmol) (3S,4R,1'R)-3-(1'-Hydroxyethyl)-4-(N-allyloxycarbonylglycylthio)-2-azetidinon (Rohprodukt vom Verfahren 34) in 80 ml Methylenchlorid (frisch über Alox filtriert) wird unter Rühren und Feuchtigkeitsausschluss bei -10 bis -15° nacheinander mit 3,86 ml (31,5 mmol) frisch destilliertem Oxalsäureallylesterchloridund 7,69 ml (44,8 mmol) N-Ethyl-diisopropylamin versetzt und 30 Min. bei -10° weitergerührt. Eine IR-Probe zeigte die charakteristische Oxalimid-Carbonyl-Absorption bei 1820 $cm^{-1}$.

Zum Aufarbeiten wird das Reaktionsgemisch mit 50 ml $CH_2Cl_2$ verdünnt, dreimal mit eiskaltem Wasser und einmal mit Eiswasser und 5 ml gesättigter, wässriger $NaHCO_3$-Lösung gewaschen. Die wässrigen Anteile werden einmal mit $CH_2Cl_2$ nachextrahiert. Die vereinigten organischen Phasen werden mit $Na_2SO_4$ getrocknet, im Vakuum eingedampft und das Rohprodukt am Hochvakuum getrocknet. Der erhaltene dickflüssige Rückstand enthaltend den (3S,4R,1'R)-2-[4-(N-Allyloxycarbonylglycylthio)-3-(1'-allyloxalyloxyethyl)-2-oxo-1-azetidinyl]-2-oxoessigsäureallylester (IR($CH_2Cl_2$) 3440 (NH); 1820

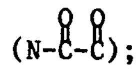

1720-1760

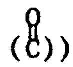

wird in 30 ml Dioxan gelöst und unter $N_2$-Atmosphäre bei Raumtemperatur mit 7 ml (40,2 mmmol) Triethylphosphit versetzt. Nach 15 Stunden bei Raumtemperatur kann im IR kein Oxalimid mehr festgestellt werden (Bande bei 1820 $cm^{-1}$). Das Reaktionsgemisch wird am Vakuum eingedampft, der Rückstand dreimal mit 2 ml Toluol und 2 ml Decan versetzt und die Schleppmittel zur Entfernung überschüssigen Phosphits/Phosphats jeweils am Hochvakuum wieder abgezogen. Das erhaltene rohe Phosphoran wird zur Cyclisierung in 250 ml Dioxan gelöst und 5 Stunden bei 105-110° gerührt. Das Reaktionsgemisch, enthaltend den (5R,6S,1'R)-2-Allyoxycarbonylaminomethyl-6-[1'-(allyloxalyloxy)ethyl]-2-penem-3-carbonsäureallylester (für analytische Zwecke wird eine Probe des rohen Cyclisierungsproduktes an Kieselgel mit Toluol/Essigsäureethylester 4:1 gereinigt: UV (EtOH) 315 nm; IR($CH_2Cl_2$) 3440 (NH); 1790, 1770, 1745, 1720

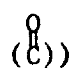

wird im Vakuum auf ca 15 ml eingedampft, mit 100 ml Methanol/$H_2O$ 8:2 und mit 25 ml gesättigter wässriger $NaHCO_3$-Lösung bei 0° versetzt. Nach 20 Minuten wird das Reaktionsgemisch auf 300 ml Essigsäureethylester und 50 ml $H_2O$ gegossen, die organische Phase abgetrennt und die wässrige Phase nochmals mit 100 ml Essigsäureethylester nachextrahiert. Die vereinigten organischen Phasen werden über $Na_2SO_4$ getrocknet und im Vakuum bis auf ca. 50 ml Volumen eingeengt. Nach Verdünnen mit 200 ml Methylenchlorid wird mit $Na_2SO_4$ getrocknet und im Vakuum vollständig eingedampft. Der Rückstand wird an 80 g Kieselgel mit Toluol/Essigsäureethylester 3:2 chromatographiert, wobei nach Kristallisation aus Ether/Hexan 3:7 die Titelverbindung erhalten wird F. 141 - 141,5°. Rf-Wert 0,20 (Merck Fertigplatten, Toluol/Essigsäureethylester 1:1).

Beispiel 36: (5R,6S,1'R)-2-Aninomethyl-6-(1'-hydroxyethyl)-2-penem-3-carbonsäure

Eine Lösung von 313 mg (0,85 mmol) (5R,6S,1'R)-2-Allyloxycarbonylaminomethyl-6-(1'-hydroxyethyl)-2-penem-3-carbonsäureallylester und 174,6 mg (1,24 mmol) Dimedon in 7,5 ml Tetrahydrofuran wird während 5 Minuten mit Argon gespült und unter Argon-Atmosphäre bei Raumtemperatur mit 27 mg (0,023 mmol) Tetrakistriphenylphosphinpalladium versetzt. Nach ca. 5 Min beginnt ein Niederschlag auszufallen, der nach

weiteren 2 Stunden Rühren abfiltriert, mit ca. 10 ml Tetrahydrofuran gewaschen und anschliessend in 4 ml $H_2O$ gelöst wird. Zur beigen Lösung wird eine Mikrospatelspitze Aktivkohle und 2 Mikrospatelspitzen Tonsil gegeben, 5 Min gut gerührt und über Hyflo klar filtriert. Am Hochvakuum wird eingeengt und der erhaltene dicke Kristallbrei mit 3 ml kaltem Ethanol versetzt und abgenutscht. Die farblosen Kristalle werden mit 2 ml Tetrahydrofuran gewaschen und am Hochvakuum getrocknet. Man erhält die Titelverbindung vom Rf-Wert = 0,50 (DC:$H_2O$, OPTI $UPC_{12}$); $[\alpha]_D^{20}$ = +176° (c = 0,5, $H_2O$).

**Patentansprüche**

**Patentansprüche für folgende Vertragsstaaten : BE, CH, DE, FR, GB, IT, LI, NL, SE**

**1.** (4R)-3,4-trans-disubstituierte Azetidinone der Formel

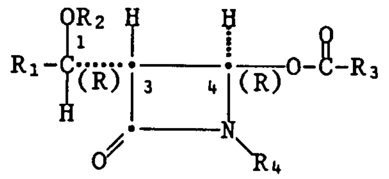

(I) ,

worin $R_1$ Wasserstoff oder $C_1$-$C_4$-Alkyl $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeutet.

**2.** Verbindungen der Formel I nach Anspruch 1, worin $R_1$ Wasserstoff oder Methyl, $R_2$ Wasserstoff, 2,2,2-Trichlorethoxycarbonyl, Allyloxycarbonyl oder 3,5-Dinitrobenzoyl, $R_3$ Phenyl und $R_4$ Wasserstoff oder p-Methoxyphenyl ist.

**3.** (3R,4R,1'R)-N-p-Methoxyphenyl-3-(1'-hydroxyethyl)-4-benzoyloxyazetidin-2-on nach Anspruch 1.

**4.** (3R,4R,1'R)-3-(1'-Hydroxyethyl)-4-benzoyloxyazetidin-2-on nach Anspruch 1.

**5.** (3R,4R,1'R)-3-[1'-(2,2,2-Trichlorethoxycarbonyloxy)ethyl]-4-benzoyloxyazetidin-2-on nach Anspruch 1.

**6.** (3R,4R,1'R)-3-(1'-Allyloxycarbonyloxyethyl)-4-benzoyloxyazetidin-2-on nach Anspruch 1.

**7.** (3R,4R,1'R)-3-[1'-(Dimethyl-2,3-dimethylbut-2-ylsilyloxy)ethyl]-4-benzoyloxyazetidin-2-on nach Anspruch 1.

**8.** (3R,4R,1'R)-3-[1'-(3,5-Dinitrobenzoyloxy)ethyl]4-benzoyloxyazetidin-2-on nach Anspruch 1.

**9.** Verfahren zur Herstellung von (4R)-3,4-trans-disubstituierten Azetidinonen der Formel

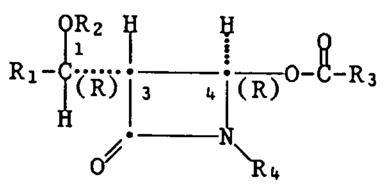

(I) ,

worin $R_1$ Wasserstoff oder $C_1$-$C_4$-Alkyl $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeuten, dadurch gekennzeichnet, dass man eine Verbindung der Formel

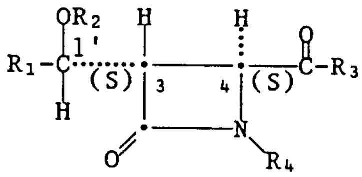

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, und gewünschtenfalls in einer erhältlichen Verbindung der Formel I, worin $R_2$ Wasserstoff bedeutet, die freie Hydroxygruppe in die geschützte Hydroxygruppe $OR_2'$ überführt, und/oder gewünschtenfalls in einer erhältlichen Verbindung der Formel I die Hydroxyschutzgruppe $R_2'$ und/oder die Aminoschutzgruppe $R_4'$ abspaltet.

**10.** Verfahren zur Herstellung von Verbindungen der Formel I, nach Anspruch 9, dadurch gekennzeichnet, dass man von Verbindungen der Formel II ausgeht, worin $R_1$ Wasserstoff oder Methyl, $R_2$ Wasserstoff oder eine Hydroxyschutzgruppe $R_2'$ , $R_3$ Phenyl und $R_4$ Wasserstoff oder eine Aminoschutzgruppe $R_4'$ bedeutet.

**11.** Eine Verbindung der Formel

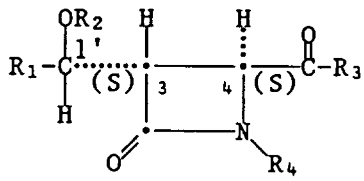

(II) ,

worin $R_1$ Wasserstoff oder $C_1$-$C_4$-Alkyl $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeutet.

**12.** Eine Verbindung der Formel II nach Anspruch 11, worin $R_1$ Wasserstoff oder Methyl, $R_2$ Wasserstoff oder eine Hydroxyschutzgruppe $R_2'$ , $R_3$ Phenyl und $R_4$ Wasserstoff oder einen Aminoschutzgruppe $R_4'$ bedeuten.

**13.** Eine Verbindung der Formel II nach Anspruch 11, worin $R_1$ Methyl, $R_2$ Wasserstoff, 2,2,2-Trichlorethoxycarbonyl, Allyloxycarbonyl, tert-Butyl-dimethylsilyl, Dimethyl-2,3-dimethylbut-2-ylsilyl oder 3,5-Dinitrobenzoyl, $R_3$ Phenyl und $R_4$ Wasserstoff oder p-Methoxyphenyl bedeutet.

**14.** (3S,4S,1'R)-N-p-Methoxyphenyl-3-(1'-hydroxyethyl)-4-benzoylazetidin-2-on nach Anspruch 11.

**15.** (3S,4S,1'R)-3-(1'-Hydroxyethyl)-4-benzoylazetidin-2-on,
(3S,4S,1'R)-N-p-Methoxyphenyl-3-[1'-(2,2,2-trichlorethoxycarbonyloxy) ethyl]-4-benzoylazetidin-2-on,
(3S,4S,1'R)-3-(1'-(2,2,2-Trichlorethoxycarbonyloxy)ethyl]-4-benzoylazetidin-2-on,
(3S,4S,1'R)-N-p-Methoxyphenyl-3-(1'-allyloxycarbonyloxyethyl)-4-benzoylazetidin-2-on und
(3S,4S,1'R)-3-(1'-Allyloxycarbonyloxyethyl)-4-benzoylazetidin-2-on nach Anspruch 11.

**16.** (3S,4S,1'R)-N-p-Methoxyphenyl-3-(1'-hydroxyethyl)-4-benzoylazetidin-2-on,
(3S,4S,1'R)-N-p-Methoxyphenyl-3-[1'-(3,5-dinitrobenzoyloxy)ethyl]-4-benzoylazetidin-2-on und
(3S,4S,1'R)-3-(1'-(3,5-Dinitrobenzoyloxy)ethyl]4-benzoylazetidin-2-on nach Anspruch 11.

**17.** (3S,4S,1'R)-N-p-Methoxyphenyl-3-[1'-(dimethyl-2,3-dimethylbut-2-ylsilyloxy)ethyl]-4-benzoylazetidin-2-on nach Anspruch 11.

**18.** (3S,4S,1'R)-3-(1'-(Dimethyl-2,3-dimethylbut-2-ylsilyloxy)ethyl]-4-benzoylazetidin-2-on nach Anspruch 11.

**19.** Verfahren zur Herstellung von Verbindungen der Formel

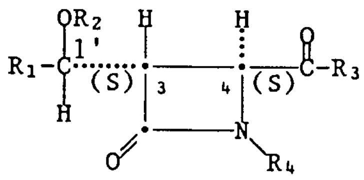

(II) ,

worin $R_1$ Wasserstoff oder $C_1$-$C_4$-Alkyl $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeutet, dadurch gekennzeichnet, dass man a) ein $\alpha$-Carbanion einer Verbindung der Formel

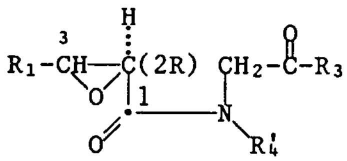

(III) ,

worin $R_1$, $R_3$ und $R_4'$ die unter Formel II genannten Bedeutungen haben, und das 2-C-Atom die R- und, wenn $R_1$ $C_1$-$C_4$-Alkyl bedeutet, das 3-C-Atom die R- oder die S-Konfiguration hat, oder eine Verbindung der Formel

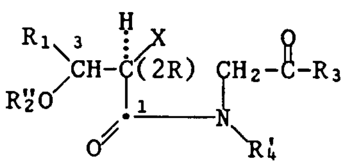

(IV),

worin $R_1$, $R_3$ und $R_4'$ die unter Formel II genannten Bedeutungen haben, $R''_2$ eine unter den Bedingungen des Ringschlussverfahrens nicht abspaltbare Hydroxyschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, zyklisiert oder b) eine cis-Verbindung der Formel

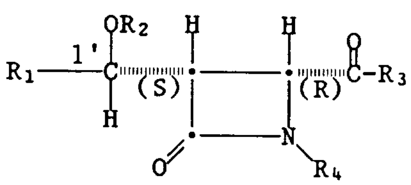

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder, wenn erwünscht eine erhältliche Verbindung der Formel II in eine andere Verbindung der Formel II umwandelt.

**20.** Verfahren nach Anspruch 19 zur Herstellung von Verbindungen der Formel II, worin $R_1$ Wasserstoff oder Methyl, $R_2$ Wasserstoff oder eine Hydroxyschutzgruppe $R_2'$ , $R_3$ Phenyl und $R_4$ Wasserstoff oder eine Aminoschutzgruppe $R_4'$ bedeutet, dadurch gekennzeichnet, dass man a) ein $\alpha$-Carbanion einer Verbindung der Formel

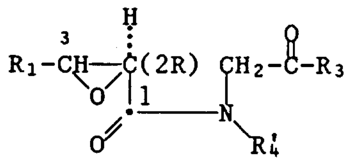

(III) ,

worin $R_1$, $R_3$ und $R_4'$ die unter Formel II genannten Bedeutungen haben, und das 2-C-Atom die R- und, wenn $R_1$ Methyl bedeutet, das 3-C-Atom die R- oder die S-Konfiguration hat, oder einer Verbindung der Formel

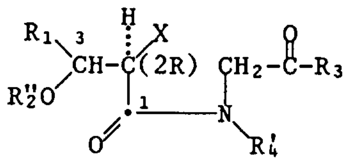

(IV),

worin $R_1$, $R_3$ und $R_4'$ die unter Formel II genannten Bedeutungen haben, $R_2''$ eine unter den Bedingungen des Ringschlussverfahrens nicht abspaltbare Hydroxyschutzgruppe, und X eine nukleofuge Abgangsgruppe darstellt und das 2-C-Atom die R- und, wenn $R_1$ Methyl bedeutet, das 3-C-Atom die R- oder die S-Konfiguration hat, zyklisiert und, wenn erwünscht, die Aminoschutzgruppe $R_4'$ abspaltet und durch Wasserstoff ersetzt und/oder, wenn erwünscht eine erhältliche Verbindung der Formel II in eine andere Verbindung der Formel II umwandelt.

**21.** Verfahren zur Herstellung von (4R)-3,4-trans-disubstituierten Azetidinonen der Formel

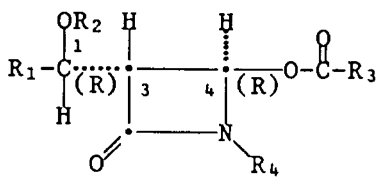

(I) ,

worin $R_1$ Wasserstoff oder $C_1$-$C_4$-Alkyl, $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeuten, dadurch gekennzeichnet, dass man eine Verbindung der Formel

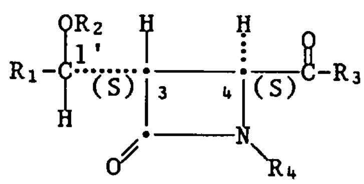

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, und gewünschtenfalls in einer erhältlichen Verbindung der Formel I, worin $R_2$ Wasserstoff bedeutet, die freie Hydroxygruppe in die geschützte Hydroxygruppe $OR_2'$ überführt, und/oder gewünschtenfalls in einer erhältlichen Verbindung der Formel I die Hydroxyschutzgruppe $R_2'$ und/oder die Aminoschutzgruppe $R_4'$ abspaltet,
wobei das Verfahren zur Herstellung von Verbindungen der Formel

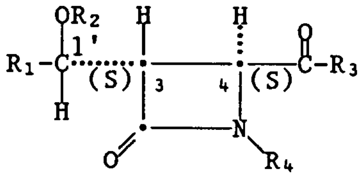

(II) ,

worin $R_1$ Wasserstoff oder $C_1$-$C_4$-Alkyl, $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeutet, dadurch gekennzeichnet ist, dass man a) ein $\alpha$-Carbanion einer Verbindung der Formel

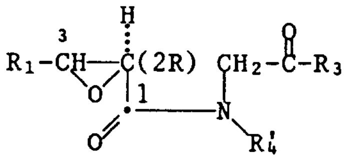

(III) ,

worin $R_1$, $R_3$ und $R_4'$ die unter Formel II genannten Bedeutungen haben, und das 2-C-Atom die R- und, wenn $R_1$ $C_1$-$C_4$-Alkyl bedeutet, das 3-C-Atom die R- oder die S-Konfiguration hat, oder eine Verbindung der Formel

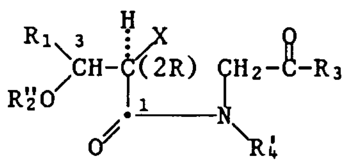

(IV),

worin $R_1$, $R_3$ und $R_4'$ die unter Formel II genannten Bedeutungen haben, $R''_2$ eine unter den Bedingungen des Ringschlussverfahrens nicht abspaltbare Hydroxyschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, zyklisiert oder b) eine cis-Verbindung der Formel

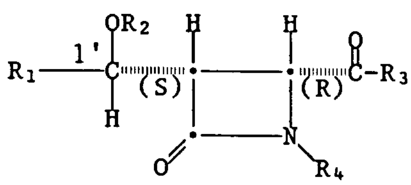

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder, wenn erwünscht eine erhältliche Verbindung der Formel II in eine andere Verbindung der Formel II umwandelt, das Verfahren zur Herstellung von Verbindungen der Formel

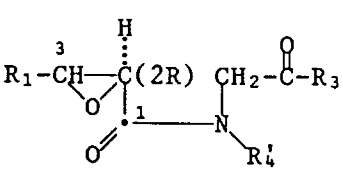

(III) ,

worin $R_1$ Wasserstoff oder $C_1$-$C_4$-Alkyl, $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4'$ eine Aminoschutzgruppe bedeutet, und

das 2-C-Atom die R- und, wenn $R_1$ $C_1$-$C_4$-Alkyl bedeutet, das 3-C-Atom die R- oder die S-Konfiguration hat, dadurch gekennzeichnet ist, dass man eine Verbindung der Formel

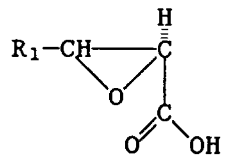

(VIII)

mit einem Amin der Formel

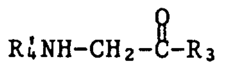

(VII),

worin $R_3$ und $R'_4$ die unter Formel (V) genannten Bedeutungen haben, umsetzt oder aus Verbindungen der Formel

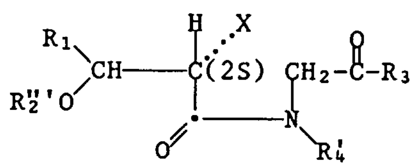

(V),

worin $R_1$, $R_3$, $R_4'$ und X die genannten Bedeutungen haben, $R_2'''$ Wasserstoff oder eine unter den Bedingungen der Epoxidbildung abspaltbare Hydroxyschutzgruppe darstellt und worin das 3-C-Atom die R- oder S-Konfiguration hat, wenn $R_1$ $C_1$-$C_4$-Alkyl bedeutet, unter carbanionenbildenden Bedingungen und unter Waldenscher Umkehr am 2-C-Atom herstellt,
das Verfahren zur Herstellung von Verbindungen der Formel

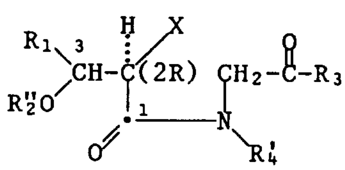

(IV),

worin $R_1$ Wasserstoff oder $C_1$-$C_4$-Alkyl, $R_2''$ eine unter den Bedingungen des Ringschlussverfahrens nicht abspaltbare Hydroxyschutzgruppe, $R_3$ Phenyl, durch $C_1$-$C_4$-Alkoxy, $C_1$-$C_4$-Alkyl und/oder Halogen mono- bis tri-substituiertes Phenyl oder 4-Nitrophe nyl, $R_4'$ eine Aminoschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, dadurch gekennzeichnet ist, dass man eine Carbonsäure der Formel

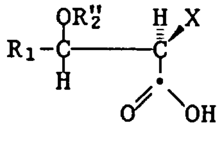

(VI')

worin $R_1$, $R_2''$ und X die unter Formel IV genannten Bedeutungen haben, mit einem Amin der Formel

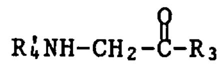

(VII),

worin $R_3$ und $R'_4$ die unter Formel (V) genannten Bedeutungen haben, umsetzt und das Verfahren zur Herstellung von Verbindungen der Formel

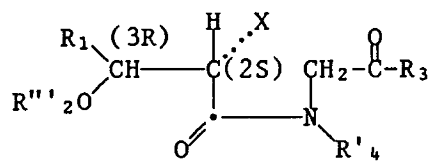

(V),

worin $R_1$ Wasserstoff oder $C_1$-$C_4$-Alkyl $R'''_2$ Wasserstoff oder eine unter den Bedingungen der Epoxidbildung abspaltbare Hydroxyschutzgruppe, $R_3$ Phenyl, durch $C_1$-$C_4$-Alkoxy, $C_1$-$C_4$-Alkyl und/oder Halogen mono- bis tri-substituiertes Phenyl oder 4-Nitrophenyl, $R_4$ eine Aminoschutzgruppe und X eine nukleofuge Abgangsgruppe darstellen und worin das 3-C-Atom die R-Konfiguration hat, wenn $R_1$ $C_1$-$C_4$-Alkyl bedeutet, dadurch gekennzeichnet ist, dass man eine Carbonsäure der Formel

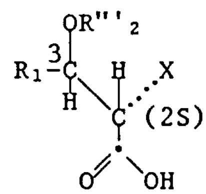

(VI)

worin $R_1$ $R'''_2$ und X die unter Formel (V) genannten Bedeutung haben, mit einem Amin der Formel

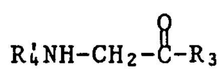

(VII),

worin $R_3$ und $R'_4$ die unter Formel (V) genannten Bedeutungen haben, umsetzt.

**Patentansprüche für folgenden Vertragsstaat : AT**

**1.** Verfahren zur Herstellung von (4R)-3,4-trans-disubstituierten Azetidinonen der Formel

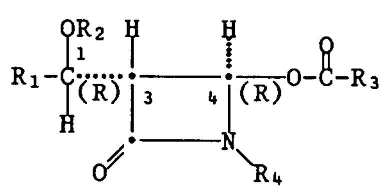

(I) ,

worin $R_1$ Wasserstoff oder $C_1$-$C_4$-Alkyl, $R_2$ Wasserstoff oder eine Hydroxyschutzgruppe $R'_2$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R'_4$ bedeuten, dadurch gekennzeichnet, dass man eine Verbindung der Formel

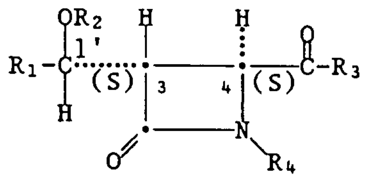

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, und gewünschtenfalls in einer erhältlichen Verbindung der Formel I, worin $R_2$ Wasserstoff bedeutet, die freie Hydroxygruppe in die geschützte Hydroxygruppe $OR_2'$ überführt, und/oder gewünschtenfalls in einer erhältlichen Verbindung der Formel I die Hydroxyschutzgruppe $R_2'$ und/oder die Aninoschutzgruppe $R_4'$ abspaltet.

2. Verfahren zur Herstellung von Verbindungen der Formel I, nach Anspruch 1, dadurch gekennzeichnet, dass man von Verbindungen der Formel II ausgeht, worin $R_1$ Wasserstoff oder Methyl, $R_2$ Wasserstoff oder eine Hydroxyschutzgruppe $R_2'$, $R_3$ Phenyl und $R_4$ Wasserstoff oder eine Aminoschutzgruppe $R_4'$ bedeutet.

3. Verfahren nach Anspruch 1, dadurch gekennzeichnet, dass man von Verbindungen der Formel II ausgeht, worin $R_1$ Wasserstoff oder Methyl, $R_2$ Wasserstoff, 2,2,2-Trichlorethoxycarbonyl, Allyloxycarbonyl oder 3,5-Dinitrobenzoyl, $R_3$ Phenyl und $R_4$ Wasserstoff oder p-Methoxyphenyl ist.

4. Verfahren zur Herstellung von (3R,4R,1'R)-N-p-Methoxyphenyl-3-(1'-hydroxyethyl)-4-benzoyloxyazetidin-2-on nach Anspruch 1.

5. Verfahren zur Herstellung von (3R,4R,1'R)-3-(1'-Hydroxyethyl)-4-benzoyloxyazetidin-2-on nach Anspruch 1.

6. Verfahren zur Herstellung von (3R,4R,1'R)-3-[1'-(2,2,2-Trichlorethoxycarbonyloxy)ethyl]-4-benzoyloxyazetidin-2-on nach Anspruch 1.

7. Verfahren zur Herstellung von (3R,4R,1'R)-3-[1'-(Dimethyl-2,3-dimethylbut-2-ylsilyloxy)ethyl]-4-benzoyloxyazetidin-2-on nach Anspruch 1.

8. Verfahren zur Herstellung von (3R,4R,1'R)-3-[1'-(3,5-Dinitrobenzoyloxy)ethyl]-4-benzoyloxyazetidin-2-on nach Anspruch 1.

9. Verfahren zur Herstellung von (3R,4R,1'R)-3-(1'-Allyloxycarbonyloxyethyl)-4-benzoyloxyazetidin-2-on nach Anspruch 1.

10. Verfahren zur Herstellung von Verbindungen der Formel

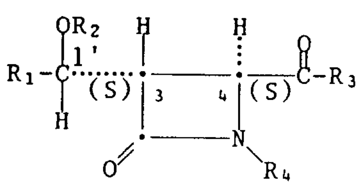

(II) ,

worin $R_1$ Wasserstoff oder $C_1$-$C_4$-Alkyl, $R_2$ Wasserstoff oder eine Hydroxyschutzgruppe $R_2'$, $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeutet, dadurch gekennzeichnet, dass man
a) ein $\alpha$-Carbanion einer Verbindung der Formel

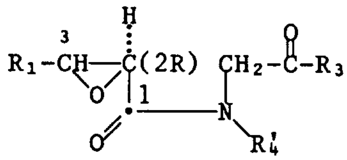

(III) ,

worin $R_1$, $R_3$ und $R_4'$ die unter Formel II genannten Bedeutungen haben, und das 2-C-Atom die R- und, wenn $R_1$ $C_1$-$C_4$-Alkyl bedeutet, das 3-C-Atom die R- oder die S-Konfiguration hat, oder eine Verbindung der Formel

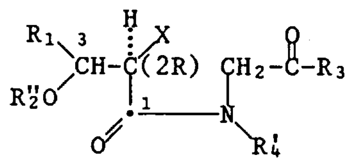

(IV),

worin $R_1$, $R_3$ und $R_4'$ die unter Formel II genannten Bedeutungen haben, $R''_2$ eine unter den Bedingungen des Ringschlussverfahrens nicht abspaltbare Hydroxyschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, zyklisiert oder b) eine cis-Verbindung der Formel

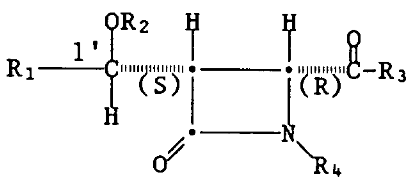

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder, wenn erwünscht eine erhältliche Verbindung der Formel II in eine andere Verbindung der Formel II umwandelt.

**11.** Verfahren nach Anspruch 10 zur Herstellung von Verbindungen der Formel II, worin $R_1$ Wasserstoff oder Methyl, $R_2$ Wasserstoff oder eine Hydroxyschutzgruppe $R_2'$, $R_3$ Phenyl und $R_4$ Wasserstoff oder eine Aminoschutzgruppe $R_4'$ bedeutet, dadurch gekennzeichnet, dass man a) ein $\alpha$-Carbanion einer Verbindung der Formel

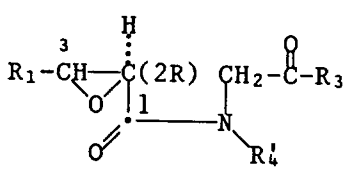

(III) ,

worin $R_1$, $R_3$ und $R_4'$ die unter Formel II genannten Bedeutungen haben, und das 2-C-Atom die R- und, wenn $R_1$ Methyl bedeutet, das 3-C-Atom die R- oder die S-Konfiguration hat, oder einer Verbindung der Formel

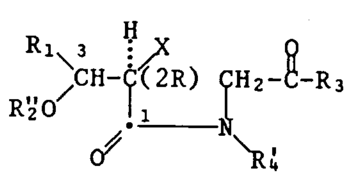

(IV),

worin $R_1$, $R_3$ und $R_4'$ die unter Formel II genannten Bedeutungen haben, $R_2''$ eine unter den Bedingungen des Ringschlussverfahrens nicht abspaltbare Hydroxyschutzgruppe, und X eine nukleofuge Abgangsgruppe darstellt und das 2-C-Atom die R- und, wenn $R_1$ Methyl bedeutet, das 3-C-Atom die R- oder die S-Konfiguration hat, zyklisiert und, wenn erwünscht, die Aninoschutzgruppe $R_4'$ abspaltet und durch Wasserstoff ersetzt und/oder, wenn erwünscht eine erhältliche Verbindung der Formel II in eine andere Verbindung der Formel II umwandelt.

**12.** Verfahren nach Anspruch 10 zur Herstellung von Verbindungen der Formel II, worin $R_1$ Methyl, $R_2$ Wasserstoff, 2,2,2-Trichlorethoxycarbonyl, Allyloxycarbonyl, tert-Butyldimethylsilyl, Dimethyl-2,3-dimethylbut-2-ylsilyl, oder 3,5-Dinitrobenzoyl, $R_3$ Phenyl und $R_4$ Wasserstoff oder p-Methoxyphenyl bedeuten.

**13.** Verfahren nach Anspruch 11 zur Herstellung von (3S,4S,1'R)-N-p-Methoxyphenyl-3-(1'-hydroxyethyl)-4-benzoylazetidin-2-on.

**14.** Verfahren zur Herstellung von (3S,4S,1'R)-3-(1'-Hydroxyethyl)-4-benzoylazetidin-2-on,
(3S,4S,1'R)-N-p-Methoxyphenyl-3-[1'-(2,2,2-trichlorethoxycarbonyloxy)ethyl]-4-benzoylazetidin-2-on ,
(3S,4S,1'R)-3-[1'-(2,2,2-Trichlorethoxycarbonyloxy)ethyl]-4-benzoylazetidin-2-on,
(3S,4S,1'R)-N-p-Methoxyphenyl-3-(1'-allyloxycarbonyloxyethyl)-4-benzoylazetidin-2-on und
(3S,4S,1'R)-3-(1'-Allyloxycarbonyloxyethyl)-4-benzoylazetidin-2-on nach Anspruch 11.

**15.** Verfahren zur Herstellung von (3S,4S,1'R)-N-p-Methoxyphenyl-3-(1'-hydroxyethyl)-4-benzoylazetidin-2-on,
(3S,4S,1'R)-N-p-Methoxyphenyl-3-[1'-(3,5-dinitrobenzoyloxy)ethyl]-4-benzoylazetidin-2-on,
(3S,4S,1'R)-3-[1'-(3,5-Dinitrobenzoyloxy)ethyl]-4-benzoylazetidin-2-on, oder
(3S,4S,1'R)-N-p-Methoxyphenyl-3-[1'-(dimethyl-2,3-dimethylbut-2-ylsilyloxy]ethyl]-4-benzoylazetidin-2-on nach Anspruch 11.

**16.** Verfahren zur Herstellung von (3S,4S,1'R)-3-[1'-(Dimethyl-2,3-dimethylbut-2-ylsilvloxy)ethyl]-4-benzoylazetidin-2-on nach Anspruch 11.

**17.** Verfahren zur Herstellung von (4R)-3,4-trans-disubstituierten Azetidinonen der Formel

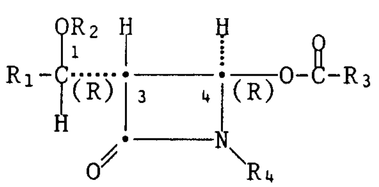

(I) ,

worin $R_1$ Wasserstoff oder $C_1$-$C_4$-Alkyl, $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeuten, dadurch gekennzeichnet, dass man eine Verbindung der Formel

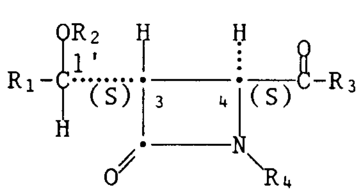

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, und gewünschtenfalls in einer erhältlichen Verbindung der Formel I, worin $R_2$ Wasserstoff bedeutet, die freie Hydroxygruppe in die geschützte Hydroxygruppe $OR_2'$ überführt, und/oder gewünschtenfalls in einer erhältlichen Verbindung der Formel I die Hydrox-

yschutzgruppe $R_2'$ und/oder die Aminoschutzgruppe $R_4'$ abspaltet, wobei das Verfahren zur Herstellung von Verbindungen der Formel

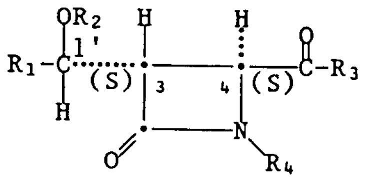

(II) ,

worin $R_1$ Wasserstoff oder $C_1$-$C_4$-Alkyl, $R_2$ Wasserstoff oder eine Hydroxyschutzgruppe $R_2'$ , $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4$ Wasserstoff oder eine Aminoschutzgruppe $R_4'$ bedeutet, dadurch gekennzeichnet ist, dass man a) ein $\alpha$-Carbanion einer Verbindung der Formel

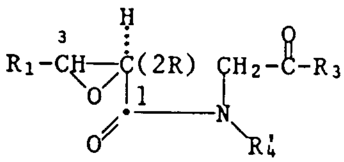

(III) ,

worin $R_1$, $R_3$ und $R_4'$ die unter Formel II genannten Bedeutungen haben, und das 2-C-Atom die R- und, wenn $R_1$ $C_1$-$C_4$-Alkyl bedeutet, das 3-C-Atom die R- oder die S-Konfiguration hat, oder eine Verbindung der Formel

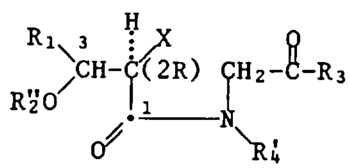

(IV),

worin $R_1$, $R_3$ und $R_4'$ die unter Formel II genannten Bedeutungen haben, $R''_2$ eine unter den Bedingungen des Ringschlussverfahrens nicht abspaltbare Hydroxyschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, zyklisiert oder b) eine cis-Verbindung der Formel

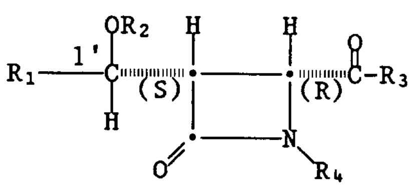

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder, wenn erwünscht eine erhältliche Verbindung der Formel II in eine andere Verbindung der Formel II umwandelt, das Verfahren zur Herstellung von Verbindungen der Formel

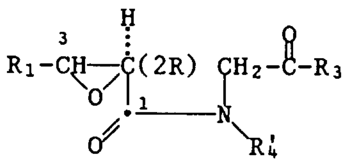

(III) ,

worin $R_1$ Wasserstoff oder $C_1$-$C_4$-Alkyl, $R_3$ Phenyl, durch $C_1$-$C_4$-Alkoxy, $C_1$-$C_4$-Alkyl und/oder Halogen mono- bis tri-substituiertes Phenyl oder 4-Nitrophenyl und $R_4'$ eine Aminoschutzgruppe bedeutet, und das 2-C-Atom die R- und, wenn $R_1$ $C_1$-$C_4$-Alkyl bedeutet, das 3-C-Atom die R- oder die S-Konfiguration hat, dadurch gekennzeichnet ist, dass man eine Verbindung der Formel

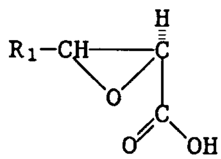

(VIII)

mit einem Amin der Formel

$$R_4'NH-CH_2-\overset{O}{\overset{\|}{C}}-R_3$$

(VII),

worin $R_3$ und $R'_4$ die unter Formel (V) genannten Bedeutungen haben, umsetzt oder aus Verbindungen der Formel

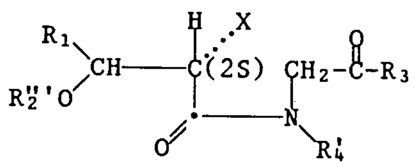

(V),

worin $R_1$, $R_3$, $R_4'$ und X die genannten Bedeutungen haben, $R_2'''$ Wasserstoff oder eine unter den Bedingungen der Epoxidbildung abspaltbare Hydroxyschutzgruppe darstellt und worin das 3-C-Atom die R- oder S-Konfiguration hat, wenn $R_1$ $C_1$-$C_4$-Alkyl bedeutet, unter carbonionenbildenden Bedingungen und unter Walderscher Umkehr am 2-C-Atom herstellt,
das Verfahren zur Herstellung von Verbindungen der Formel

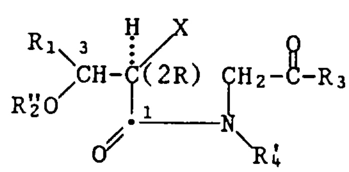

(IV),

worin $R_1$ Wasserstoff oder $C_1$-$C_4$-Alkyl, $R_2''$ eine unter den Bedingungen des Ringschlussverfahrens nicht abspaltbare Hydroxyschutzgruppe, $R_3$ Phenyl, durch $C_1$-$C_4$-Alkoxy, $C_1$-$C_4$-Alkyl und/oder Halogen mono- bis tri-substituiertes Phenyl oder 4-Nitrophenyl, $R_4'$ eine Aminoschutzgruppe, und X eine nukleofuge Abgangsgruppe darstellt und das 2-C-Atom die R- und, wenn $R_1$ $C_1$-$C_4$-Alkyl bedeutet, das 3-C-Atom die R- oder die S-Konfiguration hat, dadurch gekennzeichnet ist, dass man eine Carbonsäure der Formel

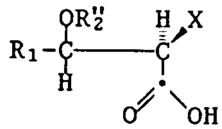

(VI')

worin $R_1$, $R_2''$ und X die unter Formel IV genannten Bedeutungen haben, mit einem Amin der Formel

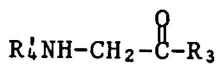

(VII),

worin $R_3$ und $R'_4$ die unter Formel (V) genannten Bedeutungen haben, umsetzt und das Verfahren zur Herstellung von Verbindungen der Formel

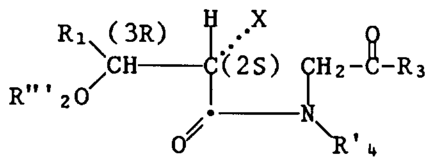

(V),

worin $R_1$ Wasserstoff oder $C_1$-$C_4$-Alkyl, $R'''_2$ Wasserstoff oder eine unter den Bedingungen der Epoxidbildung abspaltbare Hydroxyschutzgruppe, $R_3$ Phenyl, durch $C_1$-$C_4$-Alkoxy, $C_1$-$C_4$-Alkyl und/oder Halogen mono- bis tri-substituiertes Phenyl oder 4-Nitrophenyl $R_4$ eine Aminoschutzgruppe und X eine nukleofuge Abgansgruppe darstellen und worin das 3-C-Atom die R-Konfiguration hat, wenn $R_1$ $C_1$-$C_4$-Alkyl bedeutet, dadurch gekennzeichnet ist, dass man eine Carbonsäure der Formel

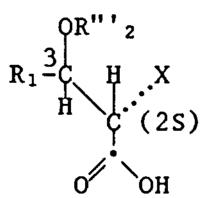

(VI)

worin $R_1$ $R'''_2$ und X die unter Formel (V) genannten Bedeutung haben, mit einem Amin der Formel

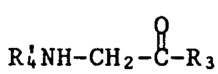

(VII),

worin $R_3$ und $R'_4$ die unter Formel (V) genannten Bedeutungen haben, umsetzt.

**Claims**

**Claims for the following Contracting States : BE, CH, DE, FR, GB, IT, LI, NL, SE**

1. A (4R)-3,4-trans-disubstituted azetidinone of the formula

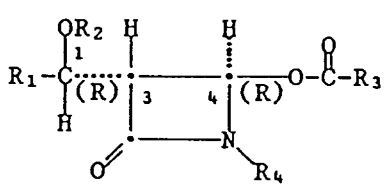

(I)

in which $R_1$ is hydrogen or $C_1$-$C_4$alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$.

**2.** A compound of the formula I according to claim 1 in which $R_1$ is hydrogen or methyl, $R_2$ is hydrogen, 2,2,2-trichloroethoxycarbonyl, allyloxycarbonyl or 3,5-dinitrobenzoyl, $R_3$ is phenyl and $R_4$ is hydrogen or p-methoxyphenyl.

**3.** (3R,4R,1'R)-N-p-methoxyphenyl-3-(1'-hydroxyethyl)-4-benzoyloxyazetidin-2-one according to claim 1.

**4.** (3R,4R,1'R)-3-(1'-hydroxyethyl)-4-benzoyloxyazetidin-2-one according to claim 1.

**5.** (3R,4R,1'R)-3-[1'-(2,2,2-trichloroethoxycarbonyloxy)ethyl]-4-benzoyloxyazetidin-2-one according to claim 1.

**6.** (3R,4R,1'R)-3-(1'-allyloxycarbonyloxyethyl)-4-benzoyloxyazetidin-2-one according to claim 1.

**7.** (3R,4R,1'R)-3-(1'-(dimethyl-2,3-dimethylbut-2-ylsilyloxy)-ethyl]-4-benzoyloxyazetidin-2-one according to claim 1.

**8.** (3R,4R,1'R)-3-[1'-(3,5-dinitrobenzoyloxy)-ethyl]-4-benzoyloxyazetidin-2-one according to claim 1.

**9.** A process for the manufacture of a (4R)-3,4-trans-disubstituted azetidinone of the formula

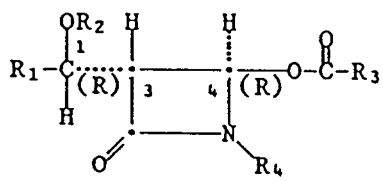

(I)

in which $R_1$ is hydrogen or $C_1$-$C_4$alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$, characterised in that a compound of the formula

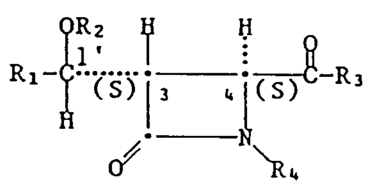

(II),

in which $R_1$, $R_2$, $R_3$ and $R_4$ have the meanings mentioned under formula I, is subjected to a Bayer-Villiger reaction by treatment with a per acid, and, if desired, in an obtainable compound of the formula I in which $R_2$ is hydrogen the free hydroxy group is converted into a protected hydroxy group $OR_2'$ and/or, if desired, in an obtainable compound of the formula I the hydroxy-protecting group $R_2'$ and/or the amino-protecting group $R_4'$ are(is) removed.

**10.** A process for the manufacture of a compound of the formula I according to claim 9, characterised in that there is used as starting material a compound of the formula II in which $R_1$ is hydrogen or methyl, $R_2$ is hydrogen or a hydroxy-protecting group $R_2'$, $R_3$ is phenyl and $R_4$ is hydrogen or an amino-protecting group $R_4'$.

**11.** A compound of the formula

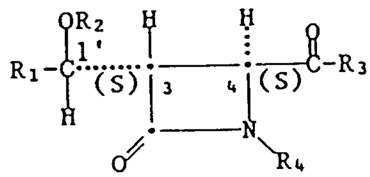

(II)

in which $R_1$ is hydrogen or $C_1$-$C_4$alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$.

**12.** A compound of the formula II according to claim 11 in which $R_1$ is hydrogen or methyl, $R_2$ is hydrogen or a hydroxy-protecting group $R_2'$, $R_3$ is phenyl and $R_4$ is hydrogen or an amino-protecting group $R_4'$.

**13.** A compound of the formula II according to claim 11 in which $R_1$ is methyl, $R_2$ is hydrogen, 2,2,2-trichloroethoxycarbonyl, allyloxycarbonyl, tert-butyldimethylsilyl, dimethyl-2,3-dimethylbut-2-ylsilyl or 3,5-dinitrobenzoyl, $R_3$ is phenyl and $R_4$ is hydrogen or p-methoxyphenyl.

**14.** (3S,4S,1'R)-N-p-methoxyphenyl-3-(1'-hydroxyethyl)-4-benzoylazetidin-2-one according to claim 11.

**15.** (3S,4S,1'R)-3-(1'-hydroxyethyl)-4-benzoylazetidin-2-one, (3S,4S,1'R)-N-p-methoxyphenyl-3-[1'-(2,2,2-trichloroethoxycarbonyloxy)-ethyl]-4-benzoylazetidin-2-one, (3S,4S,1'R)-3-(1'-(2,2,2-trichloroethoxycarbonyloxy)ethyl]-4-benzoylazetidin-2-one, (3S,4S,1'R)-N-p-methoxyphenyl-3-(1'-allyloxycarbonyloxyethyl)-4-benzoylazetidin-2-one, and (3S,4S,1'R)-3-(1'-allyloxycarbonyloxyethyl)-4-benzoylazetidin-2-one according to claim 11.

**16.** (3S,4S,1'R)-N-p-methoxyphenyl-3-(1'-hydroxyethyl)-4-benzoylazetidin-2-one, (3S,4S,1'R)-N-p-methoxyphenyl-3-[1'-(3,5-dinitrobenzoyloxy)-ethyl]-4-benzoylazetidin-2-one, and (3S,4S,1'R)-3-[1'-(3,5-dinitrobenzoyloxy)ethyl]-4-benzoylazetidin-2-one according to claim 11.

**17.** (3S,4S,1'R)-N-p-methoxyphenyl-3-[1'-(dimethyl-2,3-dimethylbut-2-ylsilyloxy)-ethyl]-4-benzoylazetidin-2-one according to claim 11.

**18.** (3S,4S,1'R)-3-[1'-(dimethyl-2,3-dimethylbut-2-ylsilyloxy)-ethyl]-4-benzoylazetidin-2-one according to claim 11.

**19.** A process for the manufacture of a compound of the formula

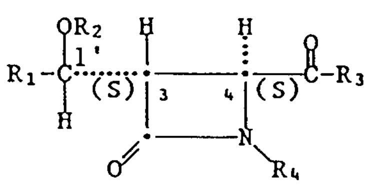

(II)

in which $R_1$ is hydrogen or $C_1$-$C_4$alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$, characterised in that a) an $\alpha$-carbanion of a compound of the formula

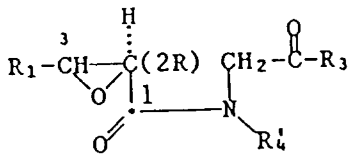

(III),

in which $R_1$, $R_3$ and $R_4'$ have the meanings mentioned under formula II and the 2-carbon atom has the R-configuration and, if $R_1$ is $C_1$-$C_4$alkyl, the 3-carbon atom has the R- or the S-configuration, or a compound of the formula

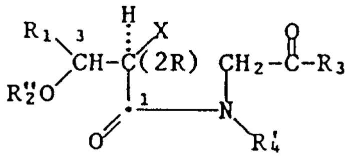

(IV),

in which $R_1$, $R_3$ and $R_4'$ have the meanings given under formula II, $R_2''$ is a hydroxy-protecting group that cannot be removed under the conditions of the cyclisation process and X is a nucleofugal leaving group, and the 2-carbon atom has the R-configuration and, if $R_1$ is $C_1$-$C_4$alkyl, the 3-carbon atom has the R- or the S-configuration, is cyclised, or b) a cis-compound of the formula

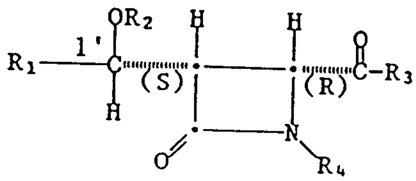

(II'),

in which $R_1$, $R_2$, $R_3$ and $R_4$ have the meanings given under formula II, is isomerised, and, if desired, the amino-protecting group $R_4'$ is removed and replaced by hydrogen and/or, if desired, an obtainable compound of the formula II is converted into a different compound of the formula II.

**20.** A process according to claim 19 for the manufacture of a compound of the formula II in which $R_1$ is hydrogen or methyl, $R_2$ is hydrogen or a hydroxy-protecting group $R_2'$ , $R_3$ is phenyl and $R_4$ is hydrogen or an amino-protecting group $R_4'$ , characterised in that a) an $\alpha$-carbanion of a compound of the formula

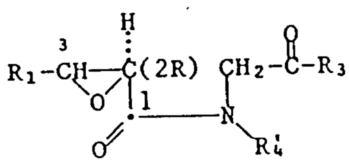

(III),

in which $R_1$, $R_3$ and $R_4'$ have the meanings mentioned under formula II and the 2-carbon atom has the R-configuration and, if $R_1$ is methyl, the 3-carbon atom has the R- or the S-configuration, or a compound of the formula

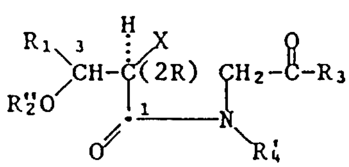

(IV),

in which $R_1$, $R_3$ and $R_4'$ have the meanings mentioned under formula II, $R_2''$ is a hydroxy-protecting group that cannot be removed under the conditions of the cyclisation process and X is a nucleofugal leaving group, and the 2-carbon atom has the R-configuration and, if $R_1$ is methyl, the 3-carbon atom has the R- or the S-configuration, is cyclised and, if desired, the amino-protecting group $R_4'$ is removed and replaced by hydrogen and/or, if desired, an obtainable compound of the formula II is converted into a different compound of the formula II.

**21.** A process for the manufacture of a (4R)-3,4-trans-disubstituted azetidinone of the formula

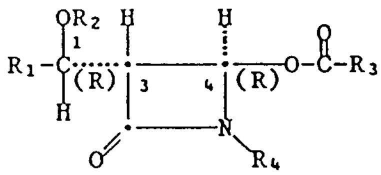

(I)

in which $R_1$ is hydrogen or $C_1$-$C_4$alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$, characterised in that a compound of the formula

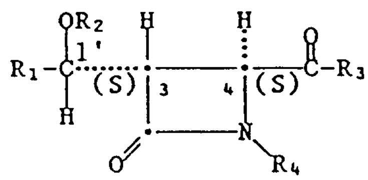

(II),

in which $R_1$, $R_2$, $R_3$ and $R_4$ have the meanings mentioned under formula I, is subjected to a Bayer-Villiger reaction by treatment with a per acid, and, if desired, in an obtainable compound of the formula I in which $R_2$ is hydrogen, the free hydroxy group is converted into a protected hydroxy group $OR_2'$ and/or, if desired, in an obtainable compound of the formula I the hydroxy-protecting group $R_2'$ and/or the amino-protecting group $R_4'$ are(is) removed,
the process for the manufacture of a compound of the formula

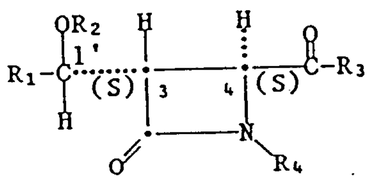

(II),

in which $R_1$ is hydrogen or $C_1$-$C_4$alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$, being characterised in that a) an $\alpha$-carbanion of a compound of the formula

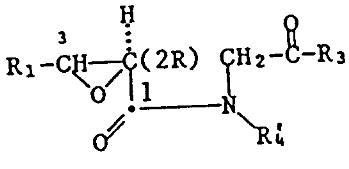

(III),

in which $R_1$, $R_3$ and $R_4'$ have the meanings mentioned under formula II and the 2-carbon atom has the R-configuration and, if $R_1$ is $C_1$-$C_4$alkyl, the 3-carbon atom has the R- or the S-configuration, or a

compound of the formula

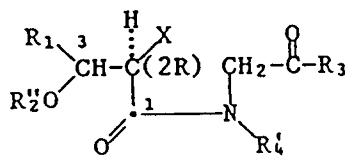

(IV),

in which $R_1$, $R_3$ and $R_4'$ have the meanings mentioned under formula II, $R_2''$ is a hydroxy-protecting group that cannot be removed under the conditions of the cyclisation process and X is a nucleofugal leaving group, and the 2-carbon atom has the R-configuration and, if $R_1$ is $C_1$-$C_4$-alkyl, the 3-carbon atom has the R- or the S-configuration, is cyclised or b) a cis-compound of the formula

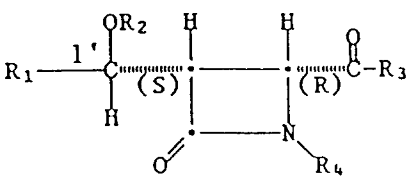

(II'),

in which $R_1$, $R_2$, $R_3$ and $R_4$ have the meanings indicated under formula II, is isomerised, and, if desired, the amino-protecting group $R_4'$ is removed and replaced by hydrogen and/or, if desired, an obtainable compound of the formula II is converted into a different compound of the formula II,
the process for the manufacture of a compound of the formula

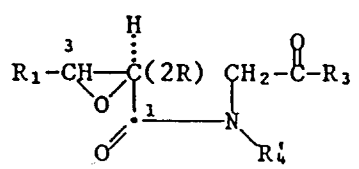

(III),

in which $R_1$ is hydrogen or $C_1$-$C_4$alkyl, $R_3$ is phenyl, phenyl that is mono- to tri-substituted by $C_1$-$C_4$alkoxy,$C_1$-$C_4$alkyl and/or by halogen, or is 4-nitrophenyl and $R_4'$ is an amino-protecting group, and the 2-carbon atom has the R-configuration and, if $R_1$ is $C_1$-$C_4$alkyl, the 3-carbon atom has the R- or the S-configuration, being characterised in that a compound of the formula

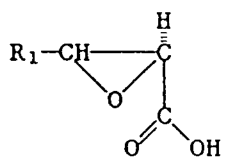

(VIII)

is reacted with an amine of the formula

$$R_4'NH-CH_2-\overset{O}{\overset{\|}{C}}-R_3 \quad (VII)$$

in which $R_3$ and $R_4'$ have the meanings mentioned under formula V, or a compound of the formula III is manufactured, under carbanion-forming conditions and with a Walden inversion at the 2-carbon atom, from a compound of the formula

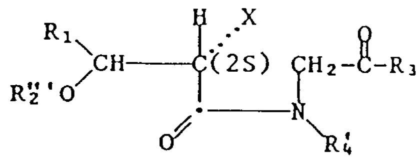

(V)

in which $R_1$, $R_3$, $R_4'$ and X have the meanings mentioned, $R_2'''$ is hydrogen or a hydroxy-protecting group that can be removed under the conditions of the epoxide formation, and in which the 3-carbon atom has the R- or the S-configuration if $R_1$ is $C_1$-$C_4$alkyl,
the process for the manufacture of a compound of the formula

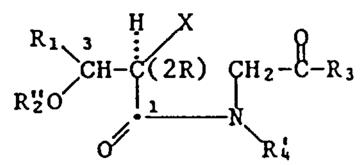

(IV),

in which $R_1$ is hydrogen or $C_1$-$C_4$alkyl, $R_2''$ is a hydroxy-protecting group that cannot be removed under the conditions of the cyclisation process, $R_3$ is phenyl, phenyl that is mono- to tri-substituted by $C_1$-$C_4$alkoxy,$C_1$-$C_4$alkyl and/or by halogen, or is 4-nitrophenyl, $R_4'$ is an amino-protecting group and X is a nucleofugal leaving group, and the 2-carbon atom has the R-configuration and, if $R_1$ is $C_1$-$C_4$alkyl, the 3-carbon atom has the R- or the S-configuration, being characterised in that a carboxylic acid of the formula

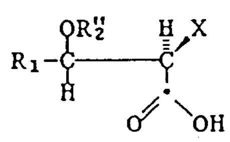

(VI'),

in which $R_1$, $R_2''$ and X have the meanings mentioned under formula IV, is reacted with an amine of the formula

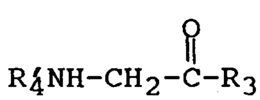

(VII)

in which $R_3$ and $R_4'$ have the meanings mentioned under formula V, and
the process for the manufacture of a compound of the formula

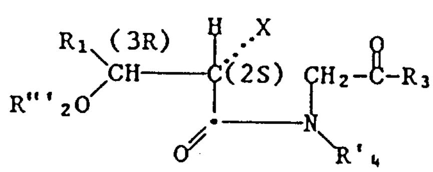

(V),

in which $R_1$ is hydrogen or $C_1$-$C_4$alkyl, $R_2'''$ is hydrogen or a hydroxy-protecting group that can he removed under the conditions of the epoxide formation, $R_3$ is phenyl, phenyl that is mono- to tri-substituted by $C_1$-$C_4$alkoxy, $C_1$-$C_4$-alkyl and/or by halogen, or is 4-nitrophenyl, $R_4$ is an amino-protecting group and X is a nucleofugal leaving group and in which the 3-carbon atom has the R-configuration if $R_1$ is $C_1$-$C_4$alkyl, being characterised in that a carboxylic acid of the formula

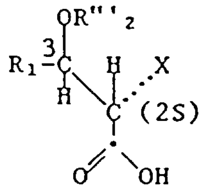

(VI),

in which $R_1$, $R_2'''$ and X have the meanings mentioned under formula V, is reacted with an amine of the formula

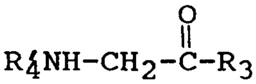

(VII)

in which $R_3$ and $R_4'$ have the meanings mentioned under formula V.

**Claims for the following Contracting State : AT**

**1.** A process for the manufacture of a (4R)-3,4-trans-disubstituted azetidinone of the formula

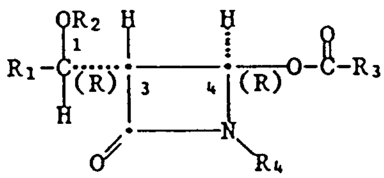

(I)

in which $R_1$ is hydrogen or $C_1$-$C_4$alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$, characterised in that a compound of the formula

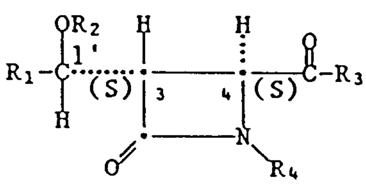

(II),

in which $R_1$, $R_2$, $R_3$ and $R_4$ have the meanings mentioned under formula I, is subjected to a Bayer-Villiger reaction by treatment with a per acid, and, if desired, in an obtainable compound of the formula I in which $R_2$ is hydrogen the free hydroxy group is converted into a protected hydroxy group $OR_2'$ and/or, if desired, in an obtainable compound of the formula I the hydroxy-protecting group $R_2'$ and/or the amino-protecting group $R_4'$ are(is) removed.

**2.** A process for the manufacture of a compound of the formula I according to claim 1, characterised in that there is used as starting material a compound of the formula II in which $R_1$ is hydrogen or methyl, $R_2$ is hydrogen or a hydroxy-protecting group $R_2'$, $R_3$ is phenyl and $R_4$ is hydrogen or an amino-protecting group $R_4'$.

**3.** A process according to claim 1, characterised in that there is used as starting material a compound of the formula II in which $R_1$ is hydrogen or methyl, $R_2$ is hydrogen, 2,2,2-trichloroethoxycarbonyl, allyloxycarbonyl or 3,5-dinitrobenzoyl, $R_3$ is phenyl and $R_4$ is hydrogen or p-methoxyphenyl.

**4.** A process for the manufacture of (3R,4R,1'R)-N-p-methoxyphenyl-3-(1'-hydroxyethyl)-4-benzoyloxyazetidin-2-one according to claim 1.

**5.** A process for the manufacture of (3R,4R,1'R)-3-(1'-hydroxyethyl)-4-benzoyloxyazetidin-2-one according to claim 1.

**6.** A process for the manufacture of (3R,4R,1'R)-3-[1'-(2,2,2-trichloroethoxycarbonyloxy)-ethyl]-4-benzoyloxyazetidin-2-one according to claim 1.

**7.** A process for the manufacture of (3R,4R,1'R)-3-[1'-(dimethyl-2,3-dimethylbut-2-ylsilyloxy)-ethyl]-4-benzoyloxyazetidin-2-one according to claim 1.

**8.** A process for the manufacture of (3R,4R,1'R)-3-[1'-(3,5-dinitrobenzoyloxy)-ethyl]-4-benzoyloxyazetidin-2-one according to claim 1.

**9.** A process for the manufacture of (3R,4R,1'R)-3-(1'-allyloxycarbonyloxyethyl)-4-benzoyloxyazetidin-2-one according to claim 1.

**10.** A process for the manufacture of a compound of the formula

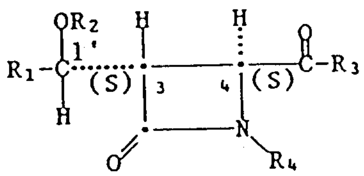

(II)

in which $R_1$ is hydrogen or lower alkyl, $R_2$ is hydrogen or a hydroxy-protecting group $R_2'$, $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$, characterised in that a) an $\alpha$-carbanion of a compound of the formula

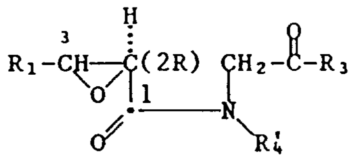

(III),

in which $R_1$, $R_3$ and $R_4'$ have the meanings mentioned under formula II and the 2-carbon atom has the R-configuration and, if $R_1$ is $C_1$-$C_4$alkyl, the 3-carbon atom has the R- or the S-configuration, or a compound of the formula

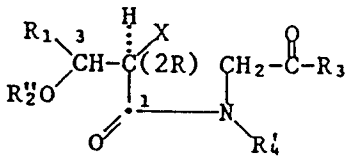

(IV),

in which $R_1$, $R_3$ and $R_4'$ have the meanings given under formula II, $R_2''$ is a hydroxy-protecting group that cannot be removed under the conditions of the cyclisation process and X is a nucleofugal leaving group, and the 2-carbon atom has the R-configuration and, if $R_1$ is $C_1$-$C_4$alkyl, the 3-carbon atom has the R- or the S-configuration, is cyclised, or b) a cis-compound of the formula

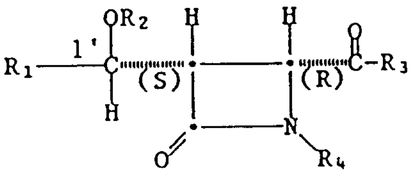

(II'),

in which $R_1$, $R_2$, $R_3$ and $R_4$ have the meanings given under formula II, is isomerised, and, if desired, the amino-protecting group $R_4'$ is removed and replaced by hydrogen and/or, if desired, an obtainable compound of the formula II is converted into a different compound of the formula II.

**11.** A process according to claim 10 for the manufacture of a compound of the formula II in which $R_1$ is hydrogen or methyl, $R_2$ is hydrogen or a hydroxy-protecting group $R_2'$, $R_3$ is phenyl and $R_4$ is hydrogen or an amino-protecting group $R_4'$, characterised in that a) an $\alpha$-carbanion of a compound of the formula

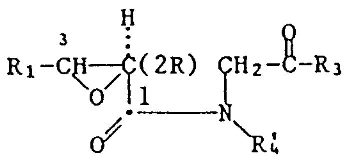

(III),

in which $R_1$, $R_3$ and $R_4'$ have the meanings mentioned under formula II and the 2-carbon atom has the R-configuration and, if $R_1$ is methyl, the 3-carbon atom has the R- or the S-configuration, or a compound of the formula

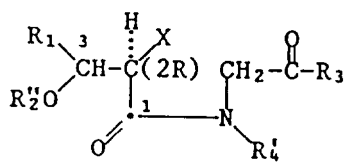

(IV),

in which $R_1$, $R_3$ and $R_4'$ have the meanings mentioned under formula II, $R_2''$ is a hydroxy-protecting group that cannot be removed under the conditions of the cyclisation process and X is a nucleofugal leaving group, and the 2-carbon atom has the R-configuration and, if $R_1$ is methyl, the 3-carbon atom has the R- or the S-configuration, is cyclised and, if desired, the amino-protecting group $R_4'$ is removed and replaced by hydrogen and/or, if desired, an obtainable compound of the formula II is converted into a different compound of the formula II.

**12.** A process according to claim 10 for the manufacture of a compound of the formula II in which $R_1$ is methyl, $R_2$ is hydrogen, 2,2,2-trichloroethoxycarbonyl, allyloxycarbonyl, tert-butyldimethylsilyl, dimethyl-2,3-dimethylbut-2-ylsilyl or 3,5-dinitrobenzoyl, $R_3$ is phenyl and $R_4$ is hydrogen or p-methoxyphenyl.

**13.** A process according to claim 11 for the manufacture of (3S,4S,1'R)-N-p-methoxyphenyl-3-(1'-hydroxyethyl)-4-benzoylazetidin-2-one.

**14.** A process for the manufacture of (3S,4S,1'R)-3-(1'-hydroxyethyl)-4-benzoylazetidin-2-one, (3S,4S,1'R)-N-p-methoxyphenyl-3-[1'-(2,2,2-trichloroethoxycarbonyloxy)-ethyl]-4-benzoylazetidin-2-one, (3S,4S,1'R)-3-[1'-(2,2,2-trichloroethoxycarbonyloxy)-ethyl]-4-benzoylazetidin-2-one, (3S,4S,1'R)-N-p-methoxyphenyl-3-(1'-allyloxycarbonylox yethyl)-4-benzoylazetidin-2-one, and (3S,4S,1'R)-(1'-allyloxycarbonyloxyethyl)-4-benzoylazetidin-2-one according to claim 11.

**15.** A process for the manufacture of (3S,4S,1'R)-N-p-methoxyphenyl-3-(1'-hydroxyethyl)-4-benzoylazetidin-2-one, (3S,4S,1'R)-N-p-methoxyphenyl-3-[1'-(3,5-dinitrobenzoyloxy)-ethyl]-4-benzoylazetidin-2-one, (3S,4S,1'R)-3-[1'-(3,5-dinitrobenzoyloxy)-ethyl]-4-benzoylazetidin-2-one or (3S,4S,1'R)-N-p-methoxyphenyl-3-[1'-(dimethyl-2,3-dimethylbut-2-ylsilyloxy)-ethyl]-4-benzoylazetidin-2-one according to claim 11.

**16.** A process for the manufacture of (3S,4S,1'R)-3-[1'-(dimethyl-2,3-dimethylbut-2-ylsilyloxy)-ethyl]-4-benzoylazetidin-2-one according to claim 11.

**17.** A process for the manufacture of a (4R)-3,4-trans-disubstituted azetidinone of the formula

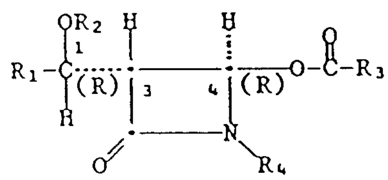

(I)

in which $R_1$ is hydrogen or $C_1$-$C_4$alkyl, $R_2$ is hydrogen or a hydroxy-protecting group $R_2'$ , $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino protecting group $R_4$, characterised in that a compound of the formula

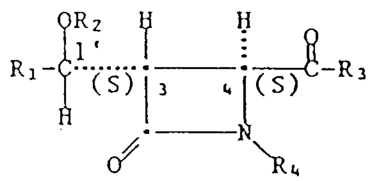

(II),

in which $R_1$, $R_2$, $R_3$ and $R_4$ have the meanings mentioned under formula I, is subjected to a Bayer-Villiger reaction by treatment with a per acid, and, if desired, in an obtainable compound of the formula I in which $R_2$ is hydrogen, the free hydroxy group is converted into a protected hydroxy group $OR_2'$ and/or, if desired, in an obtainable compound of the formula I the hydroxy-protecting group $R_2'$ and/or the amino-protecting group $R_4'$ are(is) removed,
the process for the manufacture of a compound of the formula

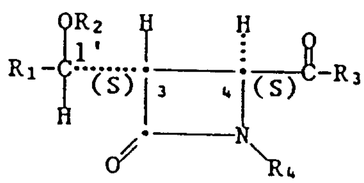

(II),

in which $R_1$ is hydrogen or $C_1$-$C_4$alkyl, $R_2$ is hydrogen or a hydroxy-protecting group $R_2'$ , $R_3$ is phenyl, phenyl that is mono- to tri-substituted by $C_1$-$C_4$alkoxy, $C_1$-$C_4$alkyl and/or by halogen, or is 4-nitrophenyl and $R_4$ is hydrogen or an amino-protecting group $R_4'$ , being characterised in that a) an $\alpha$-carbanion of a compound of the formula

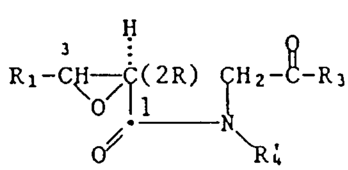

(III),

in which $R_1$, $R_3$ and $R_4'$ have the meanings mentioned under formula II and the 2-carbon atom has the R-configuration and, if $R_1$ is $C_1$-$C_4$alkyl, the 3-carbon atom has the R- or the S-configuration, or a compound of the formula

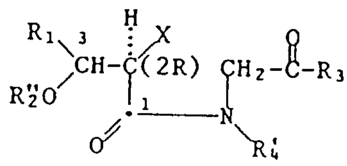

(IV),

in which $R_1$, $R_3$ and $R_4'$ have the meanings mentioned under formula II, $R_2''$ is a hydroxy-protecting group that cannot be removed under the conditions of the cyclisation process and X is a nucleofugal leaving group, and the 2-carbon atom has the R-configuration and, if $R_1$ is $C_1$-$C_4$alkyl, the 3-carbon atom has the R- or the S-configuration, is cyclised or b) a cis-compound of the formula

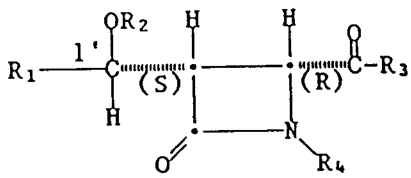

(II′),

in which $R_1$, $R_2$, $R_3$ and $R_4$ have the meanings indicated under formula II, is isomerised, and, if desired, the amino-protecting group $R_4'$ is removed and replaced by hydrogen and/or, if desired, an obtainable compound of the formula II is converted into a different compound of the formula II,
the process for the manufacture of a compound of the formula

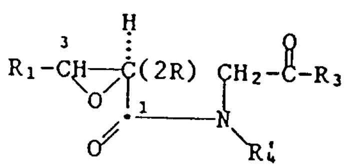

(III),

in which $R_1$ is hydrogen or $C_1$-$C_4$alkyl, $R_3$ is phenyl, phenyl that is mono- to tri-substituted by $C_1$-$C_4$alkoxy,$C_1$-$C_4$alkyl and/or by halogen, or is 4-nitrophenyl and $R_4'$ is an amino-protecting group, and the 2-carbon atom has the R-configuration and, if $R_1$ is $C_1$-$C_4$alkyl, the 3-carbon atom has the R- or the S-configuration, being characterised in that a compound of the formula

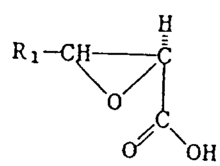

(VIII)

is reacted with an amine of the formula

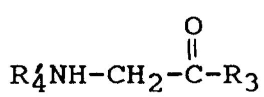

(VII)

in which $R_3$ and $R_4'$ have the meanings mentioned under formula V, or a compound of the formula III is manufactured, under carbanion-forming conditions and with a Walden inversion at the 2-carbon atom, from a compound of the formula

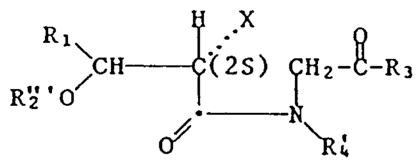

(V)

in which $R_1$, $R_3$, $R_4'$ and X have the meanings mentioned, $R_2'''$ is hydrogen or a hydroxy-protecting group that can be removed under the conditions of the epoxide formation, and in which the 3-carbon atom has the R- or the S-configuration if $R_1$ is $C_1$-$C_4$alkyl,
the process for the manufacture of a compound of the formula

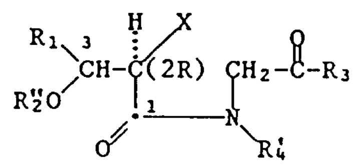

(IV),

in which $R_1$ is hydrogen or $C_1$-$C_4$alkyl, $R_2''$ is a hydroxy-protecting group that cannot be removed under the conditions of the cyclisation process, $R_3$ is phenyl, phenyl that is mono- to tri-substituted by $C_1$-$C_4$alkoxy,$C_1$-$C_4$alkyl and/or by halogen, or is 4-nitrophenyl, $R_4'$ is an amino-protecting group and X is a nucleofugal leaving group, and the 2-carbon atom has the R-configuration and, if $R_1$ is $C_1$-$C_4$alkyl, the 3-carbon atom has the R- or the S-configuration, being characterised in that a carboxylic acid of the formula

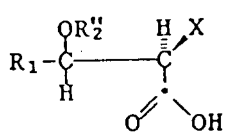

(VI'),

in which $R_1$, $R_2''$ and X have the meanings mentioned under formula IV, is reacted with an amine of the formula

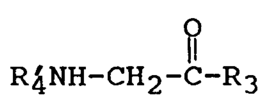

(VII)

in which $R_3$ and $R_4'$ have the meanings mentioned under formula V, and
the process for the manufacture of a compound of the formula

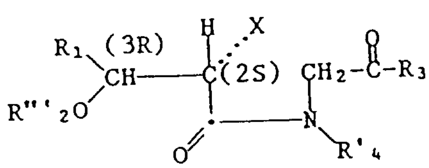

(V),

in which $R_1$ is hydrogen or $C_1$-$C_4$alkyl, $R_2'''$ is hydrogen or a hydroxy-protecting group that can be removed under the conditions of the epoxide formation, $R_3$ is phenyl, phenyl that is mono- to tri-substituted by $C_1$-$C_4$alkoxy, $C_1$-$C_4$alkyl and/or by halogen, or is 4-nitrophenyl, $R_4$ is an amino-protecting group and X is a nucleofugal leaving group and in which the 3-carbon atom has the R-configuration if $R_1$ is $C_1$-$C_4$alkyl, being characterised in that a carboxylic acid of the formula

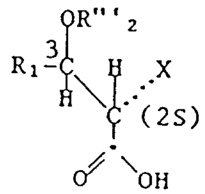

(VI),

in which $R_1$, $R_2'''$ and X have the meanings mentioned under formula V, is reacted with an amine of the formula

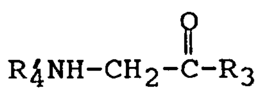

(VII)

in which $R_3$ and $R'_4$ have the meanings mentioned under formula V.

**Revendications**

**Revendications pour les Etats contractants suivants : BE, CH, DE, FR, GB, IT, LI, NL, SE**

**1.** Azétidinones (4R)-3,4-trans-disubstituées de formule

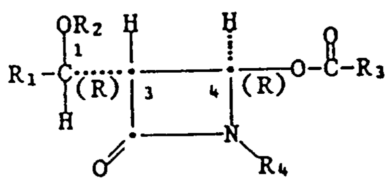

(I),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle et $R_4$ représente l'hydrogène ou un groupe protecteur du groupe amino $R'_4$.

**2.** Composés de formule I de la revendication 1, dans laquelle $R_1$ représente l'hydrogène ou un groupe méthyle, $R_2$ représente l'hydrogène, un groupe 2,2,2-trichloréthoxycarbonyle, allyloxycarbonyle ou 3,5-dinitrobenzoyle, $R_3$ représente un groupe phényle et $R_4$ l'hydrogène ou un groupe p-méthoxyphényle.

**3.** La (3R,4R,1'R)-N-p-méthoxyphényl-3-(1'-hydroxyéthyl)-4-benzoyloxyazétidine-2-one selon revendication 1.

**4.** La (3R,4R,1'R)-3-(1'-hydroxyéthyl)-4-benzoyloxyazétidine-2-one selon revendication 1.

**5.** La (3R,4R,1'R)-3-[1'-(2,2,2-trichloréthoxycarbonyloxy)-éthyl]-4-benzoyloxyazétidine-2-one selon revendication 1.

**6.** La (3R,4R,1'R)-3-(1'-allyloxycarbonyléthyl)-4-benzoyloxyazétidine-2-one selon revendication 1.

**7.** La (3R,4R,1'R)-3-[1'-(diméthyl-2,3-diméthylbuta-2-ylsilyloxy)-éthyl]-4-benzoyloxyazétidine-2-one selon revendication 1.

**8.** La (3R,4R,1'R)-3-[1'-(3,5-dinitrobenzoyloxy)-éthyl]-4-benzoyloxyazétidine-2-one selon revendication 1.

**9.** Procédé de préparation des azétidinones (4R)-3,4-trans-disubstituées de formule

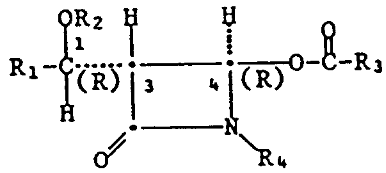

(I),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle et $R_4$ représente l'hydrogène ou un groupe protecteur du groupe amino $R'_4$, caractérisé en ce que l'on soumet un composé de formule

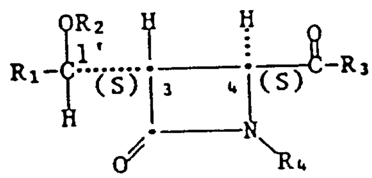

(II),

dans laquelle $R_1$, $R_2$, $R_3$ et $R_4$ ont les significations indiquées en référence à la formule I, à une réaction de Bayer-Villiger par traitement à l'aide d'un peracide et, si on le désire, dans un composé ainsi obtenu et répondant à la formule I dans laquelle $R_2$ représente l'hydrogène, on convertit le groupe hydroxy libre en le groupe hydroxy protégé $OR'_2$ et/ou, si on le désire, dans un composé ainsi obtenu répondant à la formule I, on scinde le groupe protecteur du groupe hydroxy $R'_2$ et/ou le groupe protecteur du groupe amino $R'_4$.

**10.** Procédé de préparation des composés de formule I selon revendication 9, caractérisé en ce que l'on part de composés de formule II dans laquelle $R_1$ représente l'hydrogène ou un groupe méthyle, $R_2$ représente l'hydrogène ou un groupe protecteur du groupe hydroxy $R'_2$, $R_3$ représente un groupe phényle et $R_4$ l'hydrogène ou un groupe protecteur du groupe amino $R'_4$.

**11.** Un composé de formule

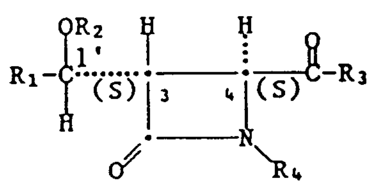

(II),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle et $R_4$ représente l'hydrogène ou un groupe protecteur du groupe amino $R'_4$.

**12.** Un composé de formule II de la revendication 11, dans laquelle $R_1$ représente l'hydrogène ou un groupe méthyle, $R_2$ représente l'hydrogène ou un groupe protecteur du groupe hydroxy $R'_2$, $R_3$ représente un groupe phényle et $R_4$ l'hydrogène ou un groupe protecteur du groupe amino $R'_4$.

**13.** Un composé de formule II de la revendication 11, dans laquelle $R_1$ représente un groupe méthyle, $R_2$ représente l'hydrogène, un groupe 2,2,2-trichloréthoxycarbonyle,allyloxycarbonyle, tert-butyl-diméthylsilyle, diméthyl-2,3-diméthylbuta-2-ylsilyle ou 3,5-dinitrobenzoyle, $R_3$ représente un groupe phényle et $R_4$

l'hydrogène ou un groupe p-méthoxyphényle.

**14.** La (3S,4S,1'R)-N-p-méthoxyphényl-3-(1'-hydroxyéthyl)-4-benzoylazétidine-2-one selon revendication 11.

**15.** La (3S,4S,1'R)-3-(1'-hydroxyéthyl)-4-benzoylazétidine-2-one,
la (3S,4S,1'R)-N-p-méthoxyphényl-3-[1'-(2,2,2-trichloréthoxycarbonyloxy)éthyl]-4-benzoylazétidine-2-one,
la (3S,4S,1'R)-3-[1'-(2,2,2-trichloréthoxycarbonyloxy)éthyl]-4-benzoylazétidine-2-one,
la (3S,4S,1'R)-N-p-méthoxyphényl-3-(1'-allyloxycarbonyloxyéthyl)-4-benzoylazétidine-2-one et
la (3S,4S,1'R)-3-(1'-allyloxycarbonyloxyéthyl)-4-benzoylazétidine-2-one selon revendication 11.

**16.** La (3S,4S,1'R)-N-p-méthoxyphényl-3-(1'-hydroxyéthyl)-4-benzoylazétidine-2-one,
la (3S,4S,1'R)-N-p-méthoxypnényl-3-[1'-(3,5-dinitrobenzoyloxy)éthyl]-4-benzoylazétidine-2-one et
la (3S,4S,1'R)-3-[1'-(3,5-dinitrobenzoyloxy)éthyl]-4-benzoylazétidine-2-one selon revendication 11.

**17.** La (3S,4S,1'R)-N-p-méthoxyphényl-3-[1'-(diméthyl-2,3-diméthylbuta-2-ylsilyloxy)éthyl]-4-benzoylazétidine-2-one selon revendication 11.

**18.** La (3S,4S,1'R)-3-[1'-(diméthyl-2,3-diméthylbuta-2-ylsilyloxy)éthyl]-4-benzoylazétidine-2-one selon revendication 11.

**19.** Procédé de préparation des composés de formule

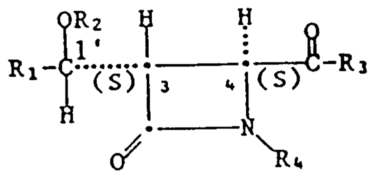

(II),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle et $R_4$ représente l'hydrogène ou un groupe protecteur du groupe amino $R'_4$, caractérisé en ce que a) on cyclise un alpha-carbanion d'un composé de formule

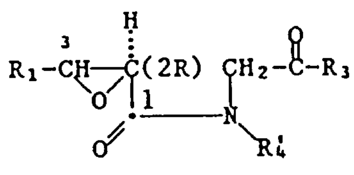

(III),

dans laquelle $R_1$, $R_3$ et $R'_4$ ont les significations indiquées en référence à la formule II, et l'atome de carbone en position 2 a la configuration R et, lorsque $R_1$ représente un groupe alkyle en C1-C4, l'atome de carbone en position 3 a la configuration R ou S, ou un composé de formule

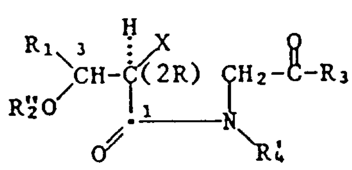

(IV),

dans laquelle $R_1$, $R_3$ et $R'_4$ ont les significations indiquées en référence à la formule II, $R''_2$ représente un groupe protecteur du groupe hydroxy non scindable dans les conditions de la cyclisation, et X représente un groupe éliminable nucléofuge, et l'atome de carbone en position 2 est en configuration R

et, lorsque $R_1$ représente un groupe alkyle en C1-C4, l'atome de carbone en position 3 est en configuration R ou S, ou bien b) on isomérise un composé à l'état d'isomère cis, de formule

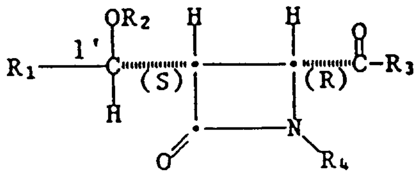

(II'),

dans laquelle $R_1$, $R_2$, $R_3$ et $R_4$ ont les significations indiquées en référence à la formule II et, si on le désire, on scinde le groupe protecteur du groupe amino $R'_4$ et on le remplace par l'hydrogène et/ou, si on le désire, on convertit un composé ainsi obtenu, répondant à la formule II, en un autre composé de formule II.

**20.** Procédé selon la revendication 19, pour la préparation des composés de formule II dans laquelle $R_1$ représente l'hydrogène ou un groupe méthyle, $R_2$ représente l'hydrogène ou un groupe protecteur du groupe hydroxy $R'_2$, $R_3$ représente un groupe phényle et $R_4$ l'hydrogène ou un groupe protecteur du groupe amino $R'_4$, caractérisé en ce que a) on cyclise un alpha-carbanion d'un composé de formule

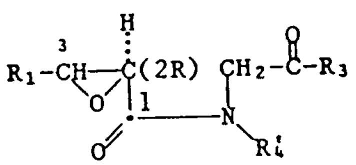

(III),

dans laquelle $R_1$, $R_3$ et $R'_4$ ont les significations indiquées en référence à la formule II et l'atome de carbone en position 2 est en configuration R et, lorsque $R_1$ représente un groupe méthyle, l'atome de carbone en position 3 est en configuration R ou S, ou un composé de formule

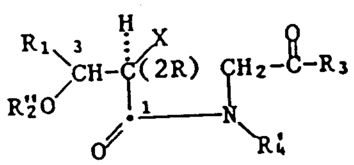

(IV),

dans laquelle $R_1$, $R_3$ et $R'_4$ ont les significations indiquées en référence à la formule II, $R''_2$ représente un groupe protecteur du groupe hydroxy non scindable dans les conditions de la cyclisation, et X représente un groupe éliminable nucléofuge, et l'atome de carbone en position 2 est en configuration R et, lorsque $R_1$ représente un groupe méthyle, l'atome de carbone en position 3 est en configuration R ou S, et si on le désire, on scinde le groupe protecteur du groupe amino $R'_4$ et on le remplace par l'hydrogène, et/ou, si on le désire, on convertit un composé ainsi obtenu, répondant à la formule II, en un autre composé de formule II.

**21.** Procédé de préparation des azétidinones (4R)-3,4-trans-disubstituées de formule

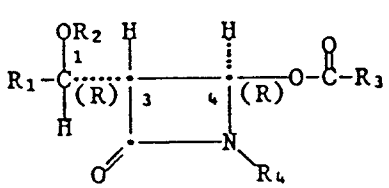

(I),

dans laquelle $R_1$ représente l'hydrogène ou un groupe alkyle en C1-C4, $R_2$ représente l'hydrogène ou

un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle, et $R_4$ représente l'hydrogène ou un groupe protecteur du groupe amino $R'_4$, caractérisé en ce que l'on soumet un composé de formule

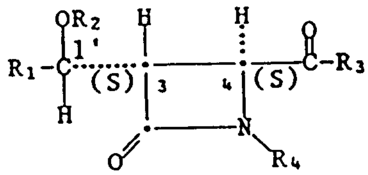

(II),

dans laquelle $R_1$, $R_2$, $R_3$ et $R_4$ ont les significations indiquées en référence à la formule I, à une réaction de Bayer-Villiger par traitement à l'aide d'un peracide, et si on le désire, dans un composé ainsi obtenu répondant à la formule I dans laquelle $R_2$ représente l'hydrogène, on convertit le groupe hydroxy libre en le groupe hydroxy protégé $OR'_2$, et/ou, si on le désire, dans un composé ainsi obtenu, répondant à la formule I, on scinde le groupe protecteur du groupe hydroxy $R'_2$ et/ou le groupe protecteur du groupe amino $R'_4$,
le procédé de préparation des composés de formule

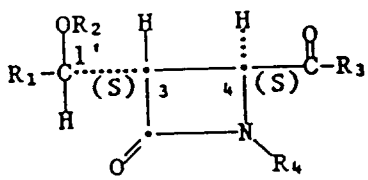

(II),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle et $R_4$ représente l'hydrogène ou un groupe protecteur du groupe amino $R'_4$, se caractérisant alors en ce que a) on cyclise un alpha-carbanion d'un composé de formule

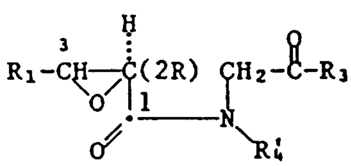

(III),

dans laquelle $R_1$, $R_3$ et $R'_4$ ont les significations indiquées en référence à la formule II, et l'atome de carbone en position 2 est en configuration R et, lorsque $R_1$ représente un groupe alkyle en C1-C4, l'atome de carbone en position 3 est en configuration R ou S, ou un composé de formule

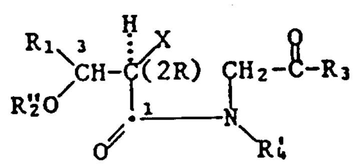

(IV),

dans laquelle $R_1$, $R_3$ et $R'_4$ ont les significations indiquées en référence à la formule II, $R''_2$ représente un groupe protecteur du groupe hydroxy non scindable dans les conditions de la cyclisation, et X représente un groupe éliminable nucléofuge, et l'atome de carbone en position 2 est en configuration R et, lorsque $R_1$ représente un groupe alkyle en C1-C4, l'atome de carbone en position 3 est en configuration R ou S, ou bien b), on isomérise un composé, à l'état d'isomère cis, de formule

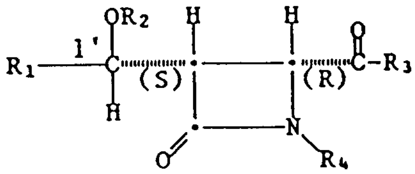

(II'),

dans laquelle $R_1$, $R_2$, $R_3$ et $R_4$ ont les significations indiquées en référence à la formule II, et si on le désire, on scinde le groupe protecteur du groupe amino $R'_4$ et on le remplace par l'hydrogène, et/ou, si on le désire, on convertit un composé ainsi obtenu, répondant à la formule II, en un autre composé de formule II,

le procédé de préparation des composés de formule

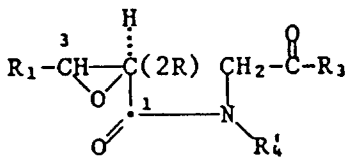

(III),

dans laquelle $R_1$ représente l'hydrogène ou un groupe alkyle en C1-C4, $R_3$ représente un groupe phényle, un groupe phényle mono- à tri-substitué par des groupes alcoxy en C1-C4, alkyle en C1-C4 et/ou des halogènes, ou un groupe 4-nitrophényle, et $R'_4$ représente un groupe protecteur du groupe amino, et l'atome de carbone en position 2 est en configuration R et, lorsque $R_1$ représente un groupe alkyle en C1-C4, l'atome de carbone en position 3 est en configuration R ou S, se caractérisant alors en ce que l'on fait réagir un composé de formule

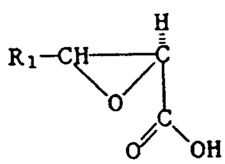

(VIII)

avec une amine de formule

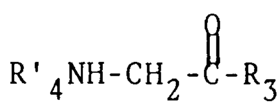

(VII)

dans laquelle $R_3$ et $R'_4$ ont les significations indiquées en référence à la formule V, ou bien on les prépare à partir d'un composé de formule

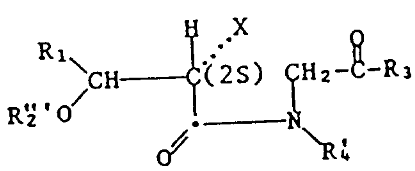

(V),

dans laquelle $R_1$, $R_3$, $R'_4$ et X ont les significations indiquées ci-dessus, $R'''_2$ représente l'hydrogène ou un groupe protecteur du groupe hydroxy scindable dans les conditions de formation de l'époxyde, et dans laquelle l'atome de carbone en position 3 est en configuration R ou S lorsque $R_1$ représente un groupe alkyle en C1-C4, dans des conditions propres à la formation de carbanions et avec inversion de Walden sur l'atome de carbone en position 2,

le procédé de préparation des composés de formule

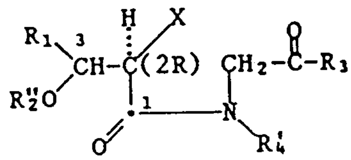

(IV),

dans laquelle $R_1$ représente l'hydrogène ou un groupe alkyle en C1-C4, $R''_2$ représente un groupe protecteur du groupe hydroxy non scindable dans les conditions de la cyclisation, $R_3$ représente un groupe phényle, un groupe phényle mono- à tri-substitué par des groupes alcoxy en C1-C4, alkyle en C1-C4 et/ou des halogènes, ou un groupe 4-nitrophényle, $R'_4$ représente un groupe protecteur du groupe amino, et X un groupe éliminable nucléofuge, et l'atome de carbone en position 2 est en configuration R et, lorsque $R_1$ représente un groupe alkyle en C1-C4, l'atome de carbone en position 3 est en configuration R ou S, se caractérisant alors en ce que l'on fait réagir un acide carboxylique de formule

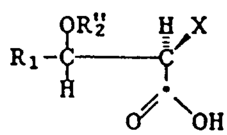

(VI')

dans laquelle $R_1$, $R''_2$ et X ont les significations indiquées en référence à la formule IV, avec une amine de formule

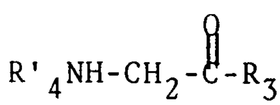

(VII),

dans laquelle $R_3$ et $R'_4$ ont les significations indiquées en référence à la formule V,
le procédé de préparation des composés de formule

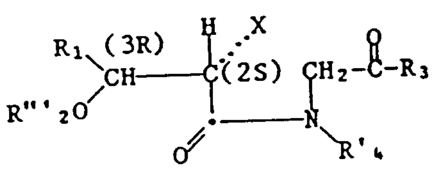

(V),

dans laquelle $R_1$ représente l'hydrogène ou un groupe alkyle en C1-C4, $R'''_2$ représente l'hydrogène ou un groupe protecteur du groupe hydroxy scindable dans les conditions de formation de l'époxyde, $R_3$ représente un groupe phényle, un groupe phényle mono- à tri-substitué par des groupes alcoxy en C1-C4, alkyle en C1-C4 et/ou des halogènes, ou un groupe 4-nitrophényle, $R_4$ représente un groupe protecteur du groupe amino et X un groupe éliminable nucléofuge, et l'atome de carbone en position 3 est en configuration R lorsque $R_1$ représente un groupe alkyle en C1-C4, se caractérisant alors en ce que l'on fait réagir un acide carboxylique de formule

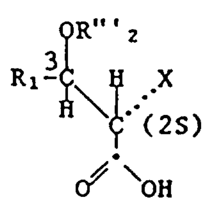

(VI)

dans laquelle $R_1$, $R'''_2$ et X ont les significations indiquées en référence à la formule V, avec une amine de formule

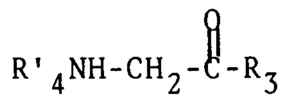

(VII),

dans laquelle $R_3$ et $R'_4$ ont les significations indiquées en référence à la formule V.

**Revendications pour l'Etat contractant suivant : AT**

1. Procédé de préparation des azétidinones (4R)-3,4-trans-disubstituées de formule

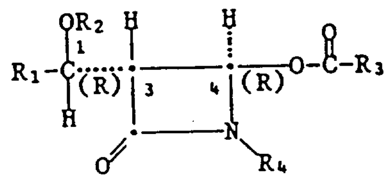

(I),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alocxy en C1-C4, alkyle en C1-C4 et/ou des halogènes, ou un groupe 4-nitrophényle et $R_4$ représente l'hydrogène ou un groupe protecteur du groupe amino $R'_4$, caractérisé en ce que l'on soumet un composé de formule

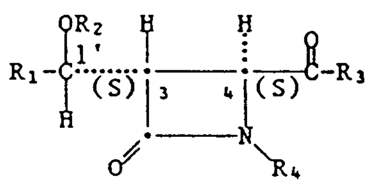

(II),

dans laquelle $R_1$, $R_2$, $R_3$ et $R_4$ ont les significations indiquées en référence à la formule I, à une réaction de Bayer-Villiger par traitement à l'aide d'un peracide et, si on le désire, dans un composé ainsi obtenu et répondant à la formule I dans laquelle $R_2$ représente l'hydrogène, on convertit le groupe hydroxy libre en le groupe hydroxy protégé $OR'_2$ et/ou, si on le désire, dans un composé ainsi obtenu répondant à la formule I, on scinde le groupe protecteur du groupe hydroxy $R'_2$ et/ou le groupe protecteur du groupe amino $R'_4$.

2. Procédé de préparation des composés de formule I selon revendication 1, caractérisé en ce que l'on part de composés de formule II dans laquelle $R_1$ représente l'hydrogène ou un groupe méthyle, $R_2$ l'hydrogène ou un groupe protecteur du groupe hydroxy $R'_2$, $R_3$ un groupe phényle et $R_4$ l'hydrogène ou un groupe protecteur du groupe amino $R'_4$.

3. Procédé selon la revendication 1, caractérisé en ce que l'on part de composés de formule II dans laquelle $R_1$ représente l'hydrogène ou un groupe méthyle, $R_2$ représente l'hydrogène, un groupe 2,2,2-trichloréthoxycarbonyle, allyloxycarbonyle ou 3,5-dinitrobenzoyle, $R_3$ un groupe phényle et $R_4$ l'hydrogène ou un groupe p-méthoxyphényle.

4. Procédé de préparation de la (3R,4R, 1'R)-N-p-méthoxyphényl-3-(1'-hydroxyéthyl)-4-benzoyloxyazétidine-2-one selon revendication 1.

**5.** Procédé de préparation de la (3R,4R, 1'R)-3-(1'-hydroxyéthyl)-4-benzoyloxyazétidine-2-one selon revendication 1.

**6.** Procédé de préparation de la (3R,4R, 1'R)-3-[1'-(2,2,2-trichloréthoxycarbonyloxy)éthyl]-4-benzoyloxyazétidine-2-one selon revendication 1.

**7.** Procédé de préparation de la (3R,4R, 1'R)-3-[1'-(diméthyl-2,3-diméthylbuta-2-ylsilyloxy)éthyl]-4-benzoyloxyazétidine-2-one selon revendication 1.

**8.** Procédé de préparation de la (3R,4R, 1'R)-3-[1'-(3,5-dinitrobenzoyloxy)éthyl]-4-benzoyloxyazétidine-2-one selon revendication 1.

**9.** Procédé de préparation de la (3R,4R, 1'R)-3-(1'-allyloxycarbonyloxyéthyl)-4-benzoyloxyazétidine-2-one selon revendication 1.

**10.** Procédé de préparation des composés de formule

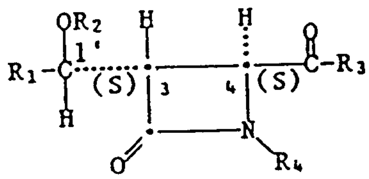

(II),

dans laquelle $R_1$ représente l'hydrogène ou un groupe alkyle en $C_1$-$C_4$ , $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle et $R_4$ représente l'hydrogène ou un groupe protecteur du groupe amino $R'_4$, caractérisé en ce que a) on cyclise un alpha-carbanion d'un composé de formule

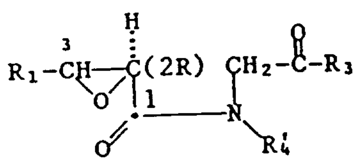

(III),

dans laquelle $R_1$, $R_3$ et $R'_4$ ont les significations indiquées en référence à la formule II, et l'atome de carbone en position 2 a la configuration R et, lorsque $R_1$ représente un groupe alkyle en C1-C4, l'atome de carbone en position 3 a la configuration R ou S, ou un composé de formule

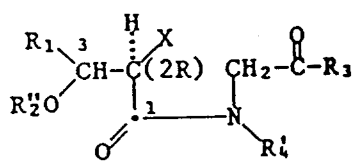

(IV),

dans laquelle $R_1$, $R_3$ et $R'_4$ ont les significations indiquées en référence à la formule II, $R''_2$ représente un groupe protecteur du groupe hydroxy non scindable dans les conditions de la cyclisation, et X représente un groupe éliminable nucléofuge, et l'atome de carbone en position 2 est en configuration R et, lorsque $R_1$ représente un groupe alkyle en C1-C4, l'atome de carbone en position 3 est en configuration R ou S, ou bien b) on isomérise un composé à l'état d'isomère cis, de formule

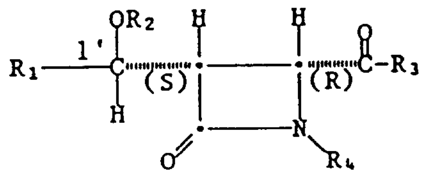

(II'),

dans laquelle $R_1$, $R_2$, $R_3$ et $R_4$ ont les significations indiquées en référence à la formule II et, si on le désire, on scinde le groupe protecteur du groupe amino $R'_4$ et on le remplace par l'hydrogène et/ou, si on le désire, on convertit un composé ainsi obtenu, répondant à la formule II, en un autre composé de formule II

**11.** Procédé selon la revendication 10, pour la préparation des composés de formule II dans laquelle $R_1$ représente l'hydrogène ou un groupe méthyle, $R_2$ représente l'hydrogène ou un groupe protecteur du groupe hydroxy $R'_2$, $R_3$ représente un groupe phényle et $R_4$ l'hydrogène ou un groupe protecteur du groupe amino $R'_4$, caractérisé en ce que a) on cyclise un alpha-carbanion d'un composé de formule

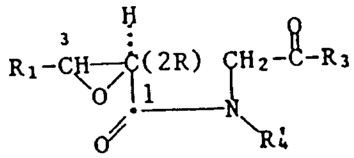

(III),

dans laquelle $R_1$, $R_3$ et $R'_4$ ont les significations indiquées en référence à la formule II et l'atome de carbone en position 2 est en configuration R et, lorsque $R_1$ représente un groupe méthyle, l'atome de carbone en position 3 est en configuration R ou S, ou un composé de formule

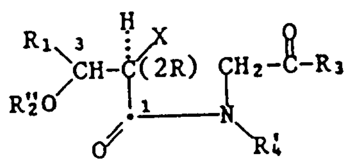

(IV),

dans laquelle $R_1$, $R_3$ et $R'_4$ ont les significations indiquées en référence à la formule II, $R''_2$ représente un groupe protecteur du groupe hydroxy non scindable dans les conditions de la cyclisation, et X représente un groupe éliminable nucléofuge, et l'atome de carbone en position 2 est en configuration R et, lorsque $R_1$ représente un groupe méthyle, l'atome de carbone en position 3 est en configuration R ou S, et si on le désire, on scinde le groupe protecteur du groupe amino $R'_4$ et on le remplace par l'hydrogène, et/ou, si on le désire, on convertit un composé ainsi obtenu, répondant à la formule II, en un autre composé de formule II.

**12.** Procédé selon la revendication 10, pour la préparation des composés de formule II dans laquelle $R_1$ représente un groupe méthyle, $R_2$ représente l'hydrogène, un groupe 2,2,2-trichloréthoxycarbonyle, allyloxycarbonyle, tert-butyldiméthylsilyle, diméthyl-2,3-diméthylbuta-2-ylsilyle ou un groupe 3,5-dinitrobenzoyle, $R_3$ représente un groupe phényle et $R_4$ un groupe p-méthoxyphényle.

**13.** Procédé selon revendication 11 pour la préparation de la (3S,4S,1'R)-N-p-méthoxyphényl-3-(1'-hydroxyéthyl)-4-benzoylazétidine-2-one.

**14.** Procédé de préparation de la (3S,4S,1'R)-3-(1'-hydroxyéthyl)-4-benzoylazétidine-2-one,
de la (3S,4S,1'R)-N-p-méthoxyphényl-3-[1'-(2,2,2-trichlorthoxycarbonyloxy)éthyl]-4-benzoylazétidine-2-one,
de la (3S,4S,1'R)-3-[1'-(2,2,2-trichloréthoxycarbonyloxy)éthyl]-4-benzoylazétidine-2-one,
de la (3S,4S,1'R)-N-p-méthoxyphényl-3-(1'-allyloxycarbonyloxyéthyl)-4-benzoylazétidine-2-one et
de la (3S,4S,1'R)-3- (1'-allyloxycarbonyloxyéthyl)-4-benzoylazétidine-2-one selon revendication 11.

**15.** Procédé de préparation de la (3S,4S,1'R)-N-p-méthoxyphényl-3-(1'-hydroxyéthyl)-4-benzoylazétidine-2-one,
de la (3S,4S,1'R)-N-p-méthoxyphényl-3-[1'-(3,5-dinitrobenzoyloxy)éthyl]-4-benzoylazétidine-2-one,
de la (3S,4S,1'R)-3-[1'-(3,5-dinitrobenzoyloxy)éthyl]-4-benzoylazétidine-2-one, ou
de la (3S,4S,1'R)-N-p-méthoxyphényl-3-[1'-(diméthyl)-2,3-diméthylbuta-2-ylsilyloxy)éthyl]-4-benzoylazétidine-2-one selon revendication 11.

**16.** Procédé de préparation de la (3S,4S,1'R)-3-[1'-(diméthyl-2,3-diméthylbuta-2-ylsilyloxy)éthyl]-4-benzoylazétidine-2-one selon revendication 11.

**17.** Procédé de préparation des azétidinones (4R)-3,4-trans-disubstituées de formule

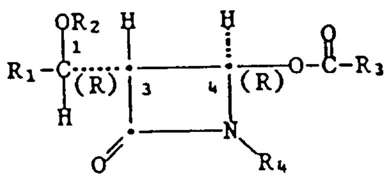

(I),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle, et $R_4$ représente l'hydrogène ou un groupe protecteur du groupe amino $R'_4$, caractérisé en ce que l'on soumet un composé de formule

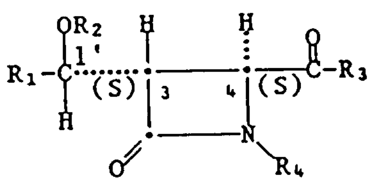

(II),

dans laquelle $R_1$, $R_2$, $R_3$ et $R_4$ ont les significations indiquées en référence à la formule I, à une réaction de Bayer-Villiger par traitement à l'aide d'un peracide, et si on le désire, dans un composé ainsi obtenu répondant à la formule I dans laquelle $R_2$ représente l'hydrogène, on convertit le groupe hydroxy libre en le groupe hydroxy protégé $OR'_2$, et/ou, si on le désire, dans un composé ainsi obtenu, répondant à la formule I, on scinde le groupe protecteur du groupe hydroxy $R'_2$ et/ou le groupe protecteur du groupe amino $R'_4$,
le procédé de préparation des composés de formule

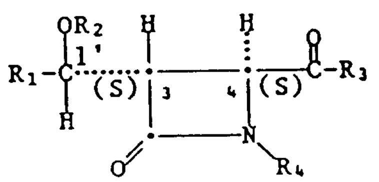

(II),

dans laquelle $R_1$ représente l'hydrogène ou un groupe alkyle en C1-C4, $R_2$ représente l'hydrogène ou un groupe protecteur du groupe hydroxy $R'_2$, $R_3$ représente un groupe phényle, un groupe phényle mono- à tri-substitué par des groupes alcoxy en C1-C4, alkyle en C1-C4 et/ou des halogènes, ou un groupe 4-nitrophényle et $R_4$ représente l'hydrogène ou un groupe protecteur du groupe amino $R'_4$, se caractérisant alors en ce que a) on cyclise un alpha-carbanion d'un composé de formule

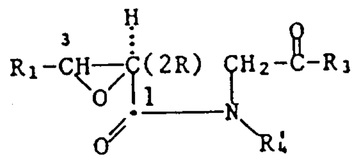

(III),

dans laquelle $R_1$, $R_3$ et $R'_4$ ont les significations indiquées en référence à la formule II, et l'atome de carbone en position 2 est en configuration R et, lorsque $R_1$ représente un groupe alkyle en C1-C4, l'atome de carbone en position 3 est en configuration R ou S, ou un composé de formule

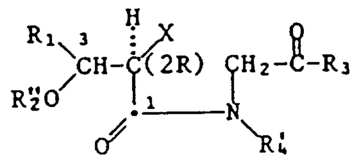

(IV),

dans laquelle $R_1$, $R_3$ et $R'_4$ ont les significations indiquées en référence à la formule II, $R''_2$ représente un groupe protecteur du groupe hydroxy non scindable dans les conditions de la cyclisation, et X représente un groupe éliminable nucléofuge, et l'atome de carbone en position 2 est en configuration R et, lorsque $R_1$ représente un groupe alkyle en C1-C4, l'atome de carbone en position 3 est en configuration R ou S, ou bien b), on isomérise un composé, à l'état d'isomère cis, de formule

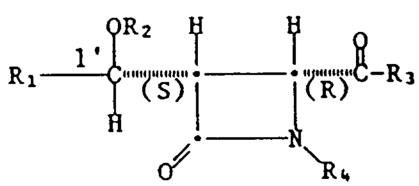

(II'),

dans laquelle $R_1$, $R_2$, $R_3$ et $R_4$ ont les significations indiquées en référence à la formule II, et si on le désire, on scinde le groupe protecteur du groupe amino $R'_4$ et on le remplace par l'hydrogène, et/ou, si on le désire, on convertit un composé ainsi obtenu, répondant à la formule II, en un autre composé de formule II,

le procédé de préparation des composés de formule

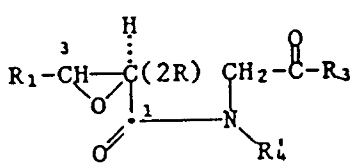

(III),

dans laquelle $R_1$ représente l'hydrogène ou un groupe alkyle en C1-C4, $R_3$ représente un groupe phényle, un groupe phényle mono- à tri-substitué par des groupes alcoxy en C1-C4, alkyle en C1-C4 et/ou des halogènes, ou un groupe 4-nitrophényle, et $R'_4$ représente un groupe protecteur du groupe amino, et l'atome de carbone en position 2 est en configuration R et, lorsque $R_1$ représente un groupe alkyle en C1-C4, l'atome de carbone en position 3 est en configuration R ou S, se caractérisant alors en ce que l'on fait réagir un composé de formule

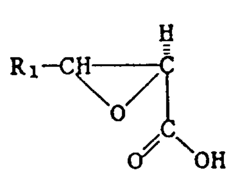

(VIII)

avec une amine de formule

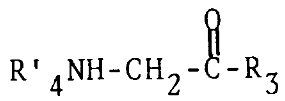

(VII)

dans laquelle $R_3$ et $R'_4$ ont les significations indiquées en référence à la formule V, ou bien on les prépare à partir d'un composé de formule

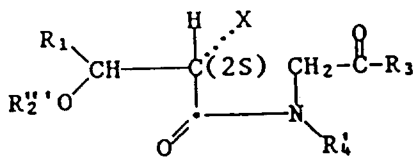

(V),

dans laquelle $R_1$, $R_3$, $R'_4$ et X ont les significations indiquées ci-dessus, $R'''_2$ représente l'hydrogène ou un groupe protecteur du groupe hydroxy scindable dans les conditions de formation de l'époxyde, et dans laquelle l'atome de carbone en position 3 est en configuration R ou S lorsque $R_1$ représente un groupe alkyle en C1-C4, dans des conditions propres à la formation de carbanions et avec inversion de Walden sur l'atome de carbone en position 2,
le procédé de préparation des composés de formule

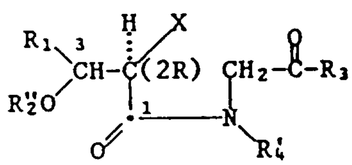

(IV),

dans laquelle $R_1$ représente l'hydrogène ou un groupe alkyle en C1-C4, $R''_2$ représente un groupe protecteur du groupe hydroxy non scindable dans les conditions de la cyclisation, $R_3$ représente un groupe phényle, un groupe phényle mono- à tri-substitué par des groupes alcoxy en C1-C4, alkyle en C1-C4 et/ou des halogènes, ou un groupe 4-nitrophényle, $R'_4$ représente un groupe protecteur du groupe amino, et X un groupe éliminable nucléofuge, et l'atome de carbone en position 2 est en configuration R et, lorsque $R_1$ représente un groupe alkyle en C1-C4, l'atome de carbone en position 3 est en configuration R ou S, se caractérisant alors en ce que l'on fait réagir un acide carboxylique de formule

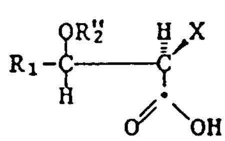

(VI')

dans laquelle $R_1$, $R''_2$ et X ont les significations indiquées en référence à la formule IV, avec une amine de formule

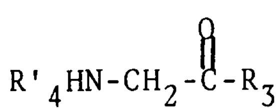

(VII),

dans laquelle $R_3$ et $R'_4$ ont les significations indiquées en référence à la formule V,
le procédé de préparation des composés de formule

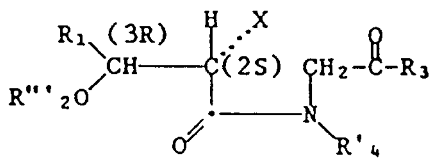

(V),

dans laquelle $R_1$ représente l'hydrogène ou un groupe allkyle en C1-C4, $R'''_2$ représente l'hydrogène ou un groupe protecteur du groupe hydroxy scindable dans les conditions de formation de l'époxyde, $R_3$ représente un groupe phényle, un groupe phényle mono- à tri-substitué par des groupes alcoxy en C1-C4, alkyle en C1-C4 et/ou des halogènes, ou un groupe 4-nitrophényle, $R_4$ représente un groupe protecteur du groupe amino et X un groupe éliminable nucléofuge, et l'atome de carbone en position 3 est en configuration R lorsque $R_1$ représente un groupe alkyle en C1-C4, se caractérisant alors en ce que l'on fait réagir un acide carboxylique de formule

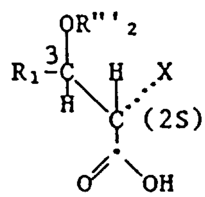

(VI)

dans laquelle $R_1$, $R'''_2$ et X ont les significations indiquées en référence à la formule V, avec une amine de formule

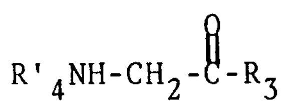

(VII),

dans laquelle $R_3$ et $R'_4$ ont les significations indiquées en référence à la formule V.